# EUROPÄISCHE PATENTSCHRIFT

(11) **EP 1 187 812 B1**
(45) Veröffentlichungstag und Bekanntmachung des Hinweises auf die Patenterteilung: **07.09.2005**
(21) Anmeldenummer: 00925290.9
(22) Anmeldetag: 16.05.2000
(51) Int. Cl.: C07D 235/12, C07D 235/14, C07D 235/30, C07D 401/12, C07D 471/04, A61K 31/4184, A61K 31/4439, A61K 31/4745, A61K 31/496, A61K 31/5377, A61P 9/00, C07D 235/24, C07D 213/56, C07C 237/20

(54) **SUBSTITUIERTE PHENYLCYCLOHEXANCARBONSÄUREAMIDE UND IHRE VERWENDUNG ALS ADENOSINAUFNAHME-INHIBITOREN**
SUBSTITUTED PHENYLCYCLOHEXANE CARBOXYLIC ACID AMIDES AND THEIR USE AS ADENOSINE UPTAKE INHIBITORS
AMIDES D'ACIDE PHENYLCYCLOHEXANECARBOXYLIQUE SUBSTITUES ET LEUR UTILISATION COMME INHIBITEURS DE L'ABSORPTION D'ADENOSINE

(30) Priorität: 29.05.1999 DE 19924819
(43) Veröffentlichungstag der Anmeldung: 20.03.2002
(73) Patentinhaber: Bayer HealthCare AG, 51368 Leverkusen (DE)
(72) Erfinder: BISCHOFF, Erwin, D-42115 Wuppertal (DE); LENSKY, Stephan, D-51515 Kürten (DE); MÜLLER, Stephan-Nicholas, D-42349 Wuppertal (DE); PAULSEN, Holger, D-42115 Wuppertal (DE); KELDENICH, Jörg, D-42113 Wuppertal (DE); KRAHN, Thomas, D-58135 Hagen (DE); SCHUHMACHER, Joachim, D-42109 Wuppertal (DE); JÄNICHEN, Jan, D-22149 Hamburg (DE); THIELEMANN, Wolfgang, D-42107 Wuppertal (DE); STEINHAGEN, Henning, D-42113 Wuppertal (DE)
(86) Internationale Anmeldenummer: PCT/EP2000/004431
(87) Internationale Veröffentlichungsnummer: WO 2000/073274

(56) Entgegenhaltungen:
- EP-A- 0 582 164
- EP-A- 0 611 767
- EP-A- 0 725 064
- WO-A-97/11060
- JACOBSEN P ET AL: "Syntheses of some aminopiperidinecarboxylic acids related to nipecotic acid" ACTA CHEMICA SCANDINAVICA, SECTION B, Bd. 35, Nr. 4, 1981, Seiten 289-294, XP002151653

## Beschreibung

Die vorliegende Erfindung betrifft substituierte Phenylcyclohexancarbonsäureamide, ein Verfahren zu ihrer Herstellung und ihre Verwendung in Arzneimitteln, insbesondere zur Behandlung von cardiovaskulären Erkrankungen, z.B. zur akuten und chronischen Behandlung von ischämischen Erkrankungen.

Adenosin ist ein endogener Effektor mit zellprotektiver Wirksamkeit, insbesondere unter zellschädigenden Bedingungen mit begrenzter Sauerstoffversorgung wie z.B bei Ischämie. Adenosin ist ein stark wirksamer Vasodilatator. Es verstärkt das ischämische "preconditioning" (R. Strasser, A. Vogt, W. Scharper, Z. Kardiologie 85, 1996, 79-89) und es kann das Wachstum von Kollateralgefäßen fördern. Es wird unter hypoxischen Bedingungen z.B. bei kardialen oder peripheren Verschlusskrankheiten freigesetzt (W. Makarewicz "Purine and Pyrimidine Metabolism in Man", Plenum Press New York, 11, 1998, 351-357). Daher schützt Adenosin vor den Folgen Ischaemie bedingter Erkrankungen, z.B. indem es die koronare oder periphere Durchblutung durch Vasodilatation steigert, die Thombozytenaggregation inhibiert und die Angiogenese stimuliert. Der Vorteil der Adenosinaufnahme-Hemmer gegenüber systemisch verabreichtem Adenosin liegt in der Ischämieselektivität. Systemisch verabreichtes Adenosin führt zu einer starken allgemeinen aber häufig unerwünschten Blutdrucksenkung. Der Adenosinaufnahmeinhibitor verstärkt die Wirkung des lokal durch die Ischämie entstandenen Adenosins und dilatiert daher nur die Gefäße in den ischämischen Bereichen. Auch hier verstärken Adenosinaufnahme-Hemmer die Wirkungen von Adenosin und können durch orale oder intravenöse Applikation zur Vorbeugung und Therapie von ischämischen Erkrankungen, z.B. von koronaren Herzkrankheiten, von stabiler und instabiler Angina pectoris, von peripheren und arteriellen Verschlusskrankheiten, von thrombotischem Gefäßverschluss, Myocard-Infarkt und Reperfusionsschäden eingesetzt werden. Außerdem sind sie durch ihr Potential, die Angiogenese zu verstärken, besonders für eine dauerhafte Therapie aller Verschlusskrankheiten geeignet.

Adenosinaufnahme-Hemmer können auch zur Potenzierung der Wirkung von Nukleobase-, Nukleosid- oder Nukleotid-Antimetaboliten in der chemotherapeutischen Behandlung von Krebs und in der antiviralen (z.B. HIV) Chemotherapie eingesetzt werden.

Aus EP-A-0 611 767 und EP-A-725 064 sind Phenylcyclohexylcarbonsäureamide bekannt, die zur Behandlung von Atherosklerose bzw. Restenose eingesetzt werden können.

EP-A- 0 582 164 offenbart strukturell interschiedliche Verbindungen, die eine analoge pharmazeutische Aktivität aufweisen.

Die vorliegende Erfindung betrifft Verwendung von Verbindungen der allgemeinen Formel (I), 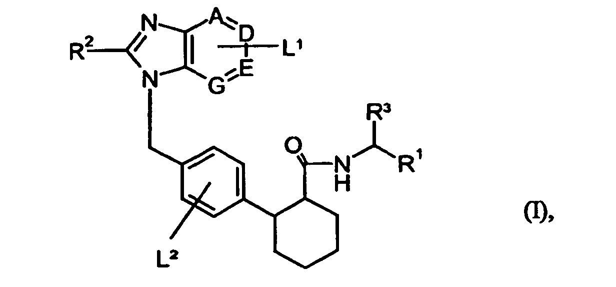 in welcher
- A, D, E und G: gleich oder verschieden sind und für CH-Gruppen oder Stickstoffatome stehen,
- L¹ und L²: gleich oder verschieden sind und unabhängig voneinander für einen oder mehrere Reste ausgewählt aus der Gruppe Wasserstoff, Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl stehen,
- R¹: für die CH₂-OH-Gruppe steht, oder
für einen Rest der Formel CO-NR⁴R⁵ steht,
worin
R⁴ und R⁵ gleich oder verschieden sind und Wasserstoff oder (C₁-C₆)-Alkyl bedeuten,
- R²: für (C₃-C₈)-Cycloalkyl steht,
für (C₁-C₈)-Alkyl steht, das gegebenenfalls durch ein Sauerstoff- oder Schwefelatom oder durch einen Rest NR⁶ unterbrochen ist,
für einen über ein Stickstoffatom an den Imidazolring gebundenen 4- bis 8-gliedrigen, gesättigten Heterocyclus steht, der gegebenenfalls ein weiteres Sauerstoff- oder Schwefelatom enthält, oder
für einen 4- bis 8-gliedrigen gesättigten Heterocyclus steht, der einen Rest der Formel NR⁷ und gegebenenfalls zusätzlich ein Stickstoff-, Sauerstoff- oder Schwefelatom enthält,
wobei (C₃-C₈)-Cycloalkyl, (C₁-C₈)-Alkyl, das gegebenenfalls durch ein Sauerstoff- oder Schwefelatom unterbrochen ist, der über ein Stickstoffatom an den Imidazolring gebundene 4- bis 8-gliedrige, gesättigte Heterocyclus, der gegebenenfalls ein weiteres Sauerstoff- oder Schwefelatom enthält, sowie gegebenenfalls (C₁-C₈)-Alkyl, das durch einen Rest NR⁶ unterbrochen ist, und gegebenenfalls der 4- bis 8-gliedrige gesättigte Heterocyclus, der einen Rest der Formel NR⁷ und gegebenenfalls zusätzlich ein Stickstoff-, Sauerstoff- oder Schwefelatom enthält, durch eine bis drei Hydroxyl-Gruppen und/oder durch einen Rest der Formel -NR⁸R⁹ substituiert sind,
worin
R⁶ und R⁷ gleich oder verschieden sind und Wasserstoff, (C₁-C₆)-Alkyl, Hydroxy-(C₁-C₆)-Alkyl oder (C₃-C₇)-Cycloalkyl bedeuten,
R⁸ und R⁹ gleich oder verschieden sind und Wasserstoff, (C₁-C₆)-Alkyl oder (C₃-C₇)-Cycloalkyl bedeuten,
oder
R⁸ und R⁹ gemeinsam mit dem Stickstoffatom einen 4- bis 8-gliedrigen gesättigten Heterocyclus bilden, der gegebenenfalls zusätzlich ein Sauerstoff- oder Schwefelatom oder einen Rest der Formel NR¹⁰ enthalten kann,
worin
R¹⁰ Wasserstoff, (C₁-C₆)-Alkyl oder (C₃-C₇)-Cycloalkyl bedeuten, und
- R³: für einen Phenyl-, Naphthyl-, Pyrimidinyl-, Pyridyl-, Furyl- oder Thienylring steht, wobei die Ringe gegebenenfalls ein- oder mehrfach mit Resten ausgewählt aus der Gruppe Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl substituiert sind,
und deren Enantiomere und Diastereomere sowie deren jeweilige Salze und Hydrate zur Herstellung von Arzneimitteln zur Behandlung und/oder Prophylase von ischämischen Erkrankungen des Herz-Kreislauf-Systems.

Hierunter bevorzugt ist die Verwendung von Verbindungen der allgemeinen Formel (I) mit der Stereochemie gemäß der folgenden allgemeinen Formel (Ia) 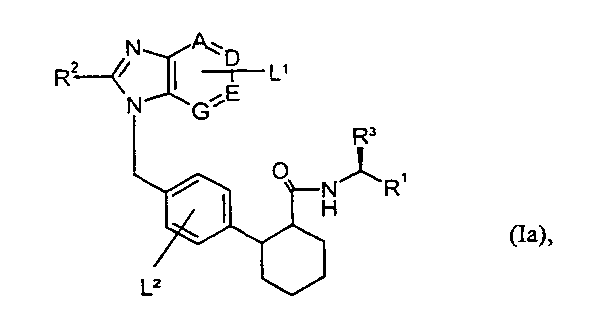 wobei die Substituenten R¹, R², R³, L¹ und L² und die Reste A, D, E und G die zuvor angegebene Bedeutung haben.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Die erfindungsgemäßen Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Als "Hydrate" werden erfindungsgemäß solche Formen der Verbindungen der obigen allgemeinen Formel (I) bezeichnet, welche in festem oder flüssigem Zustand durch Hydratation mit Wasser eine Molekül-Verbindung (Solvat) bilden. In den Hydraten sind die Wassermoleküle nebenvalent durch zwischenmolekulare Kräfte, insbesondere Wasserstoff-Brückenbindungen angelagert. Feste Hydrate enthalten Wasser als sogenanntes Kristall-Wasser in stöchiometrischen Verhältnissen, wobei die Wassermoleküle hinsichtlich ihres Bindungszustands nicht gleichwertig sein müssen. Beispiele für Hydrate sind Sesquihydrate, Monohydrate, Dihydrate oder Trihydrate. Gleichermaßen kommen auch die Hydrate von Salzen der erfindungsgemäßen Verbindungen in Betracht.

(C₁-C₈)-Alkyl, (C₁-C₆)-Alkyl etc. stehen für einen geradkettigen oder verzweigten Alkylrest mit 1 bis 8 bzw. 1 bis 6 Kohlenstoffatomen. Beispielsweise seien genannt: Methyl, Ethyl, n-Propyl, Isopropyl, tert.Butyl, n-Pentyl und n-Hexyl. Bevorzugt ist ein geradkettiger oder verzweigter Alkylrest mit 1 bis 4 Kohlenstoffatomen (C₁-C₄). Besonders bevorzugt ist ein geradkettiger oder verzweigter Alkylrest mit 1 bis 3 Kohlenstoffatomen (C₁-C₃).
(C₁-C₈)-Alkyl, (C₁-C₆)-Alkyl etc., das durch ein Sauerstoff- oder Schwefelatom unterbrochen ist, und das durch eine bis drei Hydroxyl-Gruppen und/oder durch einen Rest der Formel -NR⁸R⁹ substituiert ist, steht beispielsweise für 1,3-Dihydroxy-prop-2-oxy-methyl, 2-Hydroxy-ethoxy-methyl, 2-Hydroxy-prop-1-oxy-methyl, methyl, 3-Hydroxy-prop-1-oxy-methyl, Morpholin-4-yl-methyl, Piperidin-1-yl-methyl, 2-Amino-ethyl, 2-Dimethylamino-ethyl, oder Diethylamino-methyl. (C₁-C₈)-Alkyl, (C₁-C₆)-Alkyl etc., das durch einen Rest NR⁶ unterbrochen ist, und das gegebenenfalls durch eine bis drei Hydroxyl-Gruppen und/oder durch einen Rest der Formel -NR⁸R⁹ substituiert ist, steht beispielsweise für N-(2-Hydroxy-ethyl)-aminomethyl, N-(2-Hydroxy-ethyl)-N-methyl-aminomethyl oder N, N-Bis-(2-Hydroxy-ethyl)-aminomethyl.

Hydroxy-(C₁-C₆)-Alkyl oder Hydroxy-(C₁-C₄)-Alkyl stehen für einen geradkettigen oder verzweigten Alkylrest mit 1 bis 6 bzw. 1 bis 4 Kohlenstoffatomen. Beispielsweise seien genannt: Hydroxymethyl, 2-Hydroxy-ethyl, 2-Hydroxy-prop-1-yl, 3-Hydroxyprop-1-yl, 3-Hydroxy-prop-2-yl, 2-Hydroxy-but-1-yl, 5-Hydroxy-pent-1-yl, und 6-Hydroxy-hex-1-yl. Bevorzugt ist 2-Hydroxy-ethyl.

(C₁-C₆)-Alkoxy steht für einen geradkettigen oder verzweigten Alkoxyrest mit 1 bis 6 Kohlenstoffatomen. Beispielsweise seien genannt: Methoxy, Ethoxy, n-Propoxy, Isopropoxy, tert.Butoxy, n-Pentoxy und n-Hexoxy. Bevorzugt ist ein geradkettiger oder verzweigter Alkoxyrest mit 1 bis 4 Kohlenstoffatomen (C₁-C₄). Besonders bevorzugt ist ein geradkettiger oder verzweigter Alkoxyrest mit 1 bis 3 Kohlenstoffatomen (C₁-C₃).

(C₁-C₆)-Alkoxycarbonyl steht für einen geradkettigen oder verzweigten Alkoxycarbonylrest mit 1 bis 6 Kohlenstoffatomen. Beispielsweise seien genannt: Methoxycarbonyl, Ethoxycarbonyl, n-Propoxycarbonyl, Isopropoxycarbonyl und tert.Butoxycarbonyl. Bevorzugt ist ein geradkettiger oder verzweigter Alkoxycarbonylrest mit 1 bis 4 Kohlenstoffatomen (C₁-C₄). Besonders bevorzugt ist ein geradkettiger oder verzweigter Alkoxycarbonylrest mit 1 bis 3 Kohlenstoffatomen (C₁-C₃).

(C₃-C₈)-Cycloalkyl, (C₃-C₇)-Cycloalkyl etc. stehen im Rahmen der Erfindung für Cyclopropyl, Cyclobutyl, Cyclopentyl, Cyclohexyl, Cycloheptyl oder Cyclooctyl. Bevorzugt seien genannt: Cyclopropyl, Cyclopentyl und Cyclohexyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Ein über ein Stickstoffatom gebundener, 4- bis 8-gliedriger (bevorzugt 5- bis 7-gliedriger) gesättigter Heterocyclus, der gegebenenfalls ein weiteres Sauerstoff- oder Schwefelatom enthält, steht im Rahmen der Erfindung beispielsweise für Pyrrolidin-1-yl, Piperidin-1-yl, Morpholin-4-yl, Thiomorpholin-4-yl, oder 1*H*-Hexahydroazepin-1-yl

Ein 4- bis 8-gliedriger (bevorzugt 5- bis 7-gliedriger), gesättigter Heterocyclus, der einen Rest der Formel NR⁷ und gegebenenfalls zusätzlich ein Stickstoff-, Sauerstoff- oder Schwefelatom enthält, steht im Rahmen der Erfindung beispielsweise für Pyrrolidin-2-yl, 1-Methyl- pyrrolidin-2-yl, Pyrrolidin-3-yl, Pyrazolidin-1-yl, Piperidin-2-yl, 1-Isopropyl- piperidin-3-yl, Morpholin-2-yl, 4-Cyclohexyl-piperazin-1-yl, Thiomorpholin-3-yl, 1-Ethyl-1H-hexahydroazepin-3-yl, oder 4-Methyl-1H-hexahydro-1,4-diazepin-1-yl. Dieser Heterocyclus kann über ein Ringkohlenstoff- oder ein Ringstickstoffatom an den Imidazolring gebunden sein.

Die erfindungsgemäßen Verbindungen können in acht verschiedenen Konfigurationen vorliegen, wobei die folgenden vier unterschiedlichen Konfigurationen (A) bis (D) bevorzugt sind:

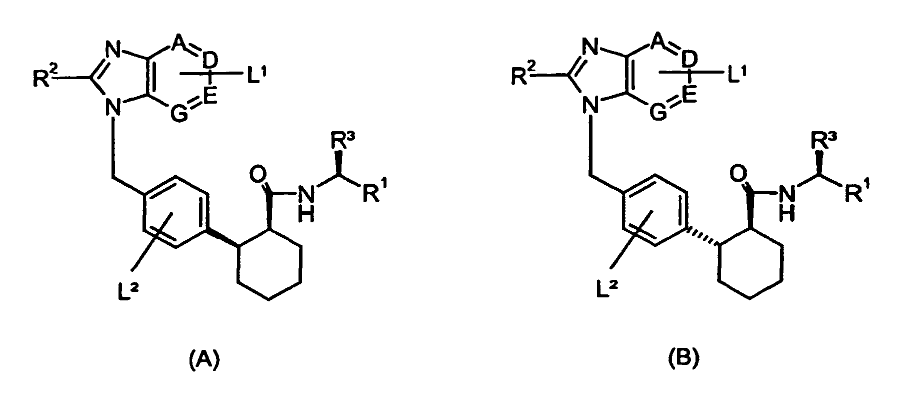

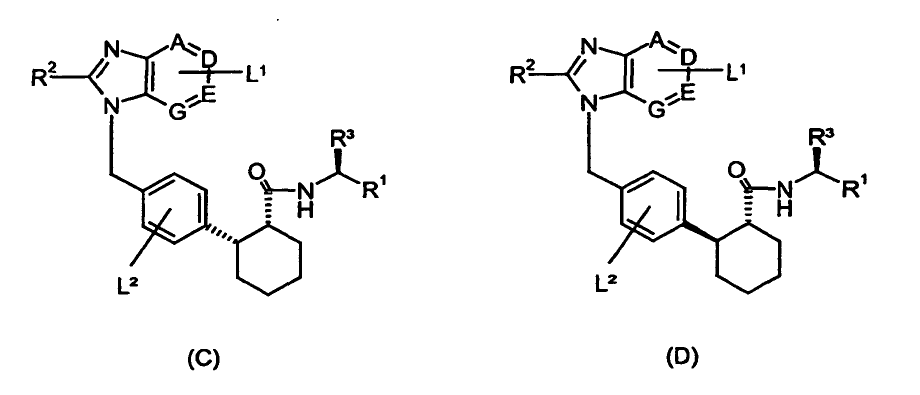

Ganz besonders bevorzugt ist die Konfiguration (D).

Ebenfalls bevorzugt ist die Verwendung von Verbindungen der allgemeinen Formel (I), in welcher R¹ für einen Rest der Formel CO-NR⁴R⁵ steht, wobei R⁴ und R⁵ die zuvor angegebene Bedeutung haben und die übrigen Reste wie zuvor definiert sind.

Besonders bevorzugt sind erfindungsgemäßeVerbindungen der allgemeinen Formel (I),
wobei
A, D, E und G jeweils für die CH-Gruppe stehen,
oder einer der Reste A, D, E und G ein Stickstoffatom bedeutet und die anderen jeweils für die CH-Gruppe stehen,
- L¹ und L²: gleich oder verschieden sind und unabhängig voneinander für einen oder mehrere Reste ausgewählt aus der Gruppe Wasserstoff, Fluor, Chlor, Cyano, Trifluormethyl oder Trifluormethoxy stehen,
- R¹: für die -CH₂-OH-Gruppe steht, oder
für einen Rest der Formel -CO-NR⁴R⁵ steht,
worin
R⁴ und R⁵ gleich oder verschieden sind und Wasserstoff oder (C₁-C₃)-Alkyl bedeuten,
- R²: für (C₃-C₇)-Cycloalkyl steht,
für (C₁-C₆)-Alkyl steht, das gegebenenfalls durch ein Sauerstoff- oder Schwefelatom oder durch einen Rest NR⁶ unterbrochen ist,
für einen über ein Stickstoffatom an den Imidazolring gebundenen 5- bis 7-gliedrigen, gesättigten Heterocyclus steht, der gegebenenfalls ein weiteres Sauerstoff- oder Schwefelatom enthält, oder
für einen 5- bis 7-gliedrigen gesättigten Heterocyclus steht, der einen Rest der Formel NR⁷ und gegebenenfalls zusätzlich ein Stickstoff-, Sauerstoff- oder Schwefelatom enthält,
wobei (C₃-C₇)-Cymoalkyl, (C₁-C₆)-Alkyl, das gegebenenfalls durch ein Sauerstoff- oder Schwefelatom unterbrochen ist, der über ein Stickstoffatom an den Imidazolring gebundene 5- bis 7-gliedrige, gesättigte Heterocyclus, der gegebenenfalls ein weiteres Sauerstoff- oder Schwefelatom enthält, sowie gegebenenfalls (C₁-C₆)-Alkyl, das durch einen Rest NR⁶ unterbrochen ist, und gegebenenfalls der 5- bis 7-gliedrige gesättigte Heterocyclus, der einen Rest der Formel NR⁷ und gegebenenfalls zusätzlich ein Stickstoff-, Sauerstoff- oder Schwefelatom enthält, durch eine Hydroxyl-Gruppe und/oder durch einen Rest der Formel -NR⁸R⁹ substituiert sind,
worin
R⁶ und R⁷ gleich oder verschieden sind und Wasserstoff, (C₁-C₄)-Alkyl, Hydroxy-(C₁-C₄)-Alkyl oder (C₃-C₆)-Cycloalkyl bedeuten,
R⁸ und R⁹ gleich oder verschieden sind und Wasserstoff, (C₁-C₄)-Alkyl oder (C₃-C₆)-Cycloalkyl bedeuten,
oder
R⁸ und R⁹ gemeinsam mit dem Stickstoffatom einen 5- bis 7-gliedrigen gesättigten Heterocyclus bilden, der gegebenenfalls zusätzlich ein Sauerstoff- oder Schwefelatom oder einen Rest der Formel NR¹⁰ enthalten kann,
worin
R¹⁰ Wasserstoff, (C₁-C₄)-Alkyl oder (C₃-C₆)-Cycloalkyl bedeutet, und
- R³: für einen Phenyl-, Pyridyl- oder Thienylring steht, der gegebenenfalls einoder mehrfach mit Resten ausgewählt aus der Gruppe Fluor, Chlor, Cyano, Trifluormethyl oder Trifluormethoxy substituiert ist,
und deren Enantiomere und Diastereomere sowie deren jeweilige Salze, und Hydrate.

Ein weiteren gegenstand der Erfindung sind Verbindungen der allgemeinen Formel (I),
wobei
A, D und E jeweils für die CH-Gruppe stehen,
G für ein Stickstoffatom oder für die CH-Gruppe steht,
L¹ und L² für Wasserstoff stehen,
- R¹: für einen Rest der Formel -CO-NR⁴R⁵ steht,
worin
R⁴ und R⁵ Wasserstoff bedeuten,
- R²: für (C₁-C₄)-Alkyl steht, das gegebenenfalls durch ein Sauerstoffatom unterbrochen ist, oder
für einen 4-R⁷-Piperazin-1-ylrest steht
wobei (C₁-C₄)-Alkyl, das gegebenenfalls durch ein Sauerstoffatom unterbrochen ist, durch eine Hydroxyl-Gruppe oder durch einen Rest der Formel -NR⁸R⁹ substituiert ist,
worin
R⁷ Wasserstoff, (C₁-C₄)-Alkyl oder (C₃-C₆)-Cycloalkyl bedeutet,
R⁸ und R⁹ gleich oder verschieden sind und Wasserstoff, (C₁-C₄)-Alkyl oder (C₃-C₆)-Cycloalkyl bedeuten,
oder
R⁸ und R⁹ gemeinsam mit dem Stickstoffatom einen Morpholinrest bilden, und
- R³: für einen Phenyl- oder Pyridylrest steht, wobei der Phenylrest ein- oder mehrfach durch Fluor substituiert sein kann,
und deren Enantiomere und Diastereomere sowie deren jeweilige Salze und Hydrate.

Bevorzugt sind Verbindungen der folgenden allgemeinen Formel (Ib) 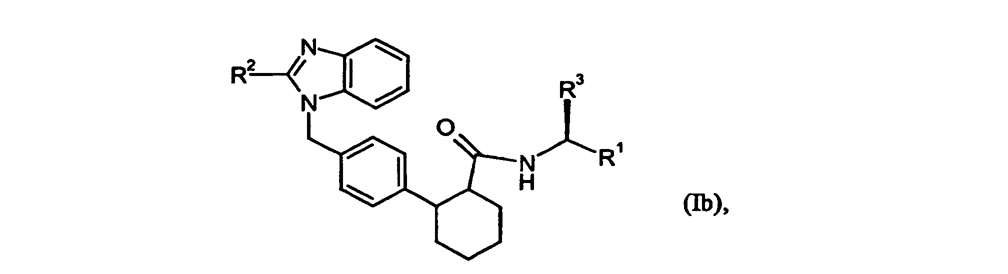 in der
- R¹: für eine Gruppe -C(O)-NH₂ steht,
- R²: für (C₁-C₃)-Alkyl steht, das am endständigen C-Atom durch eine Hydroxylgruppe substituiert ist,
- R³: für einen Phenylring steht, der in der para-Position durch Fluor substituiert ist,
oder
für einen Pyridylrest steht,
und deren Diastereomere sowie deren jeweilige Salze, Hydrate und gegebenenfalls deren Prodrugs.

Besonders bevorzugt sind die Verbindungen der allgemeinen Formel (I) mit den folgenden Strukturen:

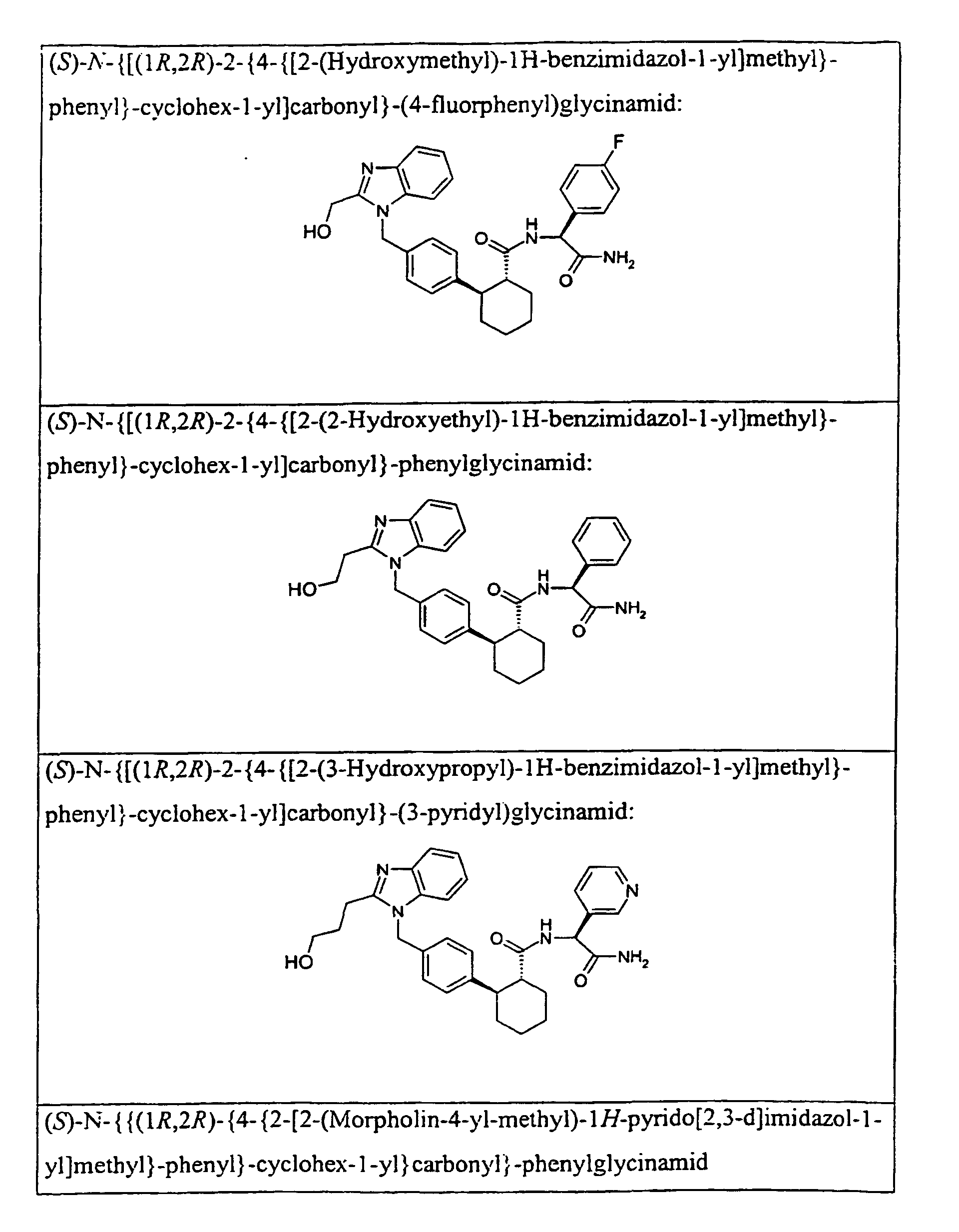

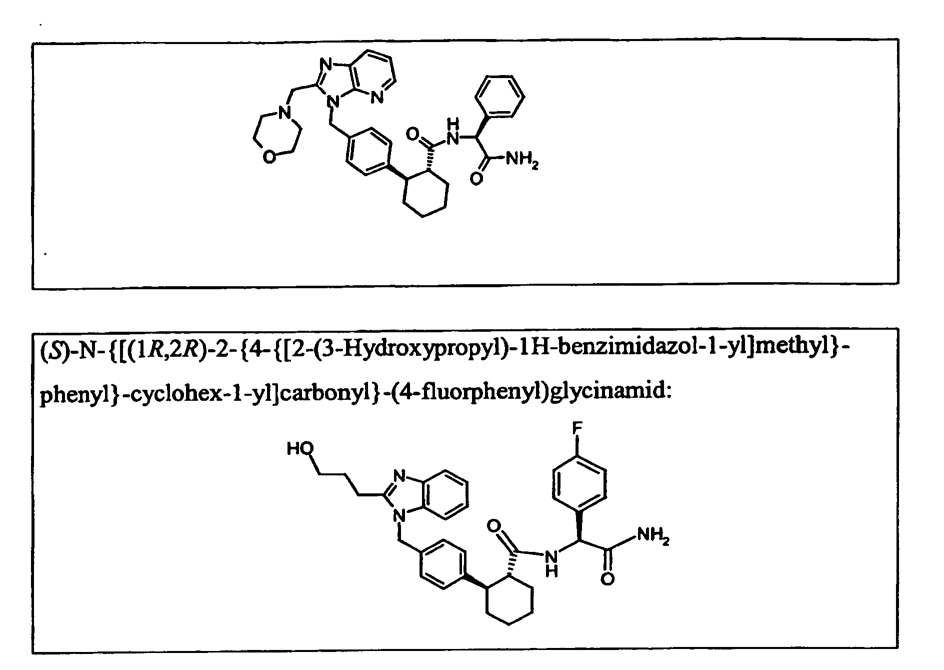

und ihre Salze und Hydrate.

Hierunter ganz besonders bevorzugt ist das (*S*)-N-{[(1*R*,2*R*)-2-{4-{[2-(3-Hydroxypropyl)-1H-benzimidazol-1-yl]methyl}phenyl}-cyclohex-1-yl]carbonyl}-(4-fluorphenyl)glycinamid: 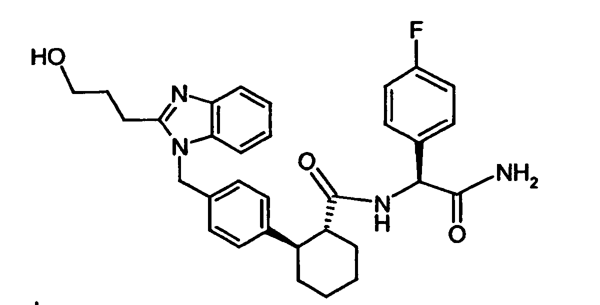 und seine Salze und Hydrate.

Außerdem wurden Verfahren zur Herstellung der erfindungsgemäßen Verbindungen der allgemeinen Formel (I) gefunden, bei dem man
[A] Verbindungen der allgemeinen Formel (II) 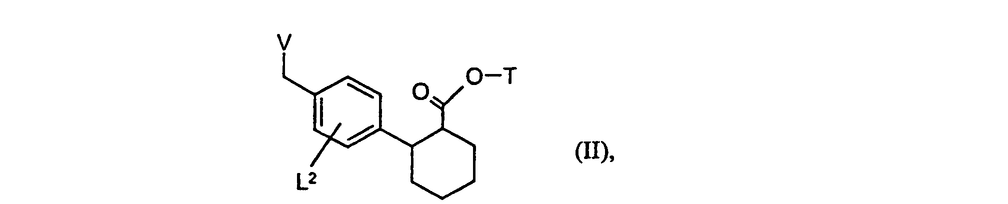 in welcher
   L² die oben angegebene Bedeutung hat,
   - T: für (C₁-C₄)-Alkyl, vorzugsweise für Methyl oder tert.Butyl steht,
   und
   - V: für eine geeignete Abgangsgruppe, wie beispielsweise Halogen, Mesylat oder Tosylat, vorzugsweise für Brom steht,
   zunächst durch Umsetzung mit Verbindungen der allgemeinen Formel (III) 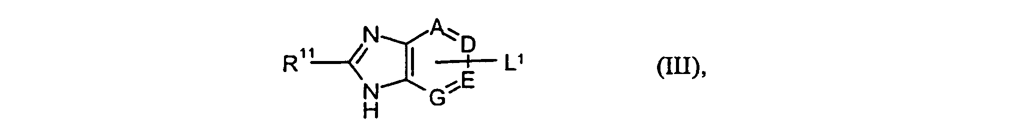 in welcher
   A, D, E, G und L¹ die oben angegebene Bedeutung haben
   und
   - R¹¹: die oben aufgeführte Bedeutung von R² hat, wobei Amino- und Hydroxyfunktionen gegebenenfalls durch geeignete Amino- bzw. Hydroxyschutzgruppen blockiert sind,
   in inerten Lösemitteln, in Abhängigkeit der Definition von R¹¹ gegebenenfalls in Anwesenheit einer Base in die Verbindungen der allgemeinen Formel (IV) 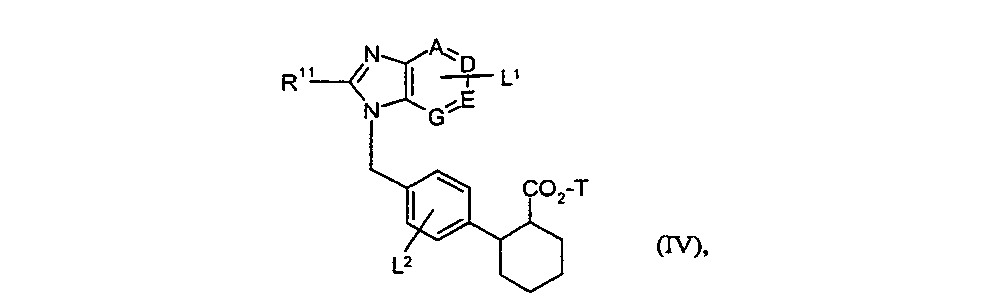 in welcher
   R¹¹, A, D, E, G, L¹, L², und T die oben angegebene Bedeutung haben,
   überführt,
   in einem nächsten Schritt mit Säuren oder Basen in die entsprechenden Carbonsäuren der allgemeinen Formel (V) 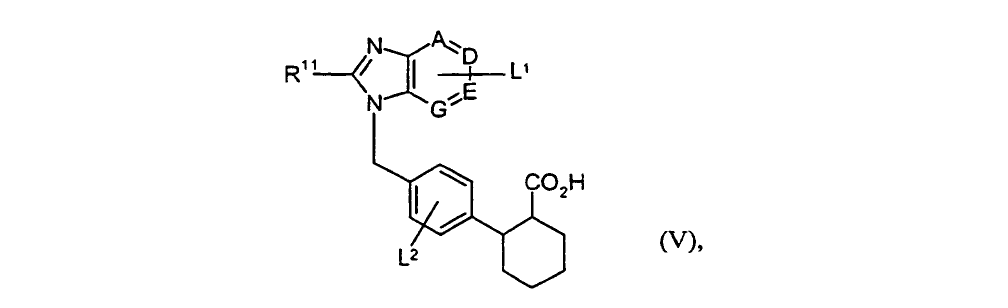 in welcher
   R¹¹, A, D, E, G, L¹ und L² die oben angegebene Bedeutung haben,
   überführt,
   diese gegebenenfalls aktiviert, insbesondere durch Überführung in ein entsprechendes Carbonsäurederivat wie Carbonsäurehalogenid, Carbonsäureanhydrid oder Carbonsäureester,
   und abschließend nach bekannten Methoden mit Verbindungen der allgemeinen Formel (VI) oder deren Salzen 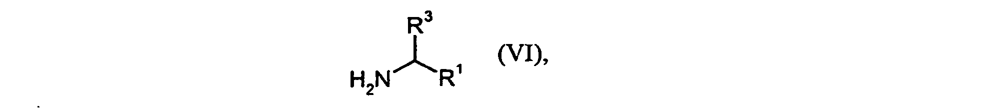 in welcher
   R¹ und R³ die oben angegebene Bedeutung haben,
   in inerten Lösemitteln umsetzt
   und gegebenenfalls im Fall, dass R¹¹ eine der oben aufgeführten Schutzgruppen trägt, diese entweder bei der Hydrolyse zu den Säuren (IV) -> (V) oder nach Umsetzung mit den Verbindungen der allgemeinen Formel (VI) nach üblichen Methoden abspaltet,
   oder
[B] im Fall, dass R² für einen direkt über ein Stickstoffatom an den Imidazolring gebundenen, gesättigten Heterocyclus steht,
   zunächst die oben aufgeführten Verbindungen der allgemeinen Formel (II) mit Verbindungen der allgemeinen Formel (IIIa) 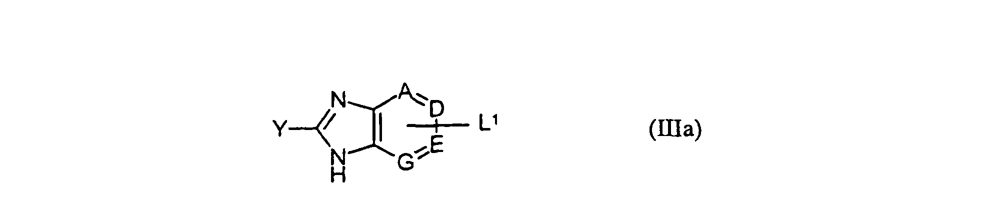
in welcher
A, D, E, G und L¹ die oben angegebene Bedeutung haben
und
- Y: für Halogen oder Mesylat , vorzugsweise für Chlor, Brom oder Mesylat steht,
in inerten Lösemitteln in die entsprechenden Verbindungen der Formel (VII) 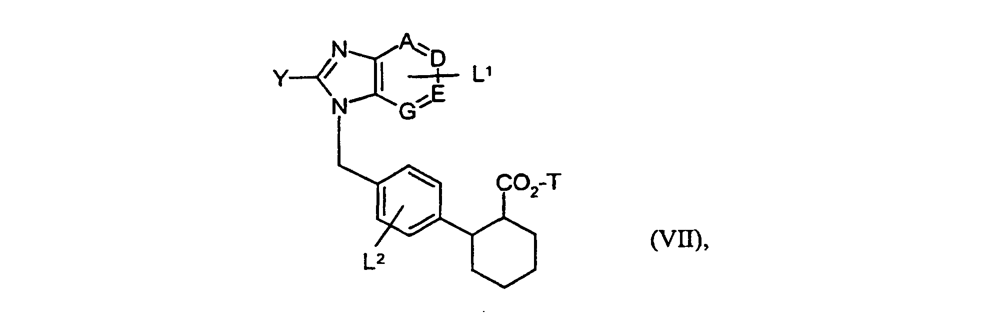 in welcher
Y, A, D, E, G, L¹, L² und T die oben angegebene Bedeutung haben,
überführt,
in einem nächsten Schritt mit Verbindungen der allgemeinen Formel (VIII)

HNR¹²R¹³ (VIII),

in welcher
- R¹² und R¹³: zusammen mit dem Stickstoffatom einen Heterocyclus gemäß der Definition von R² bilden,
zu Verbindungen der allgemeinen Formel (IX) 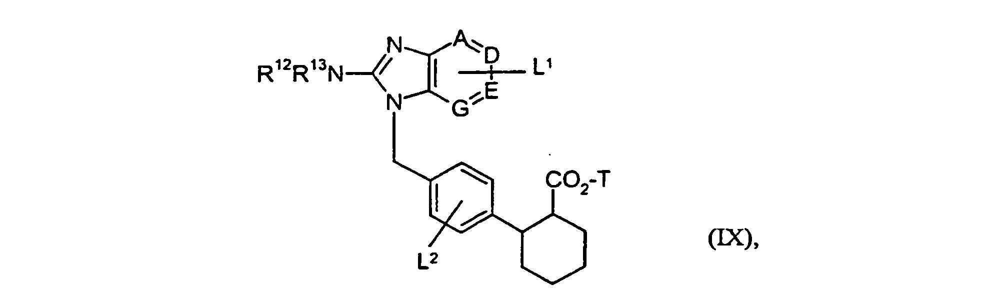 in welcher
A, D, E, G, L¹, L², R¹², R¹³ und T die oben angegebene Bedeutung haben,
umsetzt,
in den nächsten Schritten, wie unter [A] beschrieben, durch Hydrolyse in die entsprechenden Carbonsäuren der allgemeinen Formel (X) 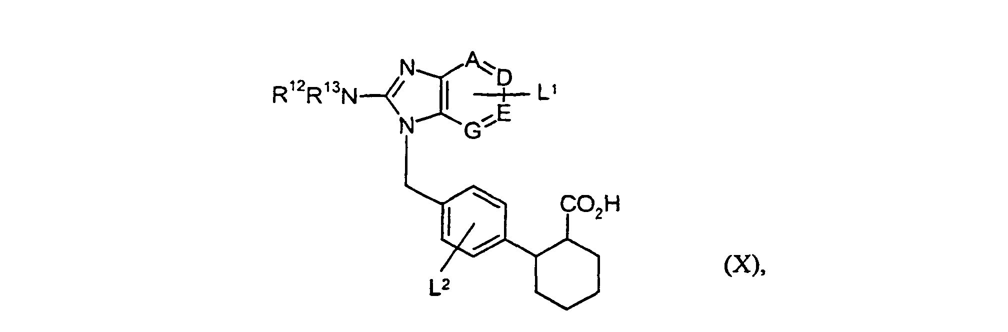 in welcher
A, D, E, G, L¹, L², R¹² und R¹³ die oben angegebene Bedeutung haben,
überführt,
und abschließend nach bekannten Methoden, wie sie für die Herstellung von Amiden aus Carbonsäuren und Aminen üblich sind (z.B. nach gegebenenfalls durchgeführter Aktivierung, insbesondere durch Überführung in ein entsprechendes aktiviertes Carbonsäurederivat wie Carbonsäurehalogenid, Carbonsäureanhydrid oder Carbonsäureester), mit den Verbindungen der allgemeinen Formel (VI) zu den Verbindungen der allgemeinen Formel (I) umsetzt.

Die gemäß der Verfahrensvariante [A] oder [B] erhaltenen Verbindungen der allgemeinen Formel (I) können gegebenenfalls anschließend durch Umsetzung z.B. mit einer Säure in die entsprechenden Salze überführt werden.

Die Herstellung der Verbindungen der entsprechenden diastereomeren und enantiomeren Formen erfolgt entsprechend, und zwar entweder unter Verwendung enantiomeren- oder diastereomerenreiner Ausgangsstoffe oder aber durch nachträgliche Trennung der gebildeten Racemate mit üblichen Methoden (z.B. Racematspaltung, Chromatographie an chiralen Säulen etc.).

So können beispielsweise die Verbindungen der bevorzugten Konfiguration der allgemeinen Formel (Ia) 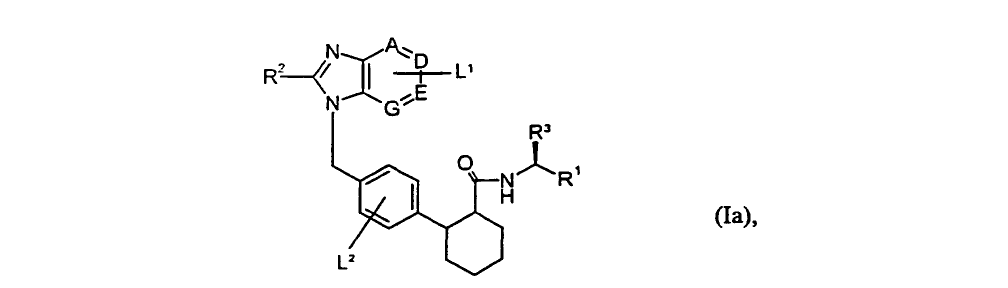 wobei die Substituenten R¹, R², R³, L¹ und L² und die Reste A, D, E und G die zuvor angegebene Bedeutung haben,
hergestellt werden, indem anstelle der racemischen Verbindung der allgemeinen Formel (VI) die enantiomerenreine Verbindung der allgemeinen Formel (VI a) 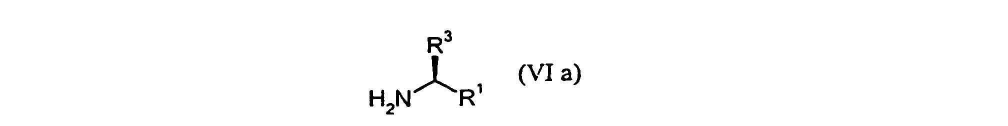 oder deren Salze eingesetzt werden.

Eine ganz besonders bevorzugte Verbindung der allgemeinen Formel (VI a) ist das (*S*)-(4-Fluorphenyl)glycinamid 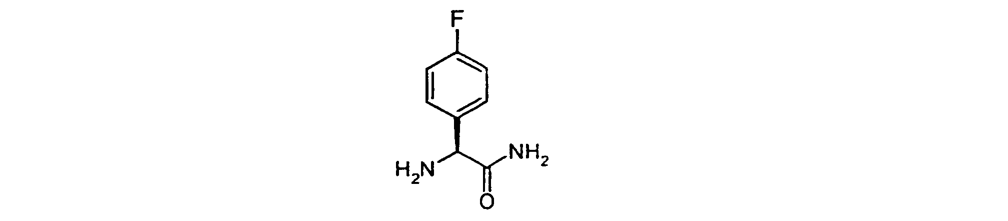 und dessen Salze (z.B. das Hydrochlorid).

Eine ebenso ganz besonders bevorzugte Verbindung der allgemeinen Formel (VI a) ist das (*S*)-(3-Pyridyl)glycinamid und seine Salze.

Die erfindungsgemäßen Verfahren können durch folgende Formelschemata beispielhaft erläutert werden:

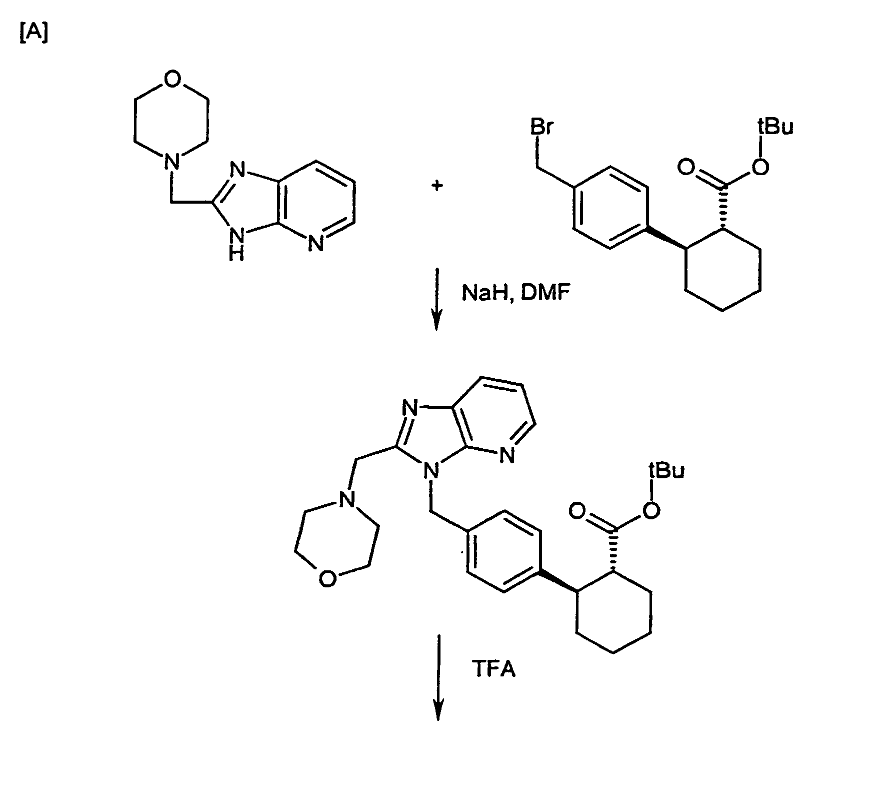

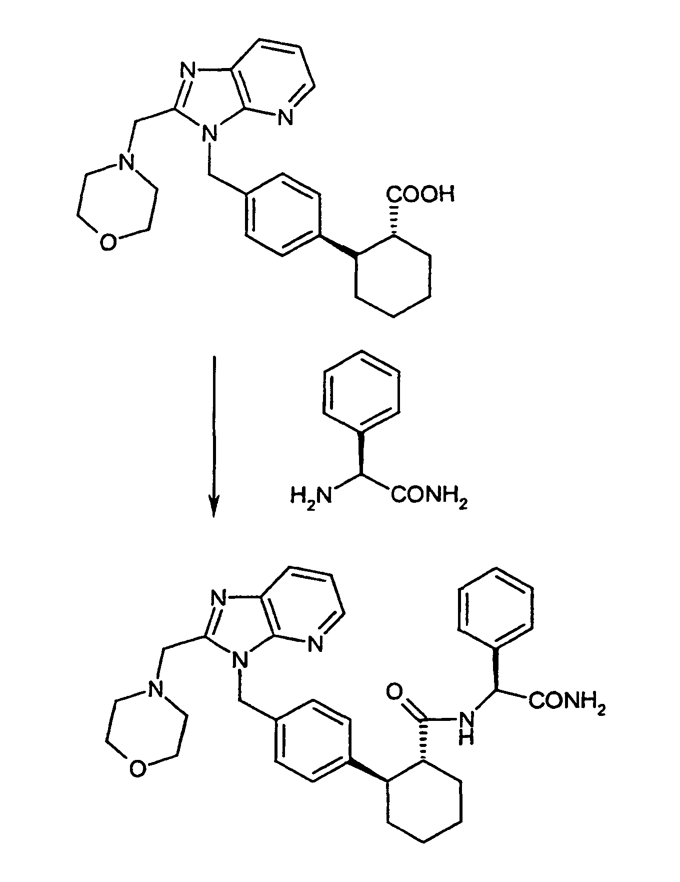

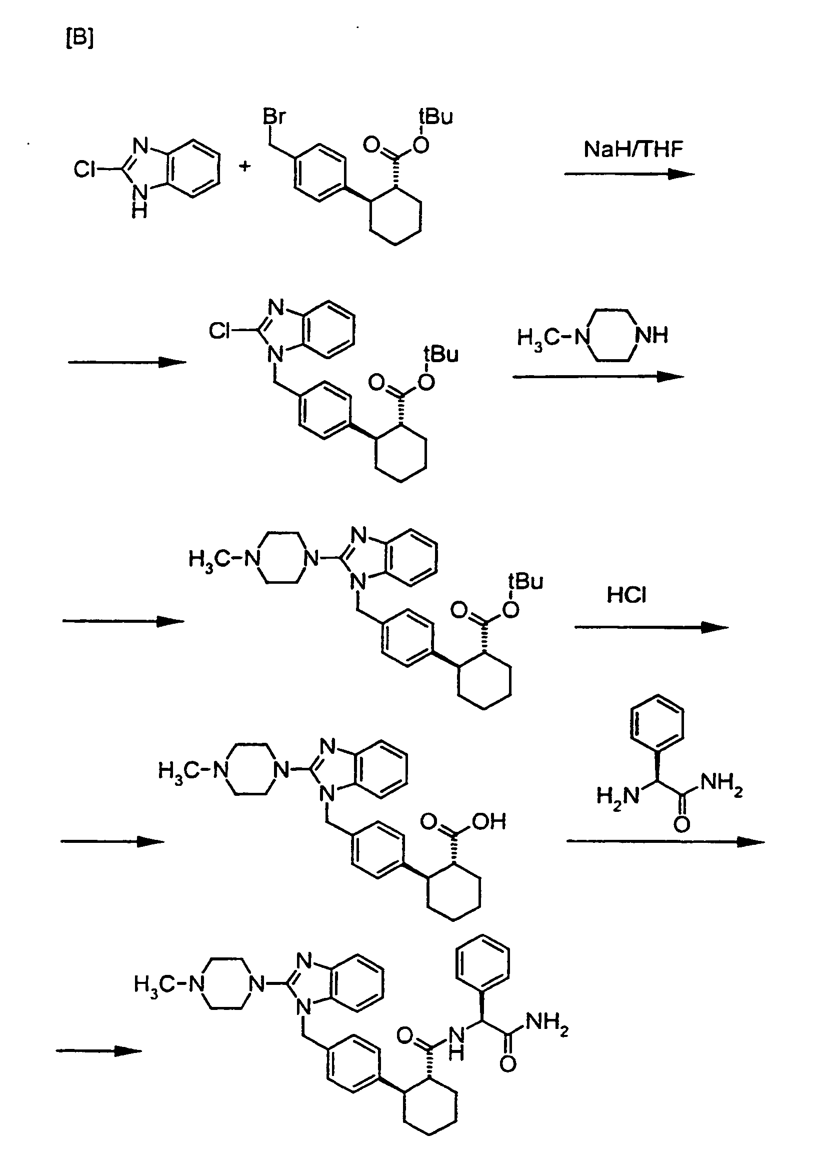

Geeignete Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl. Eine bevorzugte Schutzgruppe für primäre Amine ist Phthalimid. Bevorzugte Schutzgruppen für sekundäre Amine sind Benzyloxycarbonyl und tert.-Butoxycarbonyl.

Die Abspaltung der Aminoschutzgruppen erfolgt in an sich bekannter Weise, indem man beispielsweise unter hydrogenolytischen, sauren oder basischen Bedingungen, bevorzugt mit Säuren, wie beispielsweise Chlorwasserstoffsäure oder Trifluoressigsäure in inerten Lösemitteln wie Ether, Dioxan und Methylenchlorid arbeitet.

Eine geeignete Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.-Butyl-dimethylsilyl, tert.-Butyldiphenylsilyl, Dimethylhexylsilyl, Trimethylsilylethoxycarbonyl, Benzyl, Triphenylmethyl(Trityl), Monomethoxytrityl (MMTr), Dimethyloxytriyl (DMTr), Benzyloxycarbonyl, 2-Nitrobenzyl, 4-Nitrobenzyl, 2-Nitrobenzyloxycarbonyl, 4-Nitrobenzyloxycarbonyl, tert.Butyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]-methyl, 2-(Methylthiomethoxy)ethoxycarbonyl, Tetrahydropyranyl, Benzoyl, N-Succinimid, 4-Methylbenzoyl, 4-Nitrobenzoyl, 4-Fluorbenzoyl, 4-Chlorbenzoyl oder 4-Methoxybenzoyl. Bevorzugt ist tertiär Butyldimethylsilyl.

Die Abspaltung der Hydroxyschutzgruppe erfolgt in an sich bekannter Weise z.B. durch Säure, Base oder durch Zugabe von Tetrabutylammoniumfluorid oder erfolgt bei der Hydrolyse der Carbonsäure.

Als Lösemittel für die Verfahren eignen sich übliche organische Lösemittel, die sich unter den Reaktionsbedingungen nicht verändern. Hierzu gehören Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Pyridin, Dimethylsulfoxid, Dimethylformamid, N,N'-Dimethylpropylenharnstoff (DMPU), N-Methylpyrrolidon (NMP), Acetonitril, Aceton oder Nitromethan. Ebenso ist es möglich, Gemische der genannten Lösemittel zu verwenden. Bevorzugt sind für das Verfahren [A] (II) + (III) -> (IV) Diethylether, Tetrahydrofuran und Dimethylformamid. Besonders bevorzugt ist Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), 1,5-Diazabicyclo [4.3.0]non-5-en (DBN), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Cäsiumcarbonat, Triethylamin, Trimethylamin, Pyridin, Kalium-tert.butylat, DBU oder DABCO. Ganz besonders bevorzugt ist für den Schritt [A] (II) + (III) -> (IV) sind Natriumhydrid und Natriumhydroxid.

Im allgemeinen setzt man die Base in einer Menge von 0,05 Mol bis 10 Mol, bevorzugt von 1 Mol bis 2 Mol bezogen auf 1 Mol der Verbindung der Formel (II) ein.

Das erfindungsgemäße Verfahren (II) + (III) -> (IV) wird im allgemeinen in einem Temperaturbereich von -20°C bis +100°C, insbesondere von -20°C bis +60°C, bevorzugt von 0°C bis +60°C, durchgeführt.

Das erfindungsgemäße Verfahren (II) + (III) -> (IV) wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Hydrolyse der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden oder mit Säure im Falle der t-Butylester.

Als Basen eignen sich für die Hydrolyse die üblichen anorganischen Basen. Hierzu gehören bevorzugt Alkalihydroxide oder Erdalkalihydroxide wie beispielsweise Natriumhydroxid, Lithiumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Lithiumhydroxid eingesetzt.

Als Säuren eignen sich im allgemeinen Trifluoressigsäure, Schwefelsäure, Chlorwasserstoff, Bromwasserstoff und Essigsäure oder deren Gemisch ggf. unter Zusatz von Wasser. Bevorzugt sind Chlorwasserstoff oder Trifluoressigsäure im Falle der tertiär-Butylester und Salzsäure im Falle der Methylester.

Als Lösemittel eignen sich für die Hydrolyse Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, Dimethylformamid, Dichlormethan oder Dimethylsulfoxid. Ebenso ist es möglich, Gemische der genannten Lösemittel einzusetzen. Bevorzugt sind Wasser/Tetrahydrofuran und im Falle der Umsetzung mit Trifluoressigsäure Dichlormethan sowie im Falle von Chlorwasserstoff Tetrahydrofuran, Diethylether, Dioxan oder Wasser.

Die Hydrolyse wird im allgemeinen in einem Temperaturbereich von 0°C bis +100°C durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Hydrolysen wird die Base oder die Säure im allgemeinen in einer Menge von 1 bis 100 mol, bevorzugt von 1,5 bis 40 mol bezogen auf 1 mol des Esters eingesetzt.

Als Hilfsstoffe für die Amidbildungen werden bevorzugt Kondensationsmittel eingesetzt. Bevorzugt werden hier die üblichen Kondensationsmittel wie Carbodiimide z.B. N,N'-Diethyl-, N,N,'-Dipropyl-, N,N'-Diisopropyl-, N,N-Dicyclohexylcarbodiimid, N-(3-Dimethylaminoisopropyl)-N'-ethylcarbodiimid-Hydrochlorid (EDC), oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri-(dimethylamino)phosphoniumhexafluorophosphat, oder O-(Benzotriazol-1-yl)-N,N,N',N'-tetra-methyluronium-hexafluorophosphat (HBTU) oder O-(7-Azabenzotriazol-1-yl)-N,N,N',N'-tetramethyl-uroniumhexafluorophosphat (HATU), und als Basen Alkalicarbonate z.B. Natrium- oder Kaliumcarbonat, oder -hydrogencarbonat, oder organische Basen wie Trialkylamine z.B. Triethylamin, N-Ethylmorpholin, N-Methylpiperidin oder Diisopropylethylamin eingesetzt. Besonders bevorzugt ist die Kombination von EDC, N-Methylmorpholin und 1-Hydroxybenztriazol.

Die Verbindungen der allgemeinen Formeln (II), (IIIa), (VI) und (VIII) sind bekannt oder nach üblichen Methoden herstellbar (vgl. EP-A-0 725 061, EP-A-0 725 064).

Die Verbindungen der allgemeinen Formel (III) sind größtenteils neu und können hergestellt werden, indem man, im Fall, dass R¹¹ nicht für einen direkt über N-gebundenen Heterocyclus steht, Verbindungen der allgemeinen Formel (XI) 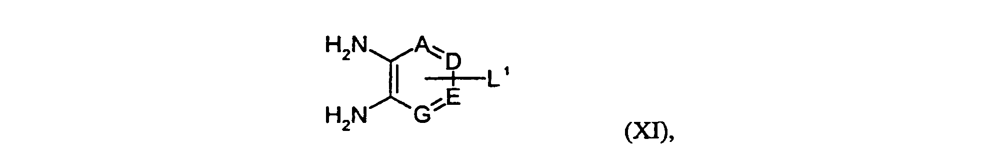 in welcher
A, D, E, G und L¹ die oben angegebene Bedeutung haben,
mit Verbindungen der allgemeinen Formel (XII)

R¹¹-CO₂H (XII),

in welcher
- R¹¹: die oben angegebene Bedeutung hat,
oder gegebenenfalls deren Estern, Lactonen oder anderen reaktiven Vorstufen (z. B. Imidoestem)
gegebenenfalls unter Entfernung des Reaktionswassers und gegebenenfalls in Anwesenheit einer Säure, vorzugsweise mit PPA, HCl und p-TsOH, umsetzt (vgl. hierzu auch *J. Org. Chem*. **1941,** *6*, 25 ff und *Bull. Soc. Chim. Fr*. **1991,** *128*, 255-259),
und im Fall, dass R¹¹ für einen der oben unter R² aufgeführten Reste steht, der gegebenenfalls auch eine Schutzgruppe trägt, Verbindungen der allgemeinen Formel (XI) zunächst durch Umsetzung mit Verbindungen der allgemeinen Formel (XIII)

HO-R¹⁴-CO₂H (XIII),

in welcher
- R¹⁴: für (C₁-C₈)-Alkandiyl steht,
oder gegebenenfalls deren Estern, Lactonen oder anderen reaktiven Vorstufen (z. B. Imidoestem)
in die Verbindungen der allgemeinen Formel (XIV) 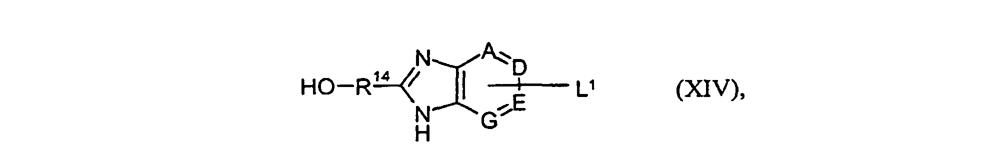 in welcher
A, B, D, G, R¹⁴ und L¹ die oben angegebene Bedeutung haben,
in inerten Lösemitteln überführt,
anschließend die Hydroxygruppe durch Halogen, Mesylat oder Tosylat substituiert und somit die Verbindungen der allgemeinen Formel (XV), 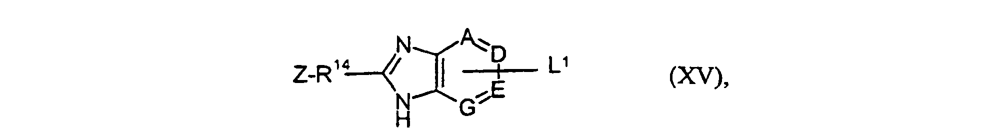 in welcher
R¹⁴, A, D, E, G und L¹ die oben angegebene Bedeutung haben
und
- Z: für Halogen, Mesylat oder Tosylat steht,
herstellt, und diese mit Aminen der allgemeinen Formel (XVI)

R⁸R⁹NH (XVI),

in welcher
R⁸ und R⁹ die oben angegebene Bedeutung haben,
umsetzt (vgl. hierzu auch *J. Am. Chem. Soc.* **1948**, *70*, 3406; *J. Helerocycl. Chem.* 1969, 759-60).

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Pyridin, Dimethylsulfoxid, Dimethylformamid, N,N'-Dimethylpropylenhamstoff (DMPU), N-Methylpyrrolidon (NMP), Acetonitril, Aceton oder Nitromethan. Ebenso ist es möglich, Gemische der genannten Lösemittel zu verwenden. Bevorzugt sind Dichlormethan, Tetrahydrofuran und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Trimethylamin, Pyridin, Kalium- tert.butylat, DBU oder DABCO.

Im allgemeinen setzt man die Base in einer Menge von 0,05 Mol bis 10 Mol, bevorzugt von 1 Mol bis 2 Mol bezogen auf 1 Mol der Verbindung der Formel (XV) ein.

Das erfindungsgemäße Verfahren wird im allgemeinen in einem Temperaturbereich von -50°C bis +100°C, bevorzugt von -30°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formeln (XII), (XIII) und (XVI) sind an sich bekannt oder nach üblichen Methoden herstellbar.

Die Verbindungen der allgemeinen Formeln (XIV) und (XV) sind teilweise neu und können nach üblichen Methoden, beispielsweise wie oben beschrieben, hergestellt werden.

Die Verbindungen der allgemeinen Formel (IV), (V), (VII), (IX) und (X) und deren Salze sind neu und können wie oben beschrieben hergestellt werden.

Überraschenderweise zeigen die erfindungsgemäßen Verbindungen der allgemeinen Formel (I) und ihre Analoga ein nicht vorhersehbares, wertvolles pharmakologisches Wirkspektrum kombiniert mit einer verbesserten Wasserlöslichkeit, die sich gegebenenfalls erst unter Zuhilfenahme von Formulierungshilfsmitteln und/oder durch Einstellung eines geeigneten pH-Wertes erreichen lässt, und gegebenenfalls auch kombiniert mit einer erhöhten metabolischen Stabilität.
Sie können daher zur Herstellung von Arzneimitteln zur Behandlung von allen ischämiebedingten Erkrankungen, insbesondere zur akuten und chronischen Behandlung von ischämischen Erkrankungen des Herz-Kreislauf-Systems (wie z.B. der koronaren Herzkrankheit, der stabilen und instabilen Angina pectoris, von peripheren und arteriellen Verschlusskrankheiten, von thrombotischen Gefäßverschlüssen, des Myocardinfarkts und von Reperfusionsschäden) verwendet werden.
Außerdem sind sie durch ihr Potential, die Angiogenese zu verstärken, besonders für eine dauerhafte Therapie aller Verschlusskrankheiten geeignet. Die Substanzen eignen sich somit zur Behandlung aller ischämiebedingter peripherer und kardiovaskulären Erkrankungen, d.h. von ischämiebedingten Erkrankungen des Herz-Kreislauf-Systems.
Eine gute Wasserlöslichkeit ist bei Arzneimittelwirkstoffen und -formulierungen bekanntermaßen eine vorteilhafte Eigenschaft; so eignen sich die erfindungsgemäßen Verbindungen beispielsweise besonders gut zur oralen und intravenösen Applikation.

### Testsysteme

### 1. Bestimmung der Löslichkeit

Zur Löslichkeitsbestimmung wurde eine Fällungsmethode herangezogen:
10 mg der Testsubstanz werden in 50µl DMSO vollständig gelöst (Stammlösung). Von dieser Lösung gibt man 20µl in 2000µl physiologische Kochsalzlösung. Diese Lösung wiederum wird zur Equilibrierung bei 25°C im Thermomixer Comfort (Fa. Eppendorf) bei 1400 upm 24 Stunden geschüttelt.

Die ausgefallenen Teile der Testsubstanz werden mit der Biofuge 15 Fa. Heraeus 5 min bei 14000 upm abzentrifugiert. 1300 µl des Überstandes werden erneut mit der Microfuge Fa. Beckmann bei 45000 rpm = 125000g zentrifugiert.

10 µl dieses Zentrifugationsüberstandes werden nun mit 1 000µl DMSO verdünnt und diese Lösung an der HPLC gemessen. (Fa. Hewlett Packard 1090, Methode: Gradient von 100% PBS-Puffer pH=4 innerhalb von 15 min auf 10% Puffer/90% Acetonitril, Säule: RP 18)

Die gemessene Peakfläche der HPLC-Messung wird mit einer Eichgerade auf die Substanzkonzentration umgerechnet. Für die Eichgerade werden 20µl der Stammlösung sukzessiv mit DMSO so verdünnt, dass 5 Konzentrationen von 2.5 mg/l bis 2000mg/l entstehen. Diese Lösungen werden ebenfalls an der HPLC gemessen (Methode s. o.) und die Peakflächen gegen die Konzentrationen aufgetragen.

### 2. Hemmung der Adenosinaufnahme in Kaninchenerythrozyten durch die erfindungsgemäßen Verbindungen.

Die Fähigkeit von Substanzen das Adenosinaufnahme-System zu beeinflussen wird durch die Bestimmung der hemmenden Wirkung der Substanzen auf die funktionelle Adenosinaufnahme untersucht.

Für den funktionellen Adenosinaufnahme-Test wird eine Erythrozyten Präparation aus Kaninchenblut verwendet. Das Blut wird intravenös entnommen, als Anticoagulans wird Citrat (3ml Monovette 9NC Firma Sarstedt) verwendet. Das Blut wird 5 min bei 3000 g zentrifugiert und die Erythrozyten in 10 mM MOPS/0,9% NaCl Lösung pH 7,4 suspendiert. Die Suspension wird auf das Einhundertfache des ursprünglichen Blutvolumens verdünnt. Je 990 µl der Suspension werden mit 10 µl einer geeigneten Konzentration der zu untersuchenden Substanz vesetzt und 5 min bei 30°C inkubiert. Danach werden 5 µl einer 4 mM Adenosinlösung zugegeben und weitere 15 min bei 30°C inkubiert. Danach werden die Proben 5 min bei 3000 g zentrifugiert und 700 µl der Überstände mit 28 µl 70 %iger HClO₄ versetzt 30 min im Eisbad stehen gelassen, 3 min bei 16000 g zentrifugiert und 350 µl der Probe mit 30 µl 5N NaOH neutralisiert. 50 µl der Probe werden auf eine Säule (Waters Symmetry C18 5µm 3,9x150mm) aufgetragen. Als Vorsäule wird eine Spherisorb ODS II 5µm 4,6x10mm verwendet. Als Fließmittel wird ein Gradient aus 50 mM KH₂PO₄/5 mM Tributylamin pH 7 (FließmittelA) und einer Mischung Fließmittel A/Methanol 1/1 (FließmittelB) verwendet. Der Gradient wird von 10 - 40 % B bei einer Fließrate von 0,5 ml/min gefahren. Das vorhandene Adenosin wird durch seine Absorbtion bei 260 nm quantifiziert, ebenso das entstandene Inosin und Hypoxanthin. Der IC₅₀ bezeichnet die Konzentration des Wirkstoffs bei der 15 min nach Adenosinzugabe noch 50 % der ursprünglich eingesetzten Adenosinkonzentration vorhanden ist.

Nach diesem Test wurde für Beispiel 3 ein IC₅₀-Wert von 15 nM, für Beispiel 8 von 20 nM, für Beispiel 10 von 25 nM und für Beispiel 16 von 10 nM bestimmt.

### 3. In vivo Testmodell zur Prüfung von "Adenosin-reuptake-Inhibitoren"

Erwachsene FBI(Foxhound-Beagle-Irish-Setter)-Hunde (20-30kg) werden initial mit einer Kombination von Trapanal 500mg und Alloferin 55 mg narkotisiert. Die Narkose wird durch Infusion eines Gemisches von Fentanyl 0,072 mg/kg, Alloferin 0,02 mg/kg und Dihydrobenzpyridyl 0,25 mg/kg x min erhalten. Die Tiere werden intubiert und mit einem Gemisch aus O₂/N₂O 1/5 mit einer Engström Atempumpe mit 16 Atemzügen pro min und einem Volumen von 18-24 ml/kg beatmet. Die Körpertemperatur wird bei 38°C ± 0,1 °C gehalten. Der arterielle Blutdruck wird über einen Katheder in der Femoralarterie gemessen. Es wird eine Thorakotomie auf der linken Seite am fünften Intercostalraum durchgeführt. Die Lunge wird zurückgelegt, fixiert und das Pericard eingeschnitten. Ein proximaler Abschnitt der LAD distal zur ersten diagonalen Verzweigung wird freipräpariert und ein kalibrierter elektromagnetischer Flussmesskopf (Gould Statham, model SP7515) um das Gefäß gelegt und mit einem Flussmessgerät (Statham, model SP-2202) verbunden. Ein mechanischer Occluder wird distal zum Flussmesskopf so angebracht, dass keine Verzweigungen zwischen Flussmesskopf und Occluder liegen.

Blutentnahmen und Substanzgaben werden durch einen Katheder in der Femoralvene durchgeführt. Ein peripheres EKG wird mit subcutan verankerten Nadeln abgeleitet. Ein MikrotipDruckmanometer (Millarmodel PC-350) wird durch den linken Vorhof geschoben um den linksventrikulären Druck zu messen. Die Messung der Herzfrequenz wird über die R-Zacke des EKGs getriggert. Die hämodynamischen Parameter und der Koronarfluss werden während des gesamten Versuchs über einen Vielfachschreiber aufgezeichnet.

Eine Okklusion von vier Minuten verursacht eine reaktive Hyperämie. Man misst die Differenz zwischen dem Koronarfluss unter Kontrollbedingungen und dem Maximalfluss während der reaktiven Hyperämie. Die Zeit, die benötigt wird, um die Hälfte dieses Maximalflusses im Abfall zu erreichen, ist ein geeigneter Parameter, um die reaktive Hyperämie zu beurteilen.

Nach einer Stabilisierungszeit von einer Stunde wird das Experiment mit einer vierminütigen Okklusion begonnen. Dreißig Minuten später wird die Substanz gegeben (i.v.) und zwei Minuten später erneut occludiert. Die reaktive Hyperämie nach Verum und Placebo wird verglichen.

### 4. Maus-Angiogenese-Modell

Um die Wirkung von Adenosin Reuptake Inhibitoren auf die Collateralisierung und die Neovaskularisation zu prüfen, wurde ein Maus Modell zur Angiogenese entwickelt. Dabei wird der Maus eine Femoralarterie am oberen Ende des Oberschenkels ligiert. Dadurch wird eine chronische Ischämie der jeweiligen Hinterlaufs induziert. Der andere Hinterlauf dient als individuelle Kontrolle. Um einen Restfluss durch das ligierte Gefäß ausschließen zu können, werden zwei Ligaturen gelegt und das Gefäß zwischen diesen beiden durchtrennt. Wenige Tage nach dieser Operation wird die Behandlung gestartet.

Als Messparameter während des laufenden Versuches wird die Pfötchentemperatur beider Hinterläufe gemessen. Dabei weist der ischämische Hinterlauf aufgrund seiner schlechteren Durchblutung die niedrigere absolute Temperatur auf Errechnet wird jeweils die Temperaturdifferenz zwischen den Pfoten der Hinterläufe. Diese individuelle Temperaturdifferenz wird in verschiedenen Behandlungsgruppen Dosisabhängig und gegenüber einer unbehandelten Kontrolle bestimmt. Adenosin Reuptake Inhibitoren zeigen in diesem Modell eine signifikante Verbesserung der Durchblutung des ischämischen Hinterlaufs im Vergleich zu entsprechenden Kontrollen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal, parenteral, perlingual, intravenös; besonders bevorzugt oral oder intravenös.

Im allgemeinen hat es sich als vorteilhaft erwiesen, bei intravenöser Applikation Mengen von etwa 0,0001 bis 10 mg/kg, vorzugsweise etwa 0,003 bis 1 mg/kg Körpergewicht zur Erzielung wirksamer Ergebnisse zu verabreichen. Bei oraler Applikation werden 0,1 bis 20 mg/kg, vorzugsweise 0,3 - 3 mg/kg Körpergewicht verwendet.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Es kann empfehlenswert sein, diese Menge in mehreren Einzelgaben über den Tag zu verteilen.

### Ausgangsverbindungen

In den Beispielen werden die folgenden Abkürzungen für Lösungsmittel verwendet:
- DMF =: N,N-Dimethylformamid
- DMSO=: Dimethylsulfoxid
- PPA =: Polyphosphorsäure
- TFA =: Trifluoressigsäure
- THF =: Tetrahydrofuran

### Beispiel 1A

### (1R, 2R)-2-(4-Methyl-phenyl)-cyclohexan-1-carbonsäure

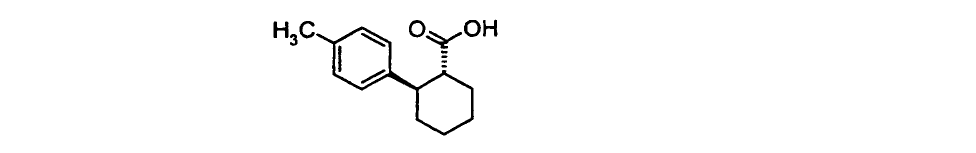

Racemische (1R*,2R*)-2-(4-Methyl-phenyl)-cyclohexan-1-carbonsäure wurde analog dem in US-A-5,395,840, Spalte16, beschriebenen Verfahren hergestellt. Das erhaltene racemische Material wurde nach folgender Vorschrift in die Enantiomeren getrennt:

Die racemische Säure (415 g; 1,9 mol) und Triethylamin (96,2 g; 0,95 mol; 131,8 mL) wurden in einem Gemisch aus THF (2,7 1) und Wasser (5,3 1) suspendiert. S-(-)-Phenethylamin (115,2 g; 0,95 mol) wurde bei 60 °C tropfenweise hinzugefügt, wobei ein Niederschlag ausfiel. Das Gemisch wurde 2 h bei 60 °C gerührt und anschließend mit einem Eisbad gekühlt. Dieser Niederschlag wurde abgesaugt und enthielt überwiegend das Phenethylaminsalz des (1*S*,2*S*)-Enantiomers. Das Filtrat wurde mit konz. HCl angesäuert und zweimal mit Dichlormethan extrahiert. Die vereinigten Extrakte wurden über Natriumsulfat getrocknet und eingeengt. Ausb.: 202,4 g (28%) eines mit dem (1*R*, 2*R*)-Isomer angereicherten Enantiomerengemisches.

Dieses Gemisch wurde wie oben beschrieben mit R-(+)-Phenethylamin behandelt, um das gewünschte Enantiomer als Salz zu fällen. Die farblosen Kristalle wurden abgesaugt und aus Acetonitril/Methanol (6:1) umkristallisiert. Die Röntgenstrukturanalyse dieser Kristalle ergab die (1*R*,2*R*)-Konfiguration. Ausb. 136,9 g (46%). Nach der Aufarbeitung (s.o.) wurden 89 g (1*R*,2*R*)-2-(4-Methylphenyl)-cyclohexan-1-carbonsäure erhalten.

### Beispiel 2A

### (1R,2R)-2-(4-Bromomethyl-phenyl)-cyclohexan-1-carbonsäure-tert-butylester:

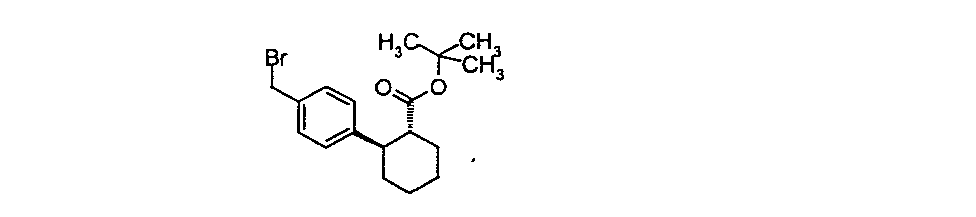

Das Zwischenprodukt wurde in Analogie zur Vorschrift für das Racemat (US-A-5 395 840, Spalte 17) hergestellt. Zur Aufreinigung wurde das erhaltene Gemisch mit Diethylether verrührt.

### Beispiel 3A

### 2-(2-Phthalimidylethyl)-1H-benzimidazol

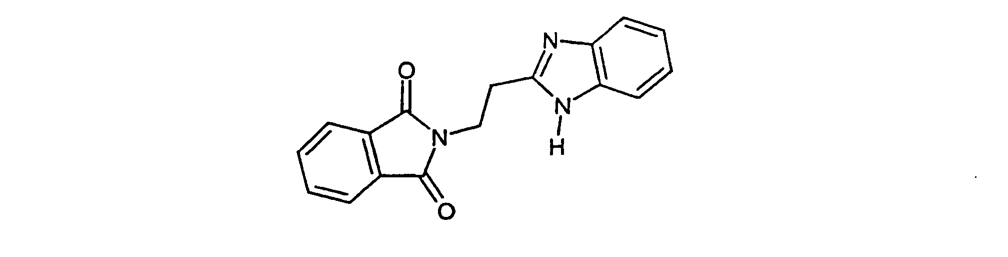

2-Aminoethylbenzimidazol-Dihydrochlorid (*Bull. Soc. Chim. Fr*. **1991**, *128*, 255-259; 2,34 g, 10 mmol), Phthalsäureanhydrid (1,63 g, 11 mmol) und Triethylamin (2,79 mL, 20 mmol) wurden über Nacht in Chloroform (25 mL) zum Rückfluss erhitzt, danach auf Raumtemperatur abgekühlt, mit Essigester verdünnt und abfiltriert. Das Filtrat wurde mit gesättigter Natriumcarbonatlösung, Puffer (pH=7) und gesättigter Kochsalzlösung gewaschen und über Natriumsulfat getrocknet. Chromatographie (Dichlormethan:Methanol 10:1, R_{f}=0,4) lieferte 2,08 g 2-(2-Phthalimidylethyl)-benzimidazol (71,4% der Theorie) als farblosen Schaum. MS(DCI, NH₃)=292 (M+H⁺). ¹H-NMR(DMSO-d₆): 3,15 (2 H, t); 4,0 (2 H, t); 7,05-7,2 (2 H, m); 7,4-7,5 (2 H, m); 7,8-7,9 (4 H, m); 12,4 (1 H, br s).

Im weiteren Verlauf der Synthese wird nach den allgemeinen Arbeitsvorschriften A, B und C wie unten angegeben verfahren und in einem letzten Schritt wie weiter unten beschrieben die Phthalimidgruppe abgespalten.

### Beispiel 4A

### 2-(2-Hydroxyethoxymethyl)-pyrido[2,3-d]-1H-imidazol

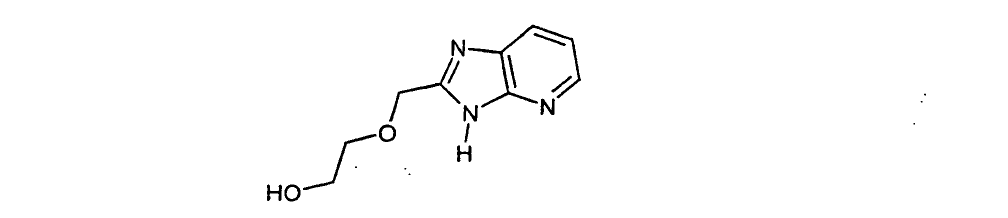

1,4-Dioxan-2-on (6,13 g, 60 mmol) und 2,3-Diaminopyridin (5,46 g, 50 mmol) wurden am Wasserabscheider während 10 h in Mesitylen (100 mL) auf Rückfluss erhitzt. Nach dem Abkühlen wurde Mesitylen abdekantiert und der Rückstand durch Chromatographie über Kieselgel (Dichlormethan:Methanol 9: 1) gereinigt (Ausbeute: 8,47 g, 87% der Theorie).
MS(DCI)=194 (M+H, 100%); ¹H-NMR(DMSO-d₆): 3,78 (2 H, m); 3,89 (2H, m); 4,91 (2 H, s); 5,3 (1 H, s); 7,18 (1 H, dd); 7,95 (1 H, d); 8,43 (1 H, dd); 12,7 (1 H, br s).

### Beispiel 5A

### 2-[2-(tert-Butyldimethylsilyloxy)ethoxymethyl]-pyrido[2,3-d]-1H-imidazol

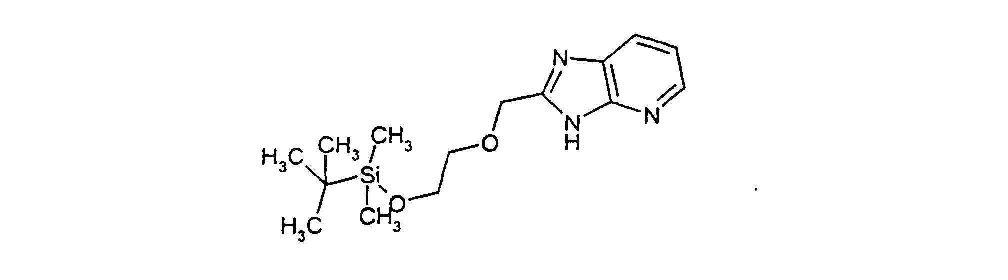

8,4 g (43,48 mmol) 2(2-Hydroxyethoxymethyl)-(pyrido-[2,3-d]-*1H*-imidazol) und 4,84 g (47,82 mmol) Triethylamin wurden in 120 mL DMF gelöst und mit 7,21 g (47,8 mmol) TBDMS-Chlorid versetzt, wobei sich das Gemisch auf ca. 40 °C erwärmte. Nach dem noch 2 h bei Raumtemperatur gerührt wurde, goss man das Gemisch auf Wasser, wobei das Produkt kristallin anfiel. Man saugte es ab, wusch mit wenig Wasser nach und trocknete im Hochvakuum. ¹H-NMR(DMSO-d₆): 0,02 (6H, s); 0,83, (9H, s); 3,52 (2 H, t); 3,75 (2H, t); 4,73 (2 H, s); 7,18 (1 H, dd); 7,90 (1 H, dd); 8,43 (1 H, dd); 12,9 (1 H, br s).

### Beispiel 6A

### 2-tert-Butyldimethylsilyloxymethyl-1H-benzimidazol:

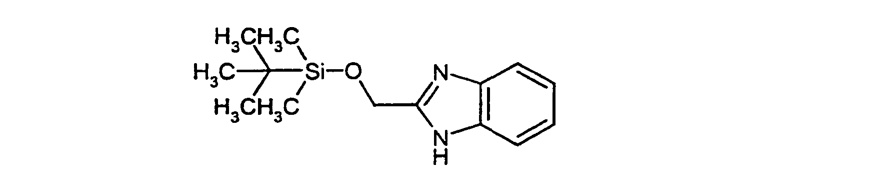

Zu einer Lösung aus 2-Hydroxymethylbenzimidazol (1,48 g, 9,95 mmol) in DMF (30 mL) wurde bei Raumtemperatur Triethylamin (2,27 mL, 16,3 mmol) und TBDMS-Chlorid (1,65 g, 10,95 mmol) gegeben. Nach 3,5 h wurde die Reaktion durch Zugabe von Wasser abgebrochen, mit Diethylether extrahiert, und die organische Phase über Natriumsulfat getrocknet. Chromatographie (Kieselgel, Cyclohexan:Essigester 2:1, R_{*f*}=0,35) lieferte 2,52 g 2-*tert*-Butyldimethylsilyloxymethylbenzimidazol (97% der Theorie) als bräunliches Pulver. MS(DCI, NH₃)=263 (M+H⁺). ¹H-NMR(DMSO-d₆): 0,00 (6 H, s); 0,80 (9 H, s); 4,75 (2 H, s); 7,0-7,1 (2 H, m); 7,4-7,5 (2 H, m); 12,15 (1 H, br s).

### Beispiel 7A

### 2-(2-Hydroxyethoxymethyl)-1H-benzimidazol:

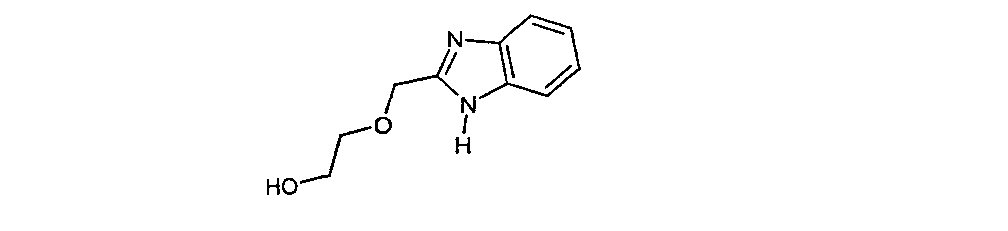

1,4-Dioxan-2-on (2,04 g, 20 mmol) und 1,2-Diaminobenzol (2,16 g, 20 mmol) wurden am Wasserabscheider während 10 h in Mesitylen (150 mL) auf Rückfluss erhitzt. Anschliessend wurden die beim Abkühlen entstandenen Kristalle abgenutscht (2,94 g, 77% der Theorie). R_{*f*} (Dichlormethan:Methanol 10:1) =0,45, MS(EI)=192 (M⁺, 20%), 148 (20%), 147 (40%), 132 (100%). ¹H-NMR(DMSO-d₆): 3,6 (4 H, s); 4,65 (1 H, s); 4,7 (2 H, s); 7,1-7,2 (2 H, m); 7,45 (1 H, d); 7,55 (1 H, d); 12,4 (1 H, br s).

### Beispiel 8A

### 2-(3-{[tert-Butyl(dimethyl)silyl]oxy}propyl)-1H-benzimidazol

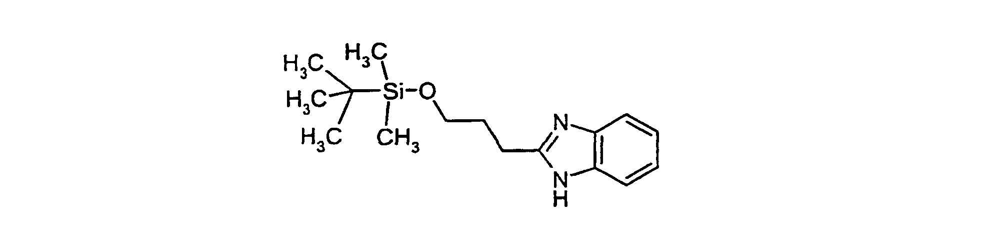

Die Silylierung von 2-(3-Hydroxypropyl)-1*H*-benzimidazol erfolgte analog dem in Beispiel 6A aufgeführten Verfahren.
Hierzu wurde zu einer Lösung von 2-(3-Hydroxypropyl)-benzimidazol (15.60 g, 88.52 mmol) in DMF (265 ml) und Triethylamin (20.27 ml, 145.41 mmol) portionsweise tert.-Butyldimethylsilylchlorid (TBDMSCI) (14.68 g, 97.37 mmol) zugegeben.

Die Mischung wurde über Nacht bei RT gerührt, anschließend mit Wasser (800 ml) versetzt und mit Diethylether extrahiert. Nach Trocknung der organischen Phase über Natriumsulfat und Chromatographie an Kieselgel (Eluens: Cyclohexan/Ethylacetat = 6:1) wurden 25 g (97 %) farbloser Kristalle erhalten.
MS (DCI): 291 (M+H)⁺
¹H-NMR (CDCl₃): 0.12 (6 H, s); 0.92 (9 H, s); 2.08 (2 H, m); 3.10 (2 H, t); 3.80 (2 H, t); 7.20 (2 H, m); 7.52 (2 H, br. s).

### Beispiel 9A

### (S)-(4-Fluorphenyl)glycinamid-Hydrochlorid

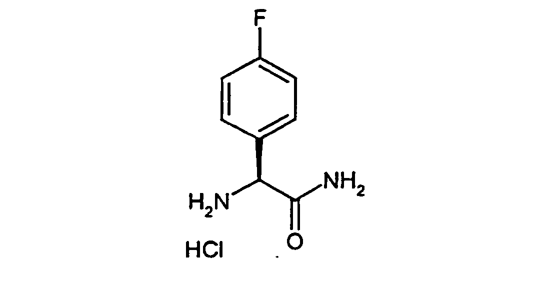

### Weg 1:

### a) (S)-N-(tert-Butoxycarbonyl)-(4-fluorphenyl)glycin

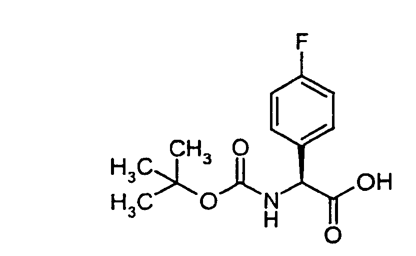

(*S*)-(4-Fluorphenyl)glycin (35 g, 207 mmol) und Natriumcarbonat (54.8 g, 517 mmol) wurden in Wasser (150 mL) und Tetrahydrofuran (75 mL) vorgelegt und bei Raumtemperatur Pyrokohlensäure-di-*tert*-butylester (Boc-Anhydrid) (52.3 ml, 228 mmol) hinzugetropft. Das Reaktionsgemisch wurde über Nacht bei RT gerührt. Die Reaktionslösung wurde mit 5N Salzsäure auf einen pH-Wert von 3 eingestellt, mit Dichlormethan und Wasser ausgeschüttelt und die wässrige Phase noch zweimal mit Dichlormethan extrahiert. Die vereinten organischen Phasen wurden über Natriumsulfat getrocknet, filtriert und das Solvens im Vakuum entfernt. Das Produkt (54.2 g, 97 % der Theorie) wurde ohne weitere Reinigung umgesetzt.
MS (ESI)= 561 (2M+Na⁺, 40 %), 292 (M+Na⁺, 100 %), 236 (40 %), 214 (70 %), 153 (95 %).
¹H-NMR (DMSO-d₆): 1,38 (9 H, s); 5,11 (1 H, br. d); 7,12-7,21 (2 H, m); 7,39-7,47 (2 H, m); 7,56 (1 H, br. d); 12,78 (1 H, br. s).

### b) (S)-N-(tert-Butoxycarbonyl)-(4-fluorphenyl)glycinamid

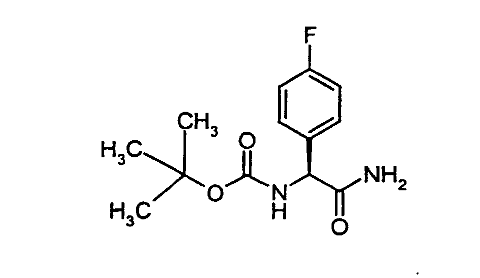

Das Produkt aus Beispiel 9A a) (54.2 g, 201 mmol) wurde unter Argon in Tetrahydrofuran (600 mL) gelöst, auf -15°C (Innentemperatur) gekühlt und nacheinander Triethylamin (201 mmol, 28.1 mL) sowie Chlorameisensäureisobutylester (201 mmol, 26.1 mL) über 5 min zugegeben. Nach 25min. Rühren bei -15°C wurde Ammoniak in Methanol (126 mL 2N Lösung) hinzugefügt und das Reaktionsgemisch 30 min bei dieser Temperatur gerührt. Das Gemisch wurde filtriert, der Filterrückstand in Dichlormethan gelöst und mit Wasser ausgeschüttelt. Die organische Phase wurde noch zweimal mit Natriumcarbonatlösung gewaschen, über Natriumsulfat getrocknet und das Solvens im Vakuum entfernt. Der Rückstand wurde mit Petrolether versetzt und bei 4°C über Nacht auskristallisiert. Nach Filtration und Nachwaschen mit Petrolether wurde das Produkt (33.9 g, 63 % der Theorie) als Feststoff isoliert.

R_{f} (Methanol : Dichlormethan = 1:10) = 0.46
MS (DCI/NH₃)= 269 (M+H⁺, 60 %), 169 (100 %).
¹H-NMR (DMSO-d₆): 1,37 (9 H, s); 5,09 (1 H, br, d); 7,1-7,3 (4 H, m); 7,37-7,51 (2
H, m); 7,57 (1 H, br. s).

### c) (S)-(4-Fluorphenyl)glycinamid-hydrochlorid

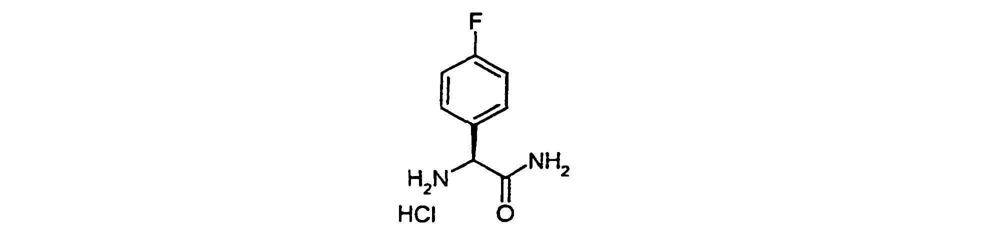

Das Produkt aus Beispiel 9A b) (33.8 g, 126 mmol) wurde in einer Lösung von Chlorwasserstoff in 1,4-Dioxan (250 mL 4N Lösung) und 1,4-Dioxan (100 mL) gelöst und 2 h bei RT gerührt. Der entstandene Niederschlag wurde abgesaugt, mit Diethylether nachgewaschen und nach der Trocknung im Vakuum nochmals mit Diethylether verrieben, abgesaugt und im Vakuum getrocknet. Das Produkt wurde als farbloser Feststoff (24.3 g, 94.4 % der Theorie) mit einer Enantiomerenreinheit >99 % ee isoliert.
MS (DCI/NH₃)= 337 [2(M-HCl)+H⁺, 20 %], 169 [(M-HCl)+H⁺, 100 %].
¹H-NMR (DMSO-d₆): 4,97 (1 H, s); 7,2-7,4 (2 H, m); 7,5-7,7 (2 H, m); 8,13 (1H, s); 8,80 (3 H, s).

### Weg 2:

Alternativ zu Weg 1 konnte die Verbindung aus Beispiel 9A c), d.h. das (*S*)-(4-Fluorphenyl)glycinamid-hydrochlorid, nach folgendem Schema hergestellt werden:

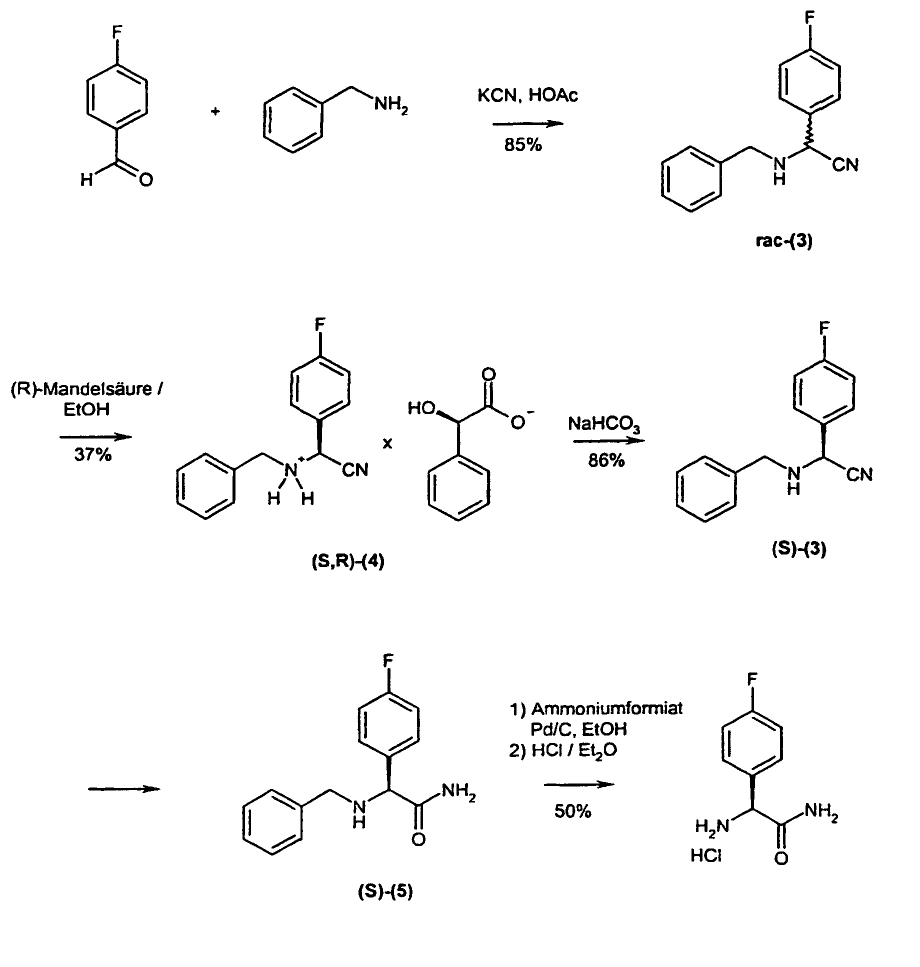

### i) rac-(2-Benzylamino)-2-(4-fluorphenyl)acetonitril [rac-(3)]

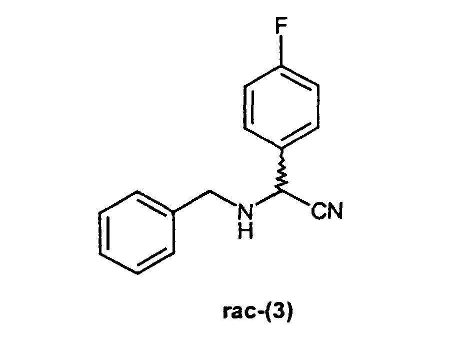

Zunächst erfolgt die Umsetzung von 4-Fluorbenzaldehyd mit Benzylamin in essigsaurer Cyanid-Lösung in einer sogenannten Strecker-Reaktion, die unter den Aminosäuresynthesen zu den Standard-Reaktionen gehört (siehe hierzu exemplarisch die vier Literaturstellen: 1.) Hassan, N.A., Bayer, E., Jochims, J.C., *J.Chem.Soc. Perkin 1* **1998**, 3747-3757; 2.) Georgiadis, M.P., Haroutounian, S.A., *Synth. Comm.* **1989**, 616; 3.) Alabaster, R.J., Gibson, A.W., Johnson, S.A., Edwards, J.S., Cottrell, I.F., *Tetrahedron Asymmetry* **1997**, *8*, 447-450, darin zitiert: Alabaster, R.J.; Cottrell, I.F.; Gibson, A.W.; Johnson, S.A.: UK-Patent Appl. 9511031.8; 4.) Inaba, T., Fujita, M., Ogura, K., *J.Org.Chem*. **1991**, *56*, 1274-1279).

Unter den in Literaturstelle 1.) (Hassan, N.A., Bayer, E., Jochims, J.C., J.Chem.Soc. Perkin 1 1998, 3747-3757) beschriebenen Bedingungen wurde das racemische (2-Benzylamino)-2-(4-fluorophenyl)acetonitril [rac-(3)] mit einer Ausbeute von 85% hergestellt.

### ii) (S)-Benzyl{cyano(4-fluorphenyl)methyl}ammonium-(R)-amygdalat [(S,R)-(4)]

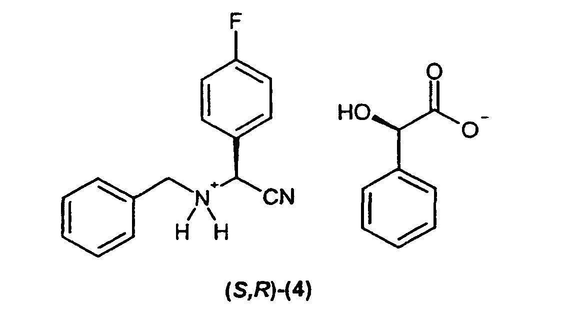

Das racemische Produkt aus Beispiel 9A i) wurde dann in Ethanol gelöst und mit (R)-Mandelsäure versetzt, um in einer kinetischen Racemattrennung das diastereomerenreine (*S*)-Benzyl[cyano(4-fluorphenyl)methyl)aminonium-(*R*)-amygdalat zu isolieren (siehe obige Literaturstelle 1.), *J.Chem.Soc. Perkin 1* **1998,** 3747-3757). Hierbei wurden 63 % des diastereomerenreinen Produktes isoliert.

### iii) (2S)-N-Benzyl-(4-fluorphenyl)acetonitril [(S)-(3)]

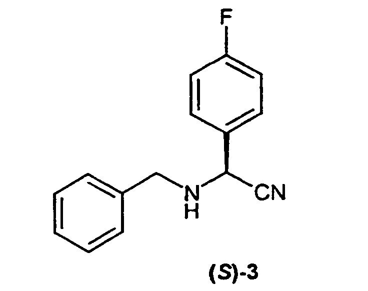

Aus dem Produkt von Beispiel 9A ii) wurde in 87%iger Ausbeute das enantiomerenreine (2*S*)-*N*-Benzyl-(4-fluorphenyl)acetonitril gewonnen (siehe Literaturstelle 1.), *J.Chem.Soc. Perkin 1* **1998**, 3747-3757).

### iv) (2S)-N-Benzyl-(4-fluorphenyl)glycinamid

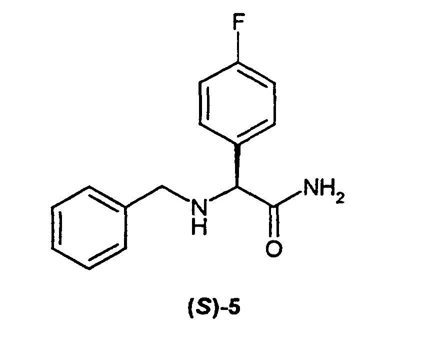

Das enantiomerenreine (2*S*)-*N*-Benzyl-(4-fluorphenyl)acetonitril aus Beispiel 9A iii) wurde mit konz. Schwefelsäure in 97%iger Ausbeute zum (2*S*)-*N*-Benzyl-(4-fluorphenyl)glycinamid hydrolysiert (siehe Literaturstelle 1.), *J.Chem.Soc. Perkin 1* **1998**, 3747-3757).

Für die Umwandlung von *rac**-*****(3)** in racemisches *N*-Benzyl-(4-fluorphenyl)glycinamid ist im Stand der Technik alternativ auch eine Methode mit H₂O₂/K₂CO₃ beschrieben (siehe Literaturstelle 3.), Alabaster, R.J., Gibson, A.W., Johnson, S.A., Edwards, J.S., Cottrell, I.F., *Tetrahedron Asymmetry* **1997,** 8, 447-450, darin zitiert: Alabaster, R.J.; Cottrell, I.F.; Gibson, A.W.; Johnson, S.A.: UK-Patent Appl. 9511031.8).

### v) (S)-(4-Fluorphenyl)glycinamid-hydrochlorid

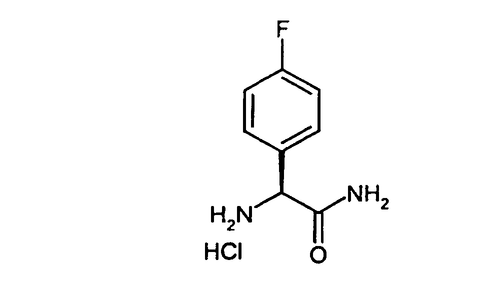

Zur bislang nicht beschriebenen Debenzylierung des Produktes aus Beispiel 9A iv) wurde zu einer Lösung dieses Produktes (1,5 g, d.h. 5,8 mmol (2*S*)-*N*-Benzyl-(4-fluorphenyl)glycinamid) in Ethanol (37,5 ml) 50% wasserfeuchte 5%ige Palladiumkohle (1,5 g) und Ammoniumformiat (1,5 g, 23,8 mmol) sowie Wasser (18,8 ml) zugesetzt und diese Mischung auf Rückflusstemperatur erwärmt. Nach 2 h am Rückfluss wurde die Mischung filtriert, die Palladiumkohle mit kaltem Ethanol (zweimal 3 ml) gewaschen und die Lösung eingeengt. Das Rohprodukt wurde mit wenig Essigester verrührt, erneut abgesaugt und bis zur Trockenheit eingeengt. Der Eindampfrückstand wurde mit 1 N HCl in Ether versetzt und bis zur Trockenheit eingeengt. Das farblose Produkt wurde im Vakuum getrocknet. Dabei wurden 0,6 g des Produktes (*S*)-4-Fluorphenylglycinamid-Hydrochlorid (50% d.Th.) isoliert.
Die MS- sowie ¹H-NMR-spektroskopischen Daten stimmten mit den oben in Beispiel 9A c) angegebenen überein.

### Allgemeine Vorschrift zur Alkylierung [A]:

In einem typischen Ansatz wurde bei 0°C Natriumhydrid (6,3 mmol) zu einer Lösung des Imidazols der allgemeinen Formel (III) (6 mmol) in trockenem DMF (30 mL) gegeben. Nach 30 min bei Raumtemperatur und 30 min bei 40°C wurde bei 0°C die Verbindung der allgemeinen Formel (II) (6,3 mmol) zugegeben und das Reaktionsgemisch über Nacht bei Raumtemperatur gerührt. Die Reaktion wurde daraufhin durch Zugabe von Wasser abgebrochen, mit Diethylether extrahiert, und die organische Phase anschliessend über Natriumsulfat getrocknet. Nach Chromatographie (Kieselgel, Cyclohexan:Essigester) wurde Produkt in 60-90% Ausbeute erhalten.
Alternativ zu der Umsetzung des Imidazols mit Natriumhydrid in DMF kann die Umsetzung auch mit Natriumhydroxid in THF durchgeführt werden.

### Allgemeine Vorschrift zur Esterspaltung [B]:

In einem typischen Ansatz wurde bei Raumtemperatur Trifluoressigsäure (5 mL) zu einer Lösung aus Ester der allgemeinen Formel (IV) (T= *tert-Bu;* 1,5 mmol) in Dichlormethan (5 mL) gegeben. Nach 2 h wurde auf 0°C gekühlt, mit wässriger Natriumhydroxidlösung (ca. 30 mL, 2 M) auf pH=2 gestellt und mit Dichlormethan extrahiert. Trocknen der organischen Phase über Natriumsulfat lieferte nach Einengen die Verbindung der allgemeinen Formel (V).

### Allgemeine Vorschrift zur Amidbildung [C]:

Eine Suspension aus Säure (V) (4 mmol), *(S)*-Phenylglycinamid-Hydrochlorid (4,2 mmol), 1-Hydroxybenzotriazol (4,4 mmol), EDC-Hydrochlorid (4,8 mmol) und Triethylamin (12 mmol) in Dichlormethan oder Dimethylformamid (DMF) (40 mL) wurde während 24-48 h bei Raumtemperatur gerührt. Nach Zugabe von Wasser wurde mit Dichlormethan (z.T. mit Methanol) extrahiert, die organische Phase über Natriumsulfat (oder Magnesiumsulfat) getrocknet und chromatographiert (Kieselgel, Dichlormethan:Methanol). Es wurde gewünschtes Produkt in 60-80% Ausbeute erhalten.
Analog kann nach Vorschrift C statt Phenylgylcinamid Phenylglycinol eingesetzt werden.

### Herstellungsbeispiele

### Beispiel 1

### (S)-N-{(1R*, 2R*)-{4-[2-(2-Aminoethyl-benzimidazol-1-yl)methyl]phenyl}-cyclohex-2-yl-carbonyl}-phenylglycinamid

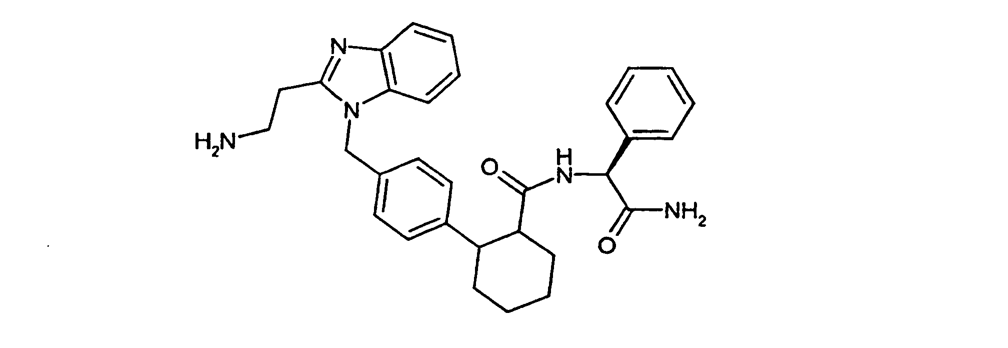

Zu einer Suspension aus *(2S)*-N-[*(2R*)*-(4-{2-(2-Phthaloylaminoethyl)-benzimidazol-1-yl-methyl)-phenyl)-cyclohexyl-*(1R*)*-carbonyl]-phenylglycinamid (hergestellt nach den allgemeinen Vorschriften [A - C] aus der Verbindung des Beispiels 3A und dem Racemat von Beispiel 2A gemäß US-A-5,395,840, Example IV; 500 mg, 0,78 mmol, Diastereomerengemisch) in Ethanol (25 mL) wurde Hydrazinhydrat (0,38 mL, 7,82 mmol) gegeben. Das Gemisch wurde über Nacht bei Raumtemperatur gerührt, dann mit Salzsäure (1 M) auf pH=2 gestellt und eingeengt. Extraktion zwischen 10% wässriger Natriumbicarbonatlösung und Dichlormethan, Trocknen der organischen Phase über Natriumsulfat und Chromatograpie (Kieselgel, Dichlormethan: Methanol:konz. wässriger Ammoniak 100:13:1,3, R_{f}(10:1:0,2)=0,1) lieferte die Titelverbindung (292 mg, 72%, Diastereomerengemisch) als gelbliches Pulver. MS(DCI, NH₃)=510 (M+H⁺). ¹H-NMR(DMSO-d₆): 1,2-1,5 (4 H, m); 1,6-1,9 (4 H, m); 2,0 (2 H, br s); 2,6-3,0 (6 H, m); 5,1-5,2 (A: 1 H, d; B:1 H, d); 5,4-5,5 (A:2 H, s; B:2 H, s); 6,85-7,0 (4 H, m); 7,1-7,3 (7 H, m); 7,4-7,5 (1 H, m); 7,55-7,65 (4 H, m); 8,05-8,15 (A: 1 H, d; B:1 H, d).

### Beispiel 2

### (S)-N-{(1R, 2R)-{4-{[2-(2-Aminoethyl)-benzimidazol-1-yl]methyl}phenyl}-cyclohex-1-yl-carbonyl}phenylglycinamid-Dihydrochlorid

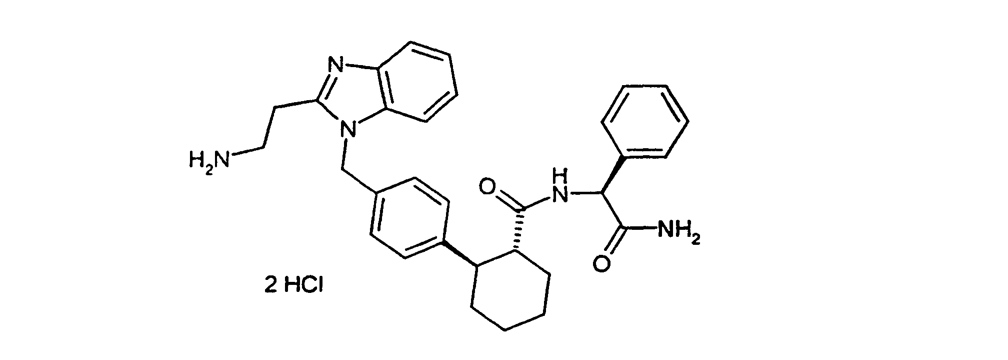

Chromatographische Trennung des Eduktes von Beispiel 1 (Kieselgel, Methylenchlorid: Methanol) lieferte diastereomerenreines *(S)*-(N)-{(1*R*, 2*R*)-2-{4{2-[2(Phthaloyl-amino)-ethyl]-benzimidazol-1-yl-methyl}-phenyl}-cyclohex-1-yl-carbonyl}-phenylglycinamid, das analog Beispiel 1 entschützt wurde und dann in einer möglichst geringen Menge Dichlormethan vollständig in Lösung gebracht und mit etwa 2 Äquivalenten 1M-HCl in Diethylether behandelt und eingeengt wurde.
Gef.: C 64,21 H 6,58
Ber.: C 63,91 H 6,49

### Beispiel 3

### (S)-N-{{(1R, 2R)-{4-{2-[2-(Morpholin-4-yl-methyl)-1H-pyrido[2,3-d]imidazol-1-yl]methyl}-phenyl}-cyclohex-1-yl}carbonyl}-phenylglycinamid

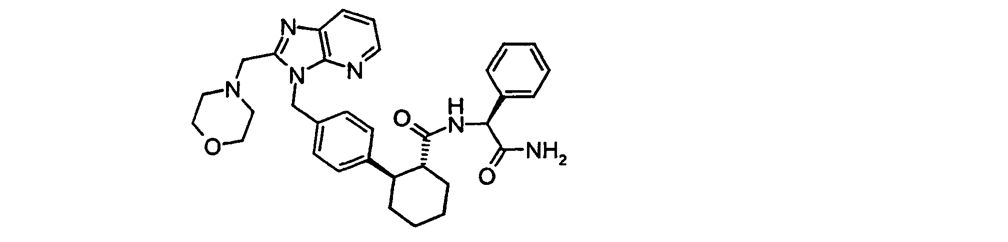

### a) 2-Hydroxymethyl-1H-pyrido[2,3-d]imidazol

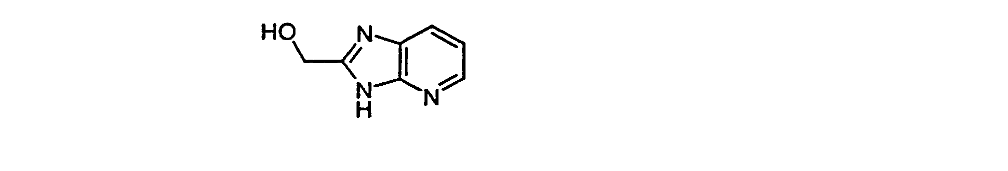

2,3-Diaminopyridin (54,6 g; 0,5 mol) und Glykolsäure (38 g; 0,5 mol) wurden in 700 mL Mesitylen am Wasserabscheider unter Rückfluss gekocht, bis sich die berechnete Menge Wasser abgeschieden hatte. Anschließend wurde das Gemisch auf Raumtemperatur gekühlt, der entstandene Niederschlag abgesaugt und 15 min in 800 mL Wasser unter Zusatz von Aktivkohle gekocht. Nachdem die Suspension heiß filtriert und wieder auf Raumtemperatur abgekühlt wurde, fielen farblose Kristalle aus, die abgesaugt und getrocknet wurden. Ausbeute: 56,4 g (75%).

### b) 2-Chlormethyl-1H-pyrido[2,3-d]imidazol Hydrochlorid:

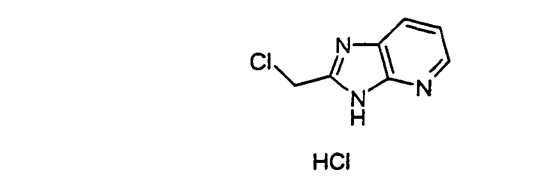

Die Verbindung aus Beispiel 3a (14,9 g; 100 mmol) wurde in 25 mL Ethanol suspendiert und ein trockener HCl-Strom bis zur Sättigung eingeleitet. Das erhaltene Hydrochlorid wurde abgesaugt und im Vakuum getrocknet. Ausb. 18,1 g (100%). Dieses wurde in 100 mL Chloroform suspendiert und mit 35 mL Thionylchlorid versetzt. Anschließend wurde das Gemisch 24 h unter Rückfluss erhitzt, dann heiß filtriert und der Niederschlag mit Chloroform gewaschen und im Vakuum getrocknet. Ausb. 18,9 g (92%).

### c) 2-(Morpholin-4-yl-methyl)-1H-pyrido[2,3-d]imidazol:

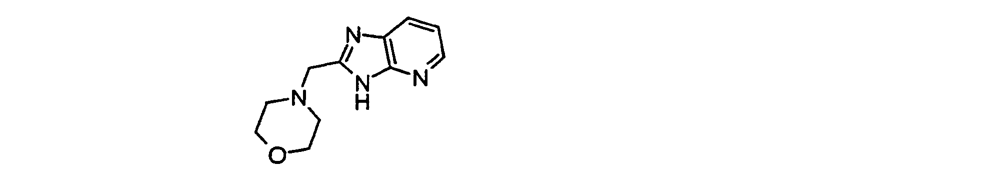

Die Verbindung aus Beispiel 3b (13,7 g; 67 mmol) und Morpholin (28,6 g; 328 mmol) wurden 3 h unter Rückfluss gekocht. Das Gemisch wurde eingeengt und der Rückstand mit Natriumhydrogenkarbonatlösung aufgenommen. Diese Suspension wurde 15 min unter Zusatz von Aktivkohle gekocht und anschließend heiß filtriert. Nachdem das Gemisch eingeengt wurde, reinigte man das entstandene Produkt durch Säulenchromatographie (Kieselgel (70-230 mesh ASTM); Eluent: 100:30:1 Essigester/ Ethanol/ Triethylamin). Das Produkt kann aus Essigester/Hexan umkristallisiert werden.

### d) (1R,3R)-{4-{[2-(Morpholin-4-yl-methyl)-1H-pyrido[2,3-d]imidazol-1-yl]-methyl}phenyl}-cyclohexan-1-carbonsäure-tert-butylester

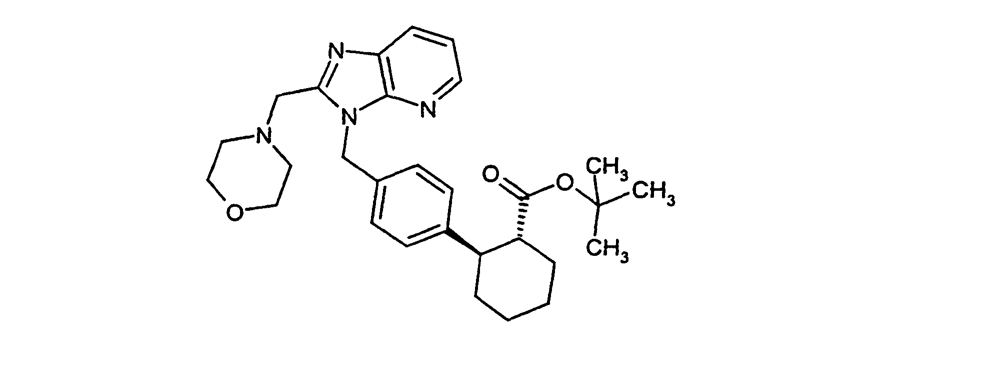

Eine 60%ige Suspension von Natriumhydrid in Öl (2 g; 51,6 mmol) wurde unter Argon in 150 mL DMF suspendiert und die Verbindung aus Beispiel 3 c) (9,5 g; 43,5 mmol) hinzugefügt. Das Gemisch wurde 30 min auf 50 °C erwärmt, wobei sich ein Niederschlag bildete. Anschließend wurde auf Raumtemperatur gekühlt und die Verbindung aus Beispiel 2A (17,3 g; 44 mmol) hinzugefügt, wonach das Gemisch 20 h bei Raumtemp. gerührt wurde. Die entstandene klare Lösung wurde im Hochvakuum eingeengt und der Rückstand mit Dichlormethan/ Wasser aufgenommen. Die organische Phase wurde abgetrennt, über Natriumsulfat getrocknet und eingeengt. Der Rückstand wurde anschließend durch Säulenchromatographie gereinigt (Kieselgel (70-230 mesh ASTM); Eluent: 100:4 Dichlormethan/ Methanol) Ausb. 10 g (47%) eines braunen, viskosen Öls.

### e) (1R,2R)-2-{4-{[2-(Morpholin-4-yl-methyl)-1H-pyrido[2,3-d]imidazol-1-yl]-methyl}phenyl}cyclohexan-1-carbonsäure

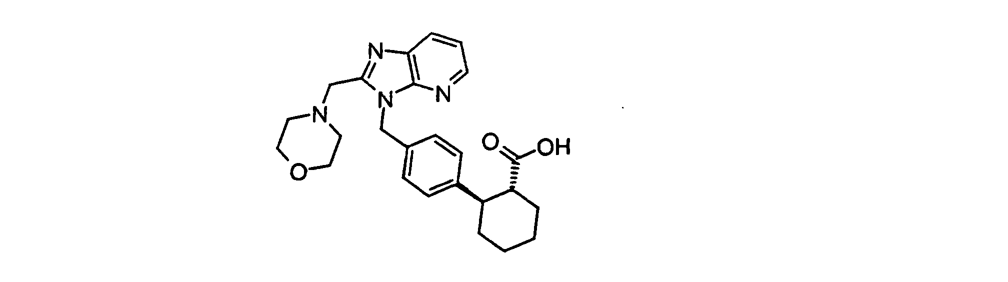

Die Verbindung aus Beispiel 3d (10 g; 20,4 mmol), 120 mL Dichlormethan und 100 mL Trifluoressigsäure wurden 1 h bei Raumtemperatur gerührt. Anschließend wurde das Gemisch unter Kühlung mit konzentrierter Natronlauge neutralisiert, die organische Phase abgetrennt, getrocknet und eingeengt. Der Rückstand wurde durch Säulenchromatographie gereinigt. (Eluent: Dichlormethan/ Methanol 100:6) Ausb. 7,3 g (80%) eines farblosen amorphen Festkörpers.

### f) (S)-N-{{(1R,2R)-2-{4-{[2-(Morpholin-4-yl-methyl)-1H-pyrido[2,3-d]imidazol-1-yl]methyl}-phenyl}-cyclohex-1-yl}carbonyl}-phenylglycinamid

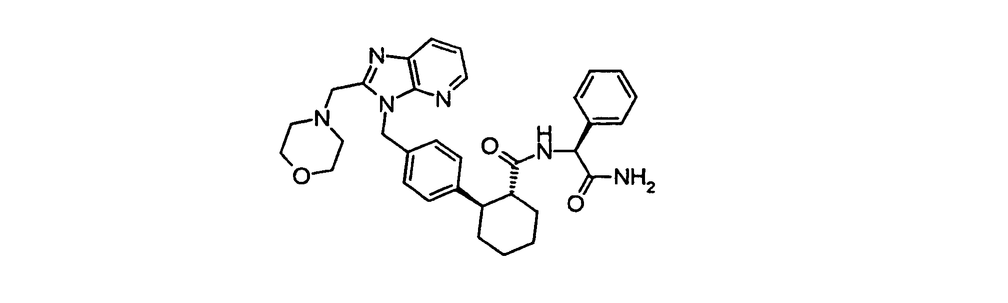

Gemäß dem allgemeinen Verfahren [C] wurde die Verbindung von Beispiel 3e (1,4 g; 3,22 mmol) unter Zusatz einer Spatelspitze DMAP (4-Dimethylaminopyridin) umgesetzt. Zur Aufarbeitung wurde das Produkt mit Dichlormethan extrahiert und durch Säulenchromatographie gereinigt (Dichlormethan/ Methanol 100:6) Ausb. 1,7 g (93%) eines blassgelblichen Pulvers.
¹H-NMR (300 MHz; CDCl₃) δ[ppm]: 1,25-1,5 (3H; br m), 1,62 (1H; dq), 1,8 (3H; m), 1,94 (1H; dd), 2,31 (1H; dt), 2,42 (4H, br m), 2,67 (1H; dt), 3,61 (6H; m), 5,21 (1H; d), 5,49 (1H, br s), 5,63 (2H; d+d), 5,72 (1H; br s), 6,41 (1H; d), 6,82 (2H; d), 6,92 (2H; d), 6,98 (2H; d), 7,13 (2H; t), 7,18 (1H; t), 7,23 (1H; dd), 8,03 (1H; d), 8,42 (1H; d)
MS (DCI/NH₃)[m/z]: 567 (100, M+H)

### Beispiel 4

### (S)-N-{{(1R, 2R)-{4-{2-[2-(Morpholin-4-yl-methyl)-1H-pyrido[2,3-d]imidazol-1-yl]methyl}-phenyl}-cyclohex-1-yl}carbonyl}-phenylglycinamid Hydrochlorid

Die Verbindung von Beispiel 3 wurde in einer möglichst geringen Menge Dichlormethan vollständig in Lösung gebracht und mit etwa 2 Äquivalenten 1M-HCl in Diethylether behandelt. Der entstandene Niederschlag wurde abgesaugt [Fp. 158°C (Zers.)].

### Beispiel 5

### (S)-N-{{(1R, 2R)-2-{4-{[2-(4-Methyl-piperazin-1-yl)-benzimidazol-1-yl]methyl}-phenyl}-cyclohex-1-yl}carbonyl}-phenylglycinamid

### a) (1R, 2R)-2-{4-[(2-Chloro-benzimidazol-1-yl)methyl]-phenyl}-cyclohexan-1-carbonsäure-tert-butylester

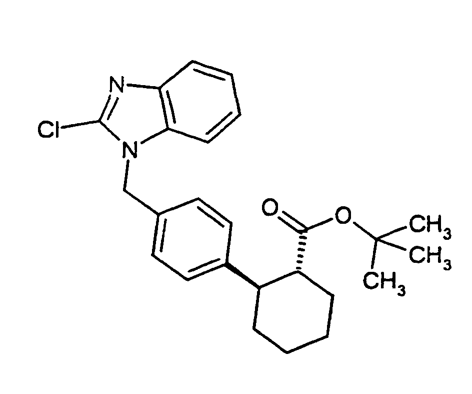

Entsprechend allgemeiner Vorschrift [A] wurde die Titelverbindung aus 2-Chlorobenzimidazol und der Verbindung aus Beispiel 2A hergestellt [R_{*f*} (Cyclohexan:Essigester = 1:1) = 0,85].

### b) (1R,2R)-2-{4-{[2-(4-Methyl-piperazin-1-yl)-benzimidazol-1-yl]methyl}-phenyl}-cyclohexan-1-carbonsäure

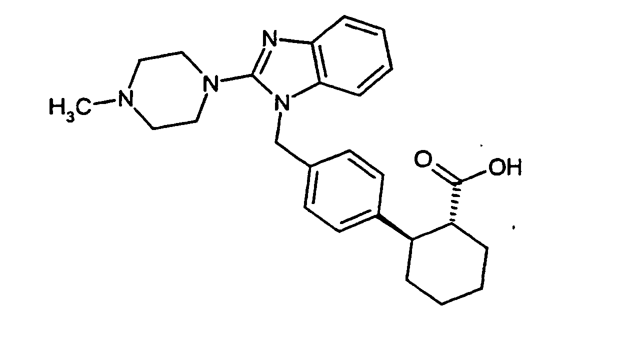

Eine Lösung der Verbindung von Beispiel 5a (34,0 g, 56,0 mmol) in N-Methylpiperazin (77,7 mL, 700 mmol) wurde über Nacht auf 100°C erhitzt, anschliessend eingeengt und chromatographiert (Kieselgel, Dichlormethan:Methanol=20:1 bis 10:1, R_{*f*}(10:1)=0,32). Es wurden 32,0 g (1*R*, 2*R*)-2-{4-{(2-(4-Methyl-piperazin-1-yl)-benzimidazol-1-yl]methyl}-phenyl}-cyclohexan-1-carbonsäure-*tert*-butylester erhalten, die über Nacht bei Raumtemperatur mit Salzsäure (180 mL, 6 M) zur Reaktion gebracht wurden. Waschen des Reaktionsgemisches bei pH=7 mit Dichlormethan, Trocknen der organischen Phase über Magnesiumsulfat und Chromatographie (Kieselgel, Dichlormethan:Methanol 5:1, R_{*f*} =0,13) lieferte 19 g (78% der Theorie über 2 Stufen) der Titelverbindung. MS(ESI)=433 (M+H⁺). ¹H-NMR(DMSO-d₆): 1,35-1,5 (4 H, m); 1,65-1,8 (3 H, m); 1,9-2,0 (1 H, m); 2,2 (3 H, s); 2,4-2,5 (5 H, m); 2,6-2,7 (1 H, m); 3,15 (4 H, ψ t); 3,4 (1 H, very br s); 5,2 (2 H, s); 7,0-7,2 (7 H, m); 7,4 (1 H, d).

### c) (S)-N-{{(1R,2R)-2-{4-{[2-(4-Methyl-piperazin-1-yl)-benzimidazol-1-yl]methyl}-phenyl}-cyclohex-1-yl}carbonyl}-phenylglycinamid

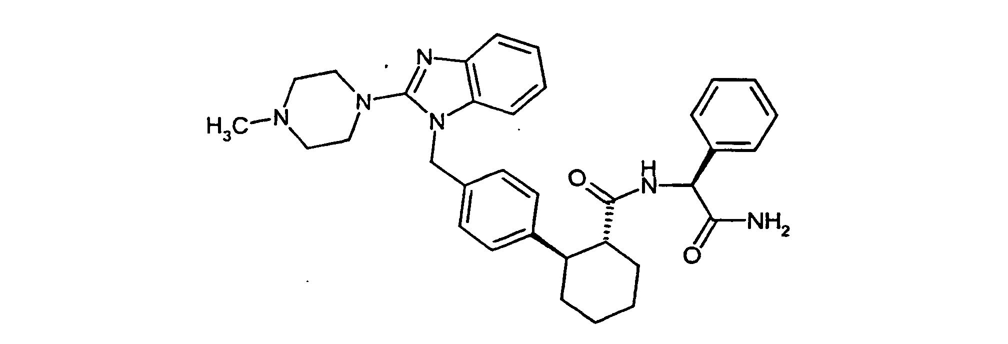

Eine Suspension der Verbindung von Beispiel 5b (19 g, 43,9 mmol), *(S)*-Phenylglycinamid-Hydrochlorid (8,61 g, 46,1 mmol), 1-Hydroxybenztriazol (7,68 g, 48,3 mmol), EDC-Hydrochlorid (9,68 g, 50,5 mmol) und Triethylamin (24,5 mL, 175,7 mmol) in Dichlormethan (1000 mL) wurde über das Wochenende bei Raumtemperatur gerührt. Nach Zugabe von Wasser wurde mit Dichlormethan/Methanol extrahiert, über Magnesiumsulfat getrocknet und eingeengt. Der leicht gelbliche Festkörper wurde in Dichlormethan/Methanol (10:1, 220 mL) verrührt, die saubere Titelverbindung wurde abfiltriert und bei 40°C am Vakuum getrocknet (14,5 g, 59%). R_{*f*} (Dichlormethan:Methanol 10:1)=0,30. MS(DCI, NH₃)=565 (M+H⁺). ¹H-NMR(DMSO-d₆): 1,2-1,5 (4 H, m);); 1,6-1,85 (4 H, m); 2,2 (3 H, s); 2,45 (4 H, ψ t); 2,65 (1 H, br t); 2,8 (1 H, td); 3,15 (4 H, ψ t); 5,15 (1 H, d); 5,2 (2 H, s); 6,9 (2 H, d); 6,95-7,2 (11 H, m); 7,45 (1 H, d); 7,6 (1 H, br s); 8,0 (1 H, d).

### Beispiel 6

### (S)-N-{{(1R, 2R)-2-{4-{[2-(4-Methyl-piperazin-1-yl)-benzimidazol-1-yl]methyl}-phenyl}-cyclohex-1-yl}carbonyl}-phenylglycinamid Hydrochlorid

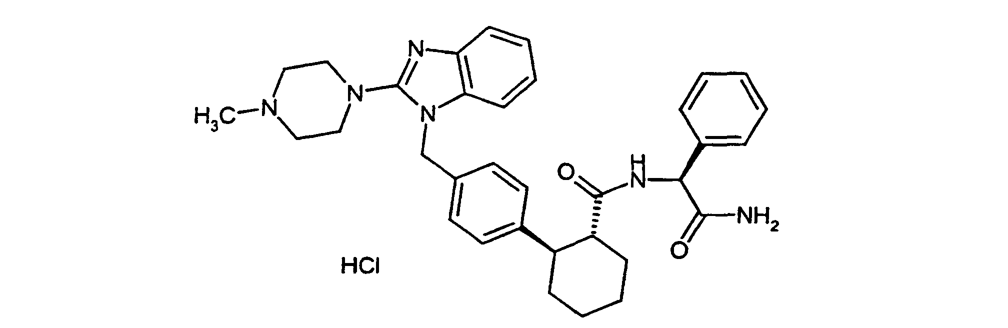

Die Verbindung von Beispiel 5 (100mg, 0,177 mmol) wurde in Dichlormethan/Methanol (2,5:1; 5mL) gelöst und mit 1M-HCl/Diethylether (0,177 mmol) versetzt, 5 min gerührt und dann in der Kälte am Vakuum eingeengt. Die Titelverbindung wurde als farbloses Pulver (106 mg) erhalten. Fp 200°C (Zers.).

Die in der folgenden Tabelle 1 aufgeführten Beispiele 7 bis 10 wurden analog Beispiel 5 unter Verwendung der entsprechend substituierten Piperazine hergestellt.

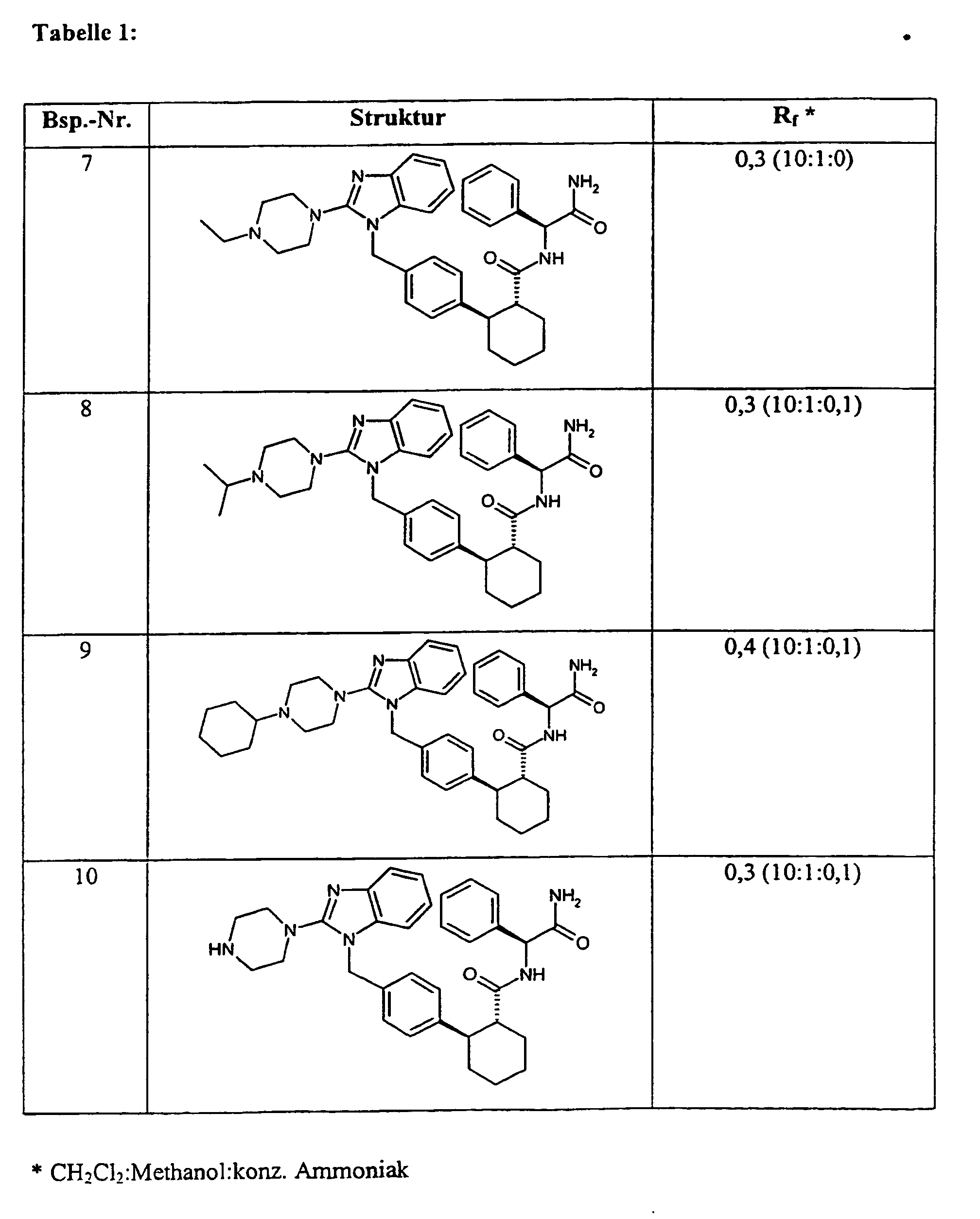

Die in der folgenden Tabelle 2 aufgeführten Beispiele 11 und 12 werden ausgehend von der Verbindung aus Beispiel 6A nach den allgemeinen Vorschriften A, B und C hergestellt.

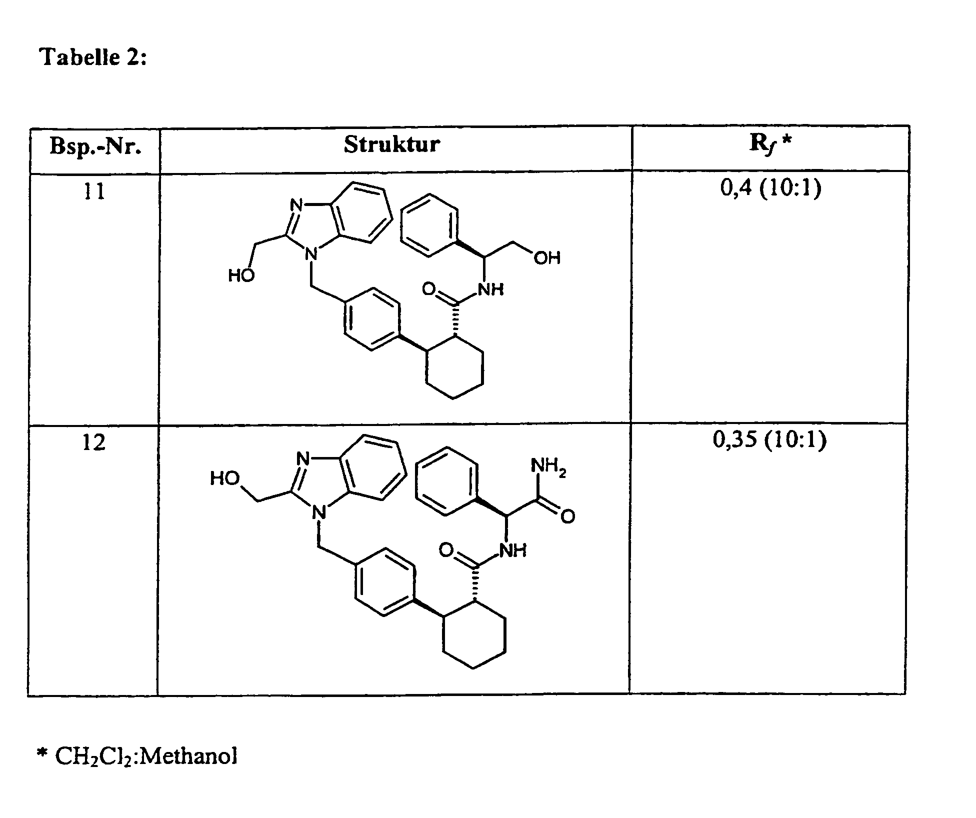

### Beispiel 13

### (S)-N-{{(1R, 2R)-2-{4-{[2-(2-Hydroxyethoxy)methyl]-benzimidazol-1-yl}methyl}-phenyl}-cyclohex-1-yl}carbonyl}-phenylglycinamid

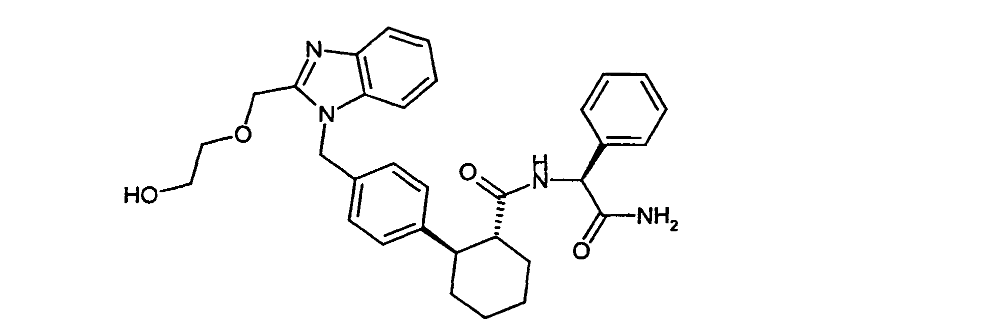

Ausgehend von der Verbindung des Beispiels 7A, das analog zu Beispiel 6A mit TBDMS-Chlorid silyliert wird und anschließend nach allgemeiner Vorschrift A, B und C umgesetzt wird, wird die Titelverbindung erhalten.
R_{*f*}(Dichlormethan:Methanol 20:1)=0,20.
MS(ESI)=541 (M+H⁺). ¹H-NMR(DMSO-d₆): 1,2-1,5 (4 H, m); 1,6-1,9 (4 H, m); 2,6-2,7 (1 H, m); 2,75-2,85 (1 H, m); 3,5 (4 H, s); 4,65 (1 H, br s); 4,6 (2 H, s); 5,15 (1 H, d); 5,55 (2 H, s); 6,9 (2 H, d); 6,95-7,2 (10 H, m); 7,45 (1 H, m); 7,6 (1 H, s); 7,65 (1 H, m); 8,05 (1 H, d).

Die in der folgenden Tabelle 3 aufgeführten Beispiele 14 bis 16 werden analog Beispiel 13 aus den entsprechenden Edukten hergestellt.

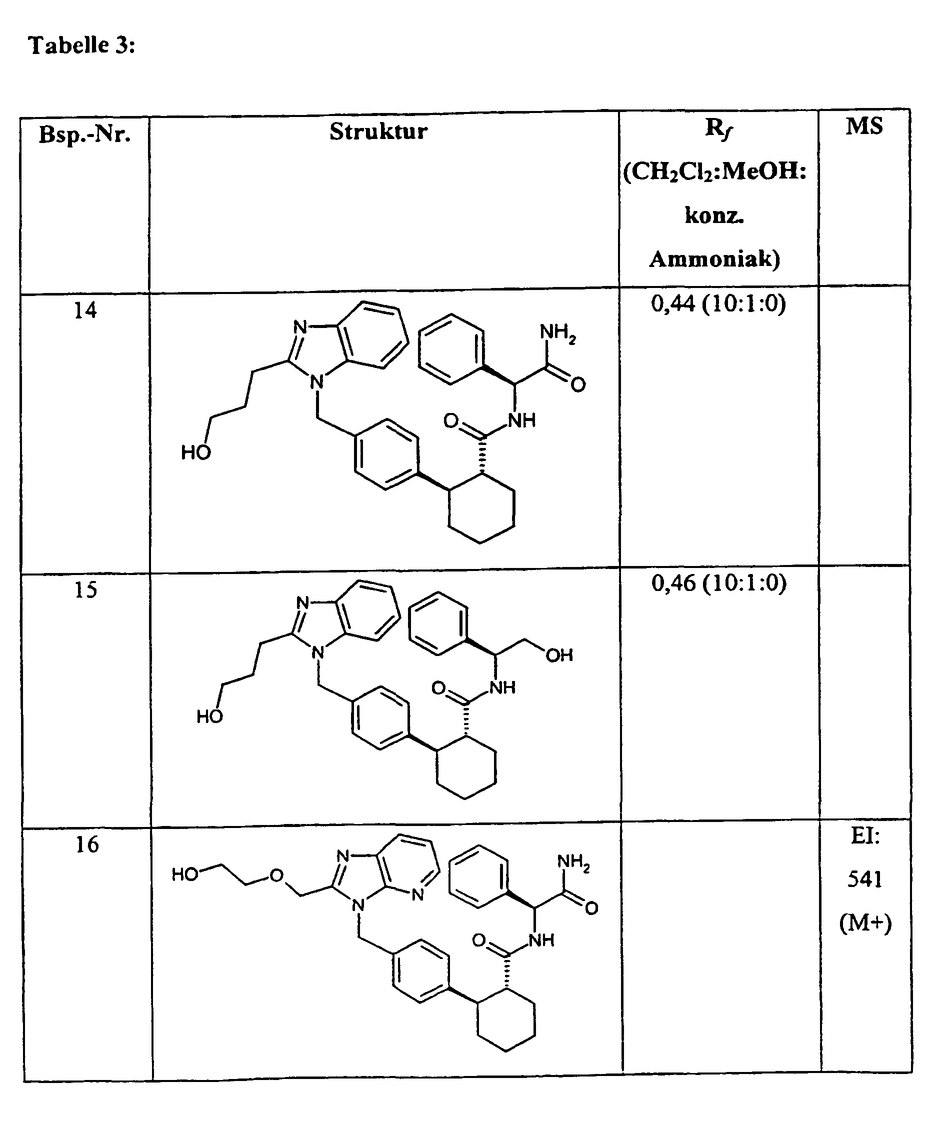

### Beispiel 17

### (S)-N-{[(1R,2R)-2-{4-{[2-(3-Hydroxypropyl)-1H-benzimidazol-1-yl]methyl}phenyl}-cyclohex-1-yl]carbonyl}-(4-fluorphenyl)glycinamid:

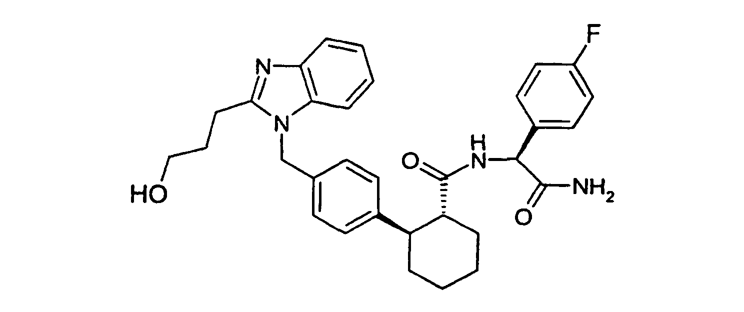

### a) (1R,2R)-2-(4-{[2-(3-{[tert-Butyl(dimethyl)silyl]oxy}propyl)-1H-benzimidazol-1-yl]methyl}phenyl)cyclohexan-1-carbonsäure-tert-butylester:

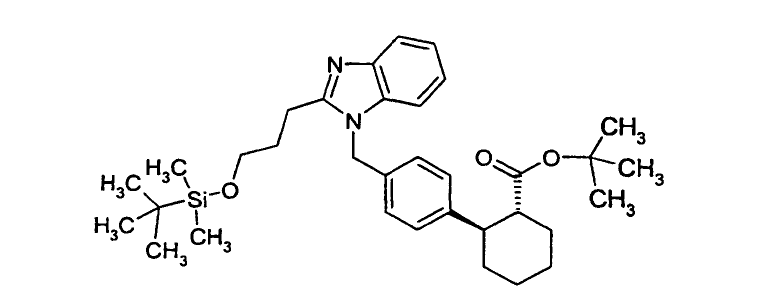

Zu einer Suspension von NaH (60 % in Mineralöl, 3.03 g, 75.77 mmol) in DMF (480 ml) wurde das 2-(3-{[*tert*-Butyl(dimethyl)silyl]oxy}propyl)-1*H*-benzimidazol aus Beispiel 8A (22.01 g, 75.77 mmol) in mehreren Portionen hinzugegeben. Nach 15 min bei RT wurde auf 40°C erhitzt und 30 min nachgerührt. Nach dem Abkühlen auf RT wurde die Verbindung aus Beispiel 2A (25.5 g, 72.17 mmol) portionsweise zugegeben und über Nacht nachgerührt. Nach Zugabe von 50 ml Wasser, Extraktion mit Diethylether, Trocknung der organischen Phase über Natriumsulfat und Chromatographie an Kieselgel (Eluens: Cyclohexan/Ethylacetat = 10:1 bis 5:1) wurden 38.86 g (95.7 %) eines farblosen Feststoffes erhalten.
MS (ESI): 563.3 (M+H)⁺
¹H-NMR (DMSO-d₆): 0.00 (3 H, s); 0.01 (3 H, s); 0.85 (9 H, s) 0.99 (9 H, s); 1.3-2.0 (10 H, m); 2.3-2.6 (2 H, m); 2.87 (2 H, t); 3.69 (2 H, t); 5.41 (2 H, s); 6.95-7.18 (6 H, m); 7.38-7.59 (2 H, m)

### b) (1R,2R)-2-(4-{[2-(3-Hydroxypropyl)-1H-benzimidazol-1-yl]methyl}phenyl)-cyclohexan-1-carbonsäure-tert-butylester:

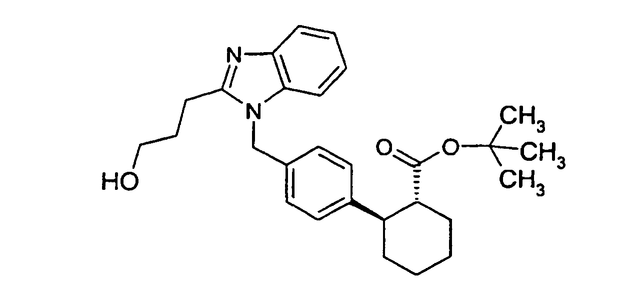

**I.)**
   Zu einer Lösung der Verbindung aus Beispiel 17a (38.80 g, 68.93 mmol) in THF (750 ml), wurde eine Lösung von Tetrabutylammoniumfluorid (TBAF) in THF (1.1 M, 68.9 ml, 75.83 mmol) zugetropft und 30 min bei RT gerührt. Nach Extraktion mit Diethylether, Trocknung der organischen Phase über Natriumsulfat und Chromatographie an Kieselgel (Eluens: Dichlormethan/Methanol = 95:5) wurden 29.0 g (93.8 %) eines farblosen Feststoffes isoliert.
   MS (ESI): 449.5 (M+H)⁺
   ¹H-NMR (DMSO-d₆): 0.99 (9 H, s); 1.25-1.55 (4 H, m); 1.6-1.8 (3 H, m); 1.8-2.0 (3 H, m); 2.3-2.65 (2 H, m); 2.86 (2 H, t); 3.49 (2 H, q); 4.60 (1 H, t); 5.42 (2 H, s); 6.99 (2 H, m); 7.08-7.18 (4 H, m); 7.33-7.44 (1 H, m); 7.5-7.6 (1 H, m).
**II.)**
   Die Verbindung des Beispiels 17b) lässt sich auch durch direkte Kupplung von 2-(3-Hydroxypropyl)-1*H*-benzimidazol an die Verbindung aus Beispiel 2A analog der allgemeinen Vorschrift zur Alkylierung A herstellen.
   Hierzu wurde 60proz. Natriumhydrid-Suspension in Mineralöl (880 mg, 22 mmol) in DMF (10 mL) vorgelegt, 2-(3-Hydroxypropyl)-1*H*-benzimidazol (3.52 g, 20 mmol) gelöst in DMF (50 mL) hinzugegeben und nach 10min. Rühren bei RT für 30 min auf 40°C erwärmt. Nach dem Abkühlen auf RT wurde die Verbindung aus Beispiel 2A (7.07 g, 20 mmol) gelöst in DMF (50 mL) hinzugegeben und das Reaktionsgemisch über Nacht bei RT gerührt. Zur Aufarbeitung wurde mit Wasser und Essigsäureethylester ausgeschüttelt, die wässrige Phase noch zweimal mit Essigsäureethylester extrahiert und die vereinten organischen Phasen einmal mit gesättigter Natriumchlorid-Lsg. gewaschen. Nach der Trocknung über Natriumsulfat wurde das Solvens im Vakuum entfernt und der Rückstand zur Kristallisation mit Diethylether/Petrolether (5:1, 70 mL) verrührt. Die kristalline Verbindung wurde abfiltriert und mit Petrolether nachgewaschen. Die Trocknung im Vakuum lieferte 6.62 g (74 % der Theorie) eines farblosen Feststoffs, welcher nach spektroskopischer Charakterisierung mit dem unter Beispiel 17 b) I.) übereinstimmt.

### c) (1R,2R)-2-(4-{[2-(3-Hydroxypropyl)-1H-benzimidazol-1-yl]methyl}phenyl)-cyclohexan-1-carbonsäure

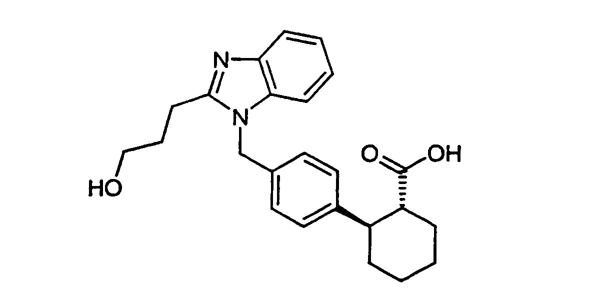

Zu einer eisgekühlten Lösung der Verbindung aus Beispiel 17 b) (26.5 g, 59.07 mmol) in Dichlormethan (530 ml) wurde Trifluoressigsäure (212 ml) hinzugegeben, langsam auf Raumtemperatur erwärmt und über Nacht bei Raumtemperatur nachgerührt. Anschließend wurde die Reaktionsmischung mit 1 N wässriger NaOH alkalisch gestellt und mit Diethylether gewaschen. Die wässrige Phase wurde mit 2 N HCl auf pH 3-4 gebracht (weiße Trübung) und mit Methylenchlorid extrahiert. Nach Trocknung der organischen Phase über Natriumsulfat wurde der Rückstand (weißer Schaum, 22.1 g, 95.3 %) am Ölpumpenvakuum getrocknet und ohne weitere Reinigung direkt umgesetzt.
MS (ESI): 393.1 (M+H)⁺
¹H-NMR (MeOH-d₄): 1.35-1.62 (4 H, m); 1.75-1.90 (3 H, m); 1.95-2.08 (3 H, m); 2,52-2,60 (1 H, m); 2,71-2,79 (1 H, m); 3.25 (2 H, t); 3.65 (2 H, t); 5.67 (2 H, s); 7.15 (2 H, d); 7.24 (2 H, d); 7.46-7.55 (2 H, m); 7.67 (1 H, d); 7.75 (1 H, d).

### d) (S)-N-{[(1R,2R)-2-{4-{[2-(3-Hydroxypropyl)-1H-benzimidazol-1-yl]methyl}-phenyl}-cyclohex-1-yl]carbonyl}-(4-fluorphenyl)glycinamid

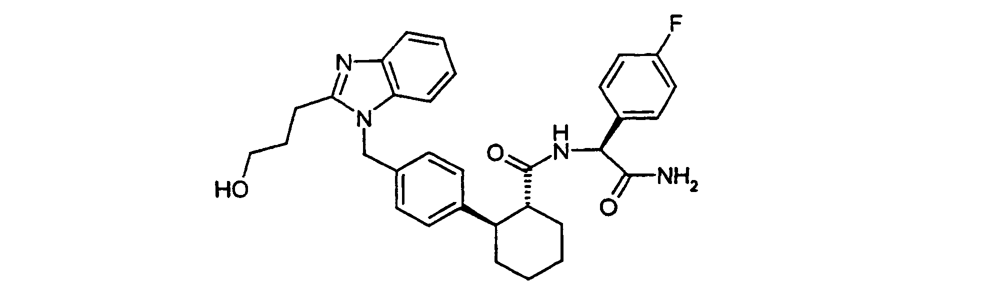

Zu einer Lösung der Verbindung aus Beispiel 17 c) (20.8 g, 53 mmol) in DMF (600 ml) wurden 1-Hydroxybenzotriazol (6.95 g, 51.4 mmol) und *N*-(3-Dimethylaminopropyl)-*N*'-ethylcarbodiimid-hydrochlorid (EDC x HCl) (11.68 g, 60.95 mmol) gegeben und 10 min bei Raumtemperatur gerührt.

Anschließend wurden N-Methylmorpholin (29.14 ml, 265 mmol), (*S*)-(4-Fluorphenyl)glycinamid-hydrochlorid (siehe Beispiel 9A c) und v)) (10.85 g, 53 mmol) und eine Spatelspitze Dimethylaminopyridin (DMAP) hinzugegeben und über Nacht bei Raumtemperatur gerührt. Nach Zugabe von Wasser (1.8 1) wurde 2 h bei Raumtemperatur und 1 h unter Eiskühlung nachgerührt. Die Titelverbindung, das (*S*)-*N*-{[(1*R*,2*R*)-2-{4-{[2-(3-Hydroxypropyl)-1H-benzimidazol-1-yl]methyl}phenyl}-cyclohex-1-yl]carbonyl}-(4-fluorphenyl)glycinamid, wurde anschließend abfiltriert und mit Wasser, n-Hexan und Diethylether nachgewaschen.

Nach Trocknung (3 Tage, 100 mbar, 45 °C) wurden 25.5 g (85.7 %) eines farblosen Feststoffes erhalten.
MS (ESI): 543 (M+H)⁺
¹H-NMR (DMSO-d₆): 1.15-1.55 (4 H, m); 1.55-2.0 (6 H, m); 2.55-2.92 (4 H, m); 3.48 (2 H, q); 4.59 (1 H, t); 5.15 (1 H, d); 5.41 (2 H, s); 6.85-6.98 (6 H, m); 7.05-7.17 (5 H, m); 7.35-7.42 (1 H, m); 7.53-7.66 (2 H, m); 8.10 (1 H, d).

Anstelle des (*S*)-(4-Fluorphenyl)glycinamid-hydrochlorids aus z.B. Beispiel 9A c) bzw. v) lässt sich zur Synthese des betreffenden Säureamids mit gleichem Erfolg das freie Amin einsetzen.

### Beispiel 18

### (S)-N-{[(1R,2R)-2-{4-{[2-(Hydroxymethyl)-1H-benzimidazol-1-yl]methyl}-phenyl}-cyclohex-1-yl]carbonyl}-(4-fluorphenyl)glycinamid:

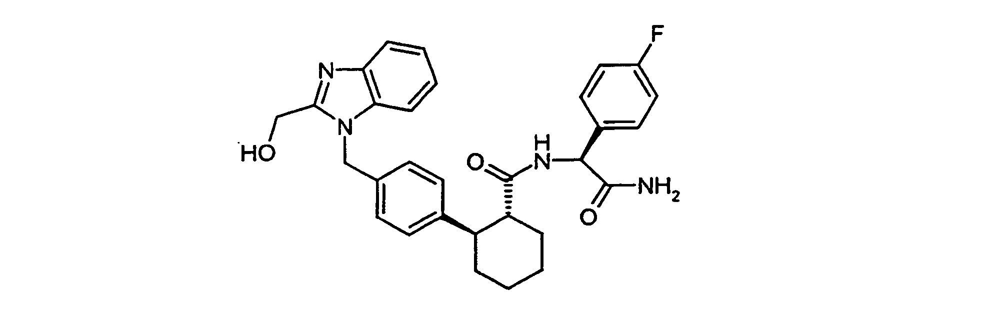

Ausgehend von den Verbindungen der Beispiele 2A, 6A und 9A c) bzw. v) wurde die Titelverbindung analog zu dem in Beispiel 17 beschriebenen Verfahren hergestellt. Anstelle der zweistufigen Schutzgruppenabspaltung wurden bei der zu Beispiel 17 a) analogen Zwischenverbindung der *tert*-Butyldimethylsilylether und der *tert*-Butylester durch die Einwirkung von konzentrierter Salzsäure in einem Schritt abgespalten.
R_{f}(Dichlormethan/Methanol = 10:1): 0,38
MS (ESI): 515 (M+H)⁺
¹H-NMR (DMSO-d₆): 1.20-1.50 (4 H, m); 1.61-1.78 (3 H, m); 1.78-1.86 (1 H, br. d); 2.65 (1 H, br. t); 2.80 (1 H, br. t); 4.71 (2 H, br. d); 5.15 (1 H, d); 5.48 (1 H, d; AB-System); 5.53 (1 H, d); 5.72 (1 H, t); 6.87-6.94 (4 H, m); 7.03-7.09 (2 H, m); 7.09-7.19 (5 H, m); 7.37 (1 H, m); 7.58-7.65 (2 H, m); 8.09 (1 H, d).

### Beispiel 19

### (S)-N-{[(1R,2R)-2-{4-{[2-(2-Hydroxyethyl)-1H-benzimidazol-1-yl]methyl}-phenyl}-cyclohex-1-yl]carbonyl}-phenylglycinamid

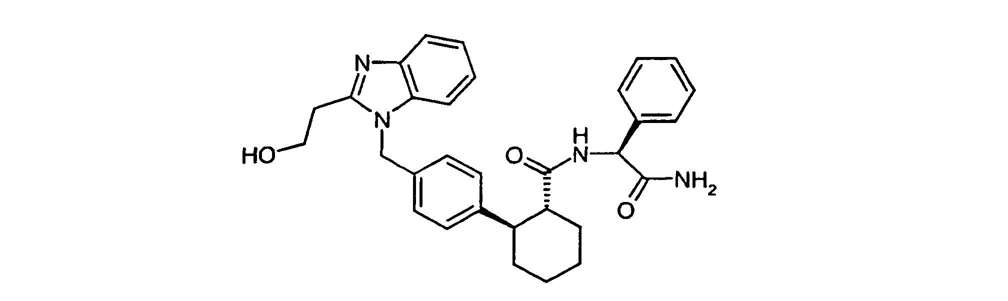

Die Titelverbindung wurde ausgehend von 2-(2-Hydroxyethyl)-1*H*-benzimidazol, welches nach Standardverfahren hergestellt wurde, analog zu dem für Beispiel 17 beschriebenen Verfahren unter Verwendung von (*S*)-Phenylglycinamid-Hydrochlorid anstelle von Beispiel 9A c) bzw. v) hergestellt.
R_{f} (Dichlormethan/Methanol = 10:1): 0,27
MS (ESI): 511 (M+H)⁺
¹H-NMR (DMSO-d₆): 1.15-1.52 (4 H, m); 1.59-1.91 (4 H, m); 2.67 (1 H, br. t); 2.83 (1 H, br. t); 2.98 (2 H, t); 3.86 (2 H; br. q); 4.89 (1 H, br. t); 5.17 (1 H, d); 5.44 (2 H, br. s); 6.83-7.22 (12 H, m); 7.38-7.48 (1 H, m); 7.54-7.67 (2 H, m); 8.10 (1 H, d).

### Beispiel 20

### (S)-N-{[(1R,2R)-2-{4-{[2-(2-Hydroxyethyl)-1H-benzimidazol-1-yl]methyl}-phenyl}-cyclohex-1-yl]carbonyl}-(4-fluorphenyl)glycinamid:

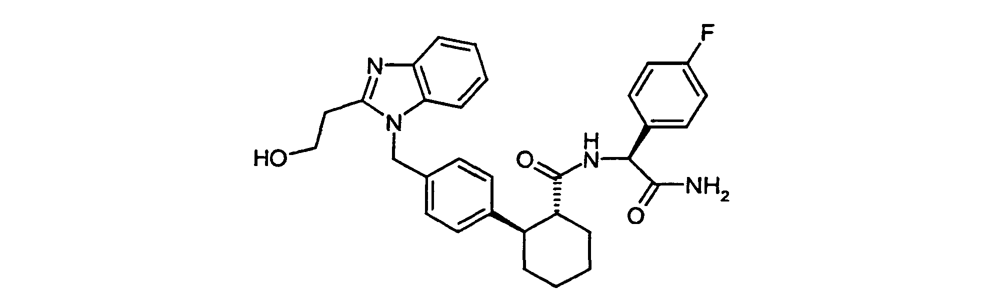

Die Titelverbindung wurde analog zu Beispiel 19 unter Verwendung des (*S*)-(4-Fluorphenyl)glycinamid-hydrochlorids aus Beispiel 9A c) bzw. v) hergestellt.
R_{f} (Dichlormethan/Methanol = 10:1): 0,26
MS (ESI): 529 (M+H)⁺
¹H-NMR (DMSO-d₆): 1.19-1.51 (4 H, m); 1.61-1.78 (3 H, m); 1.78-1.87 (1 H, br. d); 2.65 (1 H, br. t); 2.80 (1 H, br. t); 2.97 (2 H, t); 3.85 (2 H, q); 4.85 (1 H, t); 5.15 (1 H, d); 5.44 (2 H, br. s); 6.85-6.99 (6 H, m); 7.08-7.19 (5 H, m); 7.40 (1 H, pseudo-d); 7.57 (1 H, pseudo-d); 7.61 (1 H, br. s); 8.10 (1 H, d).

### Beispiel 21

### (S)-N-{[(1R,2R)-2-{4-{[2-(3-Hydroxypropyl)-1H-benzimidazol-1-yl]methyl}-phenyl}-cyclohex-1-yl]carbonyl}-(3-pyridyl)glycinamid:

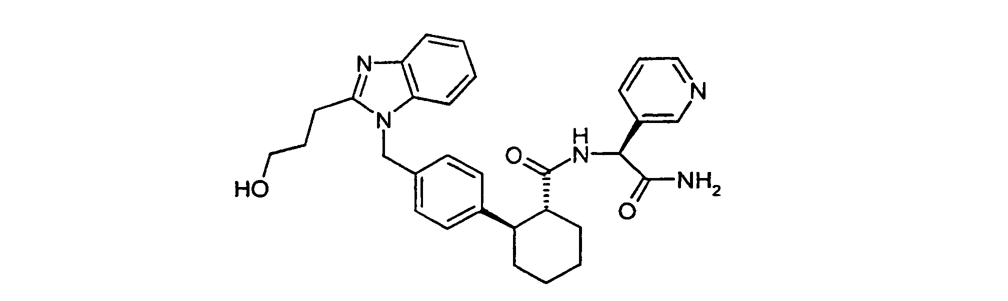

Zu einer Lösung von der Substanz aus Beispiel 17 c) (118 mg, 0.3 mmol), 1-Hydroxybenzotriazol (40.5 mg, 0.3 mmol), EDC-Hydrochlorid (69 mg, 0.36 mmol) und 4-(N,N-Dimethylamino)-pyridin (DMAP, 1 mg) in DMF (3 mL) wurde N-Methylmorpholin (0.13 mL, 1.2 mmol) hinzugegeben und das resultierende Gemisch in ein Reaktionsgefäß mit (*S*)-(3-Pyridyl)glycinamid-Hydrochlorid (101 mg, 0.45 mg) - welches analog der Substanz aus Beispiel 9A c) bzw. v) herstellbar ist - überführt. Nach dreitägiger Umsetzung bei Raumtemperatur wurde das Reaktionsgemisch direkt mittels einer präparativen Hochdruckchromalographie an reversed-phase-Kieselgel (Grom-Sil 120 ODS-4 HE 5 µm) mit Wasser/Acetonitril (Gradient: 10:90 bis 90:10) gereinigt. Die erhaltene Lösung wurde im Vakuum vom Acetonitril befreit, im Trockeneisbad eingefroren und über Nacht gefriergetrocknet. Es wurden 143 mg (91 % der Theorie) farbloses Lyophilisat erhalten.
R_{f} (Dichlormethan/Methanol = 10:1): 0,11
MS (ESI): 526 (M+H)
¹H-NMR (DMSO-d₆): 1.15-1.55 (4 H, m); 1.57-1.91 (4 H, m); 1.97 (2 H, quint.); 2.65 (1 H, br. t); 2.81 (1 H, br. t); 3.27 (2 H, t); 3.52 (2 H, t); 5.29 (1 H, d); 5.69 (2 H, s); 7.07-7.20 (4 H, m); 7.20-7.29 (1 H, m); 7.32 (1 H, br. s); 7.38-7.45 (1 H, m); 7.46-7.60 (2 H, m); 7.72-7.87 (3 H; m); 8.09 (1 H, br. s); 8.36 (1 H, d); 8.44 (1 H, m).

## Patentansprüche

1. Verwendung von Verbindungen der allgemeinen Formel (I) 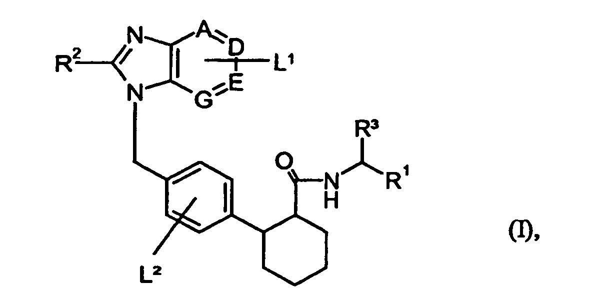 in welcher
A, D, E und G gleich oder verschieden sind und für CH-Gruppen oder Stickstoffatome stehen,
L¹ und L² gleich oder verschieden sind und unabhängig voneinander für einen oder mehrere Reste ausgewählt aus der Gruppe Wasserstoff, Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl stehen,
R¹ für die CH₂-OH-Gruppe steht, oder
für einen Rest der Formel CO-NR⁴R⁵ steht,
worin
R⁴ und R⁵ gleich oder verschieden sind und Wasserstoff oder (C₁-C₆)-Alkyl bedeuten,
R² für (C₃-C₈)-Cycloalkyl steht,
für (C₁-C₈)-Alkyl steht, das gegebenenfalls durch ein Sauerstoff- oder Schwefelatom oder durch einen Rest NR⁶ unterbrochen ist,
für einen über ein Stickstoffatom an den Imidazolring gebundenen 4- bis 8-gliedrigen, gesättigten Heterocyclus steht, der gegebenenfalls ein weiteres Sauerstoff- oder Schwefelatom enthält, oder
für einen 4- bis 8-gliedrigen gesättigten Heterocyclus steht, der einen Rest der Formel NR⁷ und gegebenenfalls zusätzlich ein Stickstoff-, Sauerstoff- oder Schwefelatom enthält,
wobei (C₃-C₈)-Cycloalkyl, (C₁-C₈)-Alkyl, das gegebenenfalls durch ein Sauerstoff- oder Schwefelatom unterbrochen ist, der über ein Stickstoffatom an den Imidazolring gebundene 4- bis 8-gliedrige, gesättigte Heterocyclus, der gegebenenfalls ein weiteres Sauerstoff- oder Schwefelatom enthält, sowie gegebenenfalls (C₁-C₈)-Alkyl, das durch einen Rest NR⁶ unterbrochen ist, und gegebenenfalls der 4- bis 8-gliedrige gesättigte Heterocyclus, der einen Rest der Formel NR⁷ und gegebenenfalls zusätzlich ein Stickstoff-, Sauerstoff- oder Schwefelatom enthält, durch eine bis drei Hydroxyl-Gruppen und/oder durch einen Rest der Formel -NR⁸R⁹ substituiert sind,
worin
R⁶ und R⁷ gleich oder verschieden sind und Wasserstoff, (C₁-C₆)-Alkyl, Hydroxy-(C₁-C₆)-Alkyl oder (C₃-C₇)-Cycloalkyl bedeuten,
R⁸ und R⁹ gleich oder verschieden sind und Wasserstoff, (C₁-C₆)-Alkyl oder (C₃-C₇)-Cycloalkyl bedeuten,
oder
R⁸ und R⁹ gemeinsam mit dem Stickstoffatom einen 4- bis 8-gliedrigen gesättigten Heterocyclus bilden, der gegebenenfalls zusätzlich ein Sauerstoff- oder Schwefelatom oder einen Rest der Formel NR¹⁰ enthalten kann,
worin
R¹⁰ Wasserstoff, (C₁-C₆)-Alkyl oder (C₃-C₇)-Cycloalkyl bedeuten,
und
R³ für einen Phenyl-, Naphthyl-, Pyrimidinyl-, Pyridyl-, Furyl- oder Thienylring steht, wobei die Ringe gegebenenfalls ein- oder mehrfach mit Resten ausgewählt aus der Gruppe Halogen, Hydroxy, Carboxyl, Cyano, Nitro, Trifluormethyl, Trifluormethoxy, (C₁-C₆)-Alkyl, (C₁-C₆)-Alkoxy oder (C₁-C₆)-Alkoxycarbonyl substituiert sind,
und deren Enantiomere und Diastereomere sowie deren jeweilige Salze und Hydrate zur Herstellung von Arzneimitteln zur Behandlung und/oder Prophylase von ischämischen Erkrankungen des Herz-Kreislauf-Systems.

2. Verwendung von Verbindungen nach Anspruch 1,
wobei
A, D, E und G jeweils für die CH-Gruppe stehen,
oder einer der Reste A, D, E und G ein Stickstoffatom bedeutet und die anderen jeweils für die CH-Gruppe stehen,
L¹ und L² gleich oder verschieden sind und unabhängig voneinander für einen oder mehrere Reste ausgewählt aus der Gruppe Wasserstoff, Fluor, Chlor, Cyano, Trifluormethyl oder Trifluormethoxy stehen,
R¹ für die -CH₂-OH-Gruppe steht, oder
für einen Rest der Formel -CO-NR⁴R⁵ steht,
worin
R⁴ und R⁵ gleich oder verschieden sind und Wasserstoff oder (C₁-C₃)-Alkyl bedeuten,
R² für (C₃-C₇)-Cycloalkyl steht,
für (C₁-C₆)-Alkyl steht, das gegebenenfalls durch ein Sauerstoff- oder Schwefelatom oder durch einen Rest NR⁶ unterbrochen ist,
für einen über ein Stickstoffatom an den Imidazolring gebundenen 5- bis 7-gliedrigen, gesättigten Heterocyclus steht, der gegebenenfalls ein weiteres Sauerstoff- oder Schwefelatom enthält, oder
für einen 5- bis 7-gliedrigen gesättigten Heterocyclus steht, der einen Rest der Formel NR⁷ und gegebenenfalls zusätzlich ein Stickstoff-, Sauerstoff- oder Schwefelatom enthält,
wobei (C₃-C₇)-Cycloalkyl, (C₁-C₆)-Alkyl, das gegebenenfalls durch ein Sauerstoff- oder Schwefelatom unterbrochen ist, der über ein Stickstoffatom an den Imidazolring gebundene 5- bis 7-gliedrige, gesättigte Heterocyclus, der gegebenenfalls ein weiteres Sauerstoff- oder Schwefelatom enthält, sowie gegebenenfalls (C₁-C₆)-Alkyl, das durch einen Rest NR⁶ unterbrochen ist, und gegebenenfalls der 5- bis 7-gliedrige gesättigte Heterocyclus, der einen Rest der Formel NR⁷ und gegebenenfalls zusätzlich ein Stickstoff-, Sauerstoff- oder Schwefelatom enthält, durch eine Hydroxyl-Gruppe und/oder durch einen Rest der Formel -NR⁸R⁹ substituiert sind,
worin
R⁶ und R⁷ gleich oder verschieden sind und Wasserstoff, (C₁-C₄)-Alkyl Hydroxy-(C₁-C₄)-Alkyl oder (C₃-C₆)-Cycloalkyl bedeuten,
R⁸ und R⁹ gleich oder verschieden sind und Wasserstoff, (C₁-C₄)-Alkyl oder (C₃-C₆)-Cycloalkyl bedeuten,
oder
R⁸ und R⁹ gemeinsam mit dem Stickstoffatom einen 5- bis 7-gliedrigen gesättigten Heterocyclus bilden, der gegebenenfalls zusätzlich ein Sauerstoff- oder Schwefelatom oder einen Rest der Formel NR¹⁰ enthalten kann,
worin
R¹⁰ Wasserstoff, (C₁-C₄)-Alkyl oder (C₃-C₆)-Cycloalkyl bedeuten,
und
R³ für einen Phenyl-, Pyridyl- oder Thienylring steht, der gegebenenfalls ein- oder mehrfach mit Resten, ausgewählt aus der Gruppe Fluor, Chlor, Cyano, Trifluormethyl oder Trifluormethoxy, substituiert ist,
und deren Enantiomere und Diastereomere sowie deren jeweilige Salze und Hydrate.

3. Verbindungen nach Anspruch 1 oder 2,
wobei
A, D und E jeweils für die CH-Gruppe stehen,
G für ein Stickstoffatom oder für die CH-Gruppe steht,
L¹ und L² für Wasserstoff stehen,
R¹ für einen Rest der Formel -CO-NR⁴R⁵ steht,
worin
R⁴ und R⁵ Wasserstoff bedeuten,
R² für (C₁-C₄)-Alkyl steht, das gegebenenfalls durch ein Sauerstoffatom unterbrochen ist, oder
für einen 4-R⁷-Piperazin-1-ylrest steht
wobei (C₁-C₄)-Alkyl, das gegebenenfalls durch ein Sauerstoffatom unterbrochen ist, durch eine Hydroxyl-Gruppe oder durch einen Rest der Formel -NR⁸R⁹ substituiert ist,
worin
R⁷ Wasserstoff, (C₁-C₄)-Alkyl oder (C₃-C₆)-Cycloalkyl bedeuten,
R⁸ und R⁹ gleich oder verschieden sind und Wasserstoff, (C₁-C₄)-Alkyl oder (C₃-C₆)-Cycloalkyl bedeuten,
oder
R⁸ und R⁹ gemeinsam mit dem Stickstoffatom einen Morpholinrest bilden,
und
R³ für einen Phenyl- oder Pyridylrest steht, wobei der Phenylrest ein- oder mehrfach durch Fluor substituiert sein kann,
und deren Enantiomere und Diastereomere sowie deren jeweilige Salze und Hydrate.

4. Verbindungen nach Anspruch 3,
wobei
der Rest R¹ für einen Rest der Formel CO-NR⁴R⁵ mit R⁴ und R⁵ wie zuvor definiert steht
und
die übrigen Reste die in Anspruch 3 angegebene Bedeutung haben.

5. Verbindungen nach einem der Ansprüche 3 oder 4, **gekennzeichnet durch** die folgende Stereochemie gemäß Formel (Ia): 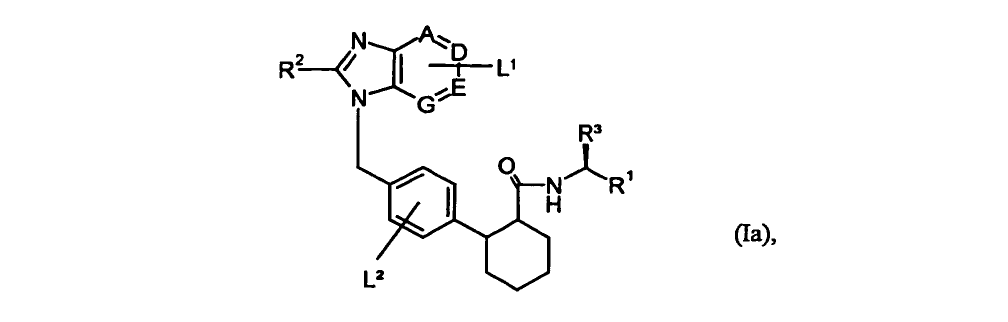 wobei die Substituenten R¹, R², R³, L¹ und L² und die Reste A, D, E und G die in den Ansprüchen 3 oder 4 angegebene Bedeutung haben.

6. Verbindungen nach einem der Ansprüche 3 bis 5, **gekennzeichnet durch** die folgende Stereochemie gemäß Formel (Ib) 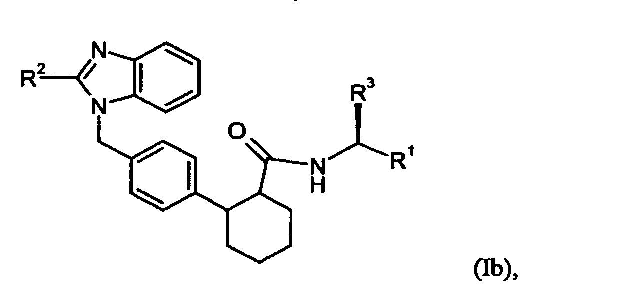 in der
R¹ für eine Gruppe -C(O)-NH₂ steht,
R² für (C₁-C₄)-Alkyl steht, das am endständigen C-Atom **durch** eine Hydroxylgruppe substituiert ist,
R³ für einen Phenylring steht, der in der para-Position **durch** Fluor substituiert ist,
oder
für einen Pyridylrest steht,
und deren Diastereomere sowie deren jeweilige Salze und Hydrate.

7. Verbindungen nach Anspruch 3 der allgemeinen Formel (I) mit den folgenden Strukturen: 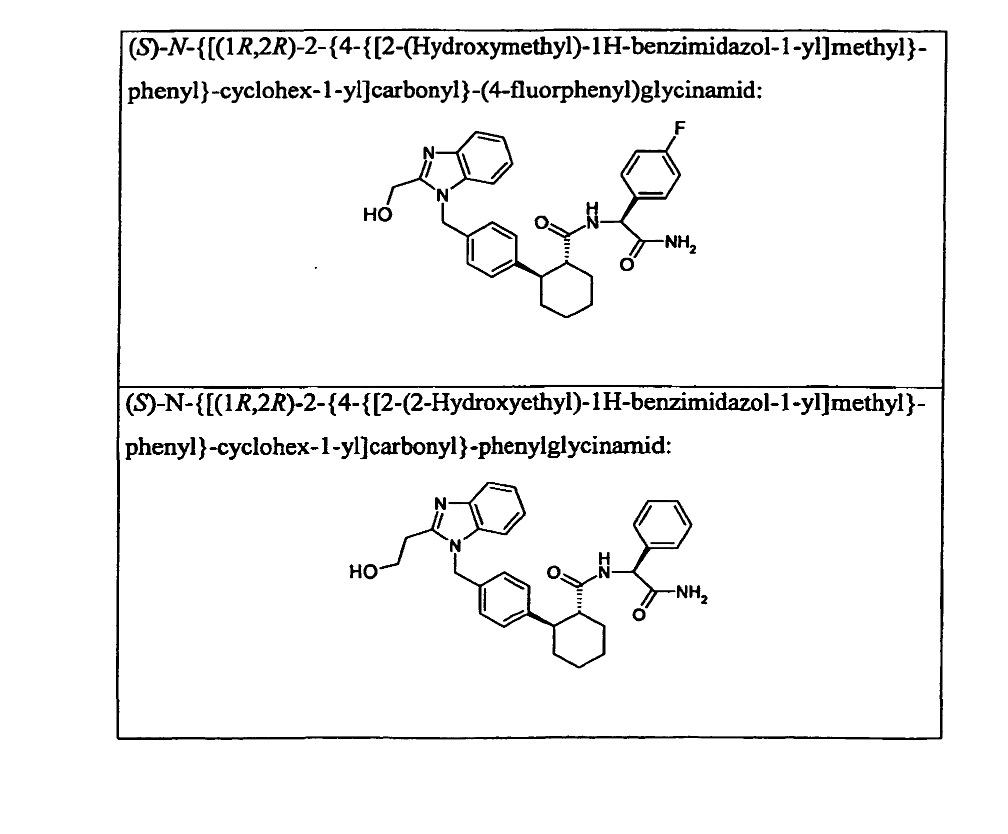 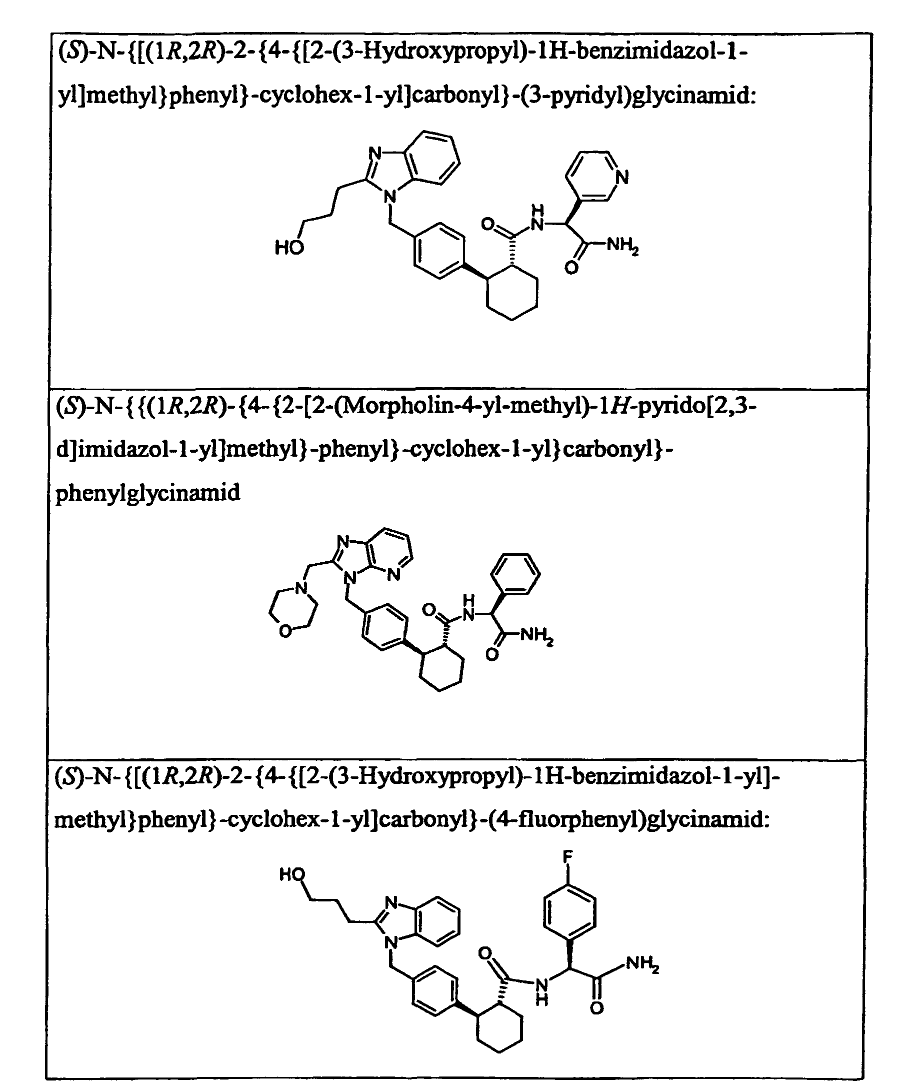 und ihre Salze und Hydrate.

8. Verfahren zur Herstellung von Verbindungen der allgemeinen Formel (I) gemäß Ansprüchen 1 bis 7, **dadurch gekennzeichnet, dass** man
[A] Verbindungen der allgemeinen Formel (II) 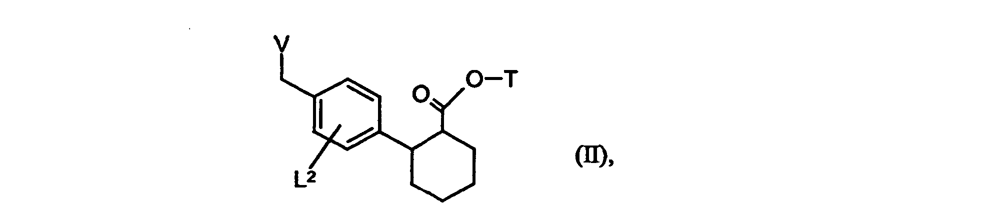 in welcher
L² die zuvor angegebene Bedeutung hat,
T für (C₁-C₄)-Alkyl, vorzugsweise für Methyl oder tert.Butyl steht,
und
V für eine geeignete Abgangsgruppe, wie beispielsweise Halogen, Mesylat oder Tosylat, vorzugsweise für Brom steht,
zunächst durch Umsetzung mit Verbindungen der allgemeinen Formel (III) 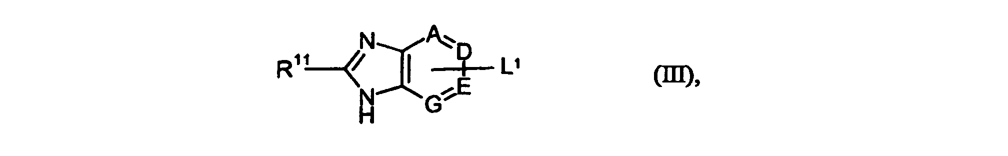 in welcher
A, D, E, G und L¹ die zuvor angegebene Bedeutung haben
und
R¹¹ die zuvor aufgeführte Bedeutung von R² hat, wobei Amino- und Hydroxyfunktionen gegebenenfalls durch geeignete Amino- bzw. Hydroxyschutzgruppen blockiert sind,
in inerten Lösemitteln, in Abhängigkeit der Definition von R¹¹ gegebenenfalls in Anwesenheit einer Base in die Verbindungen der allgemeinen Formel (IV) 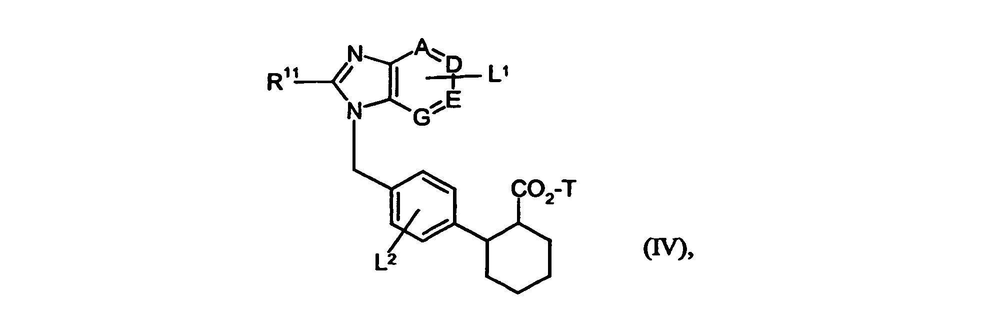 in welcher
R¹¹, A, D, E, G, L¹, L², und T die zuvor angegebene Bedeutung haben,
überführt,
in einem nächsten Schritt mit Säuren oder Basen in die entsprechenden Carbonsäuren der allgemeinen Formel (V) 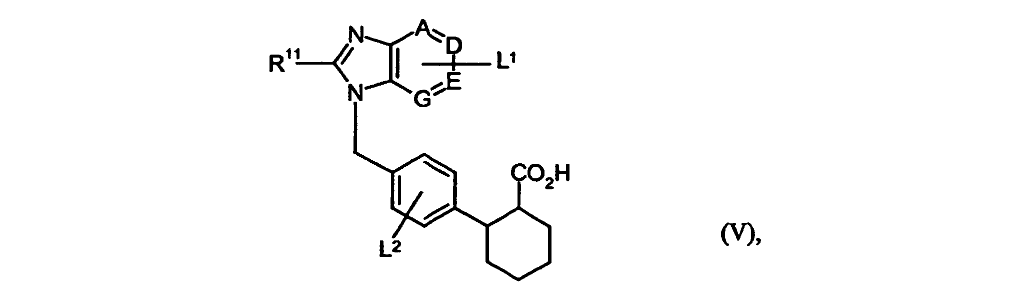 in welcher
R¹¹, A, D, E, G, L¹ und L² die zuvor angegebene Bedeutung haben,
überführt,
diese gegebenenfalls aktiviert, insbesondere durch Überführung in ein entsprechendes Carbonsäurederivat wie Carbonsäurehalogenid, Carbonsäureanhydrid oder Carbonsäureester,
und abschließend nach bekannten Methoden mit Verbindungen der allgemeinen Formel (VI) oder deren Salzen 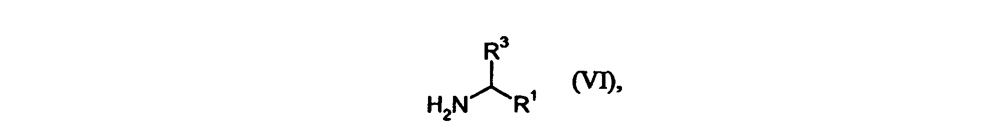 in welcher
R¹ und R³ die in den Ansprüchen 3 bis 6 angegebene Bedeutung haben,
in inerten Lösemitteln umsetzt,
und gegebenenfalls im Fall, dass R¹¹ eine der oben aufgeführten Schutzgruppen trägt, diese entweder bei der Hydrolyse zu den Säuren (IV) -> (V) oder nach Umsetzung mit den Verbindungen der allgemeinen Formel (VI) nach üblichen Methoden abspaltet,
oder
[B] im Fall, dass R² für einen direkt über ein Stickstoffatom an den Imidazolring gebundenen, gesättigten Heterocyclus steht,
zunächst die oben aufgeführten Verbindungen der allgemeinen Formel (II) mit Verbindungen der allgemeinen Formel (IIIa) 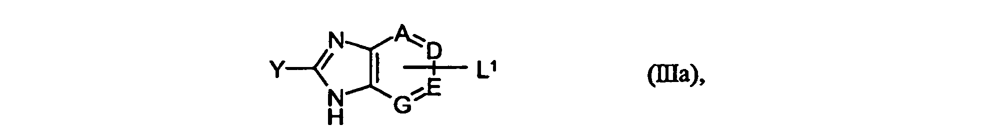
in welcher
A, D, E, G und L¹ die zuvor angegebene Bedeutung haben
und
Y für Halogen oder Mesylat, vorzugsweise für Chlor, Brom oder Mesylat steht,
in inerten Lösemitteln in die entsprechenden Verbindungen der Formel (VII) 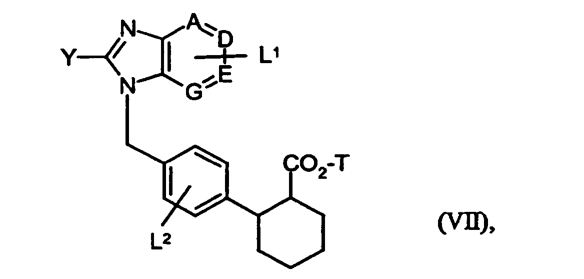 in welcher
Y, A, D, E, G, L¹, L² und T die oben angegebene Bedeutung haben,
überführt,
in einem nächsten Schritt mit Verbindungen der allgemeinen Formel (VIII)
HNR¹²R¹³ (VIII),
in welcher
R¹² und R¹³ zusammen mit dem Stickstoffatom einen Heterocyclus gemäß der Definition von R² bilden,
zu Verbindungen der allgemeinen Formel (IX) 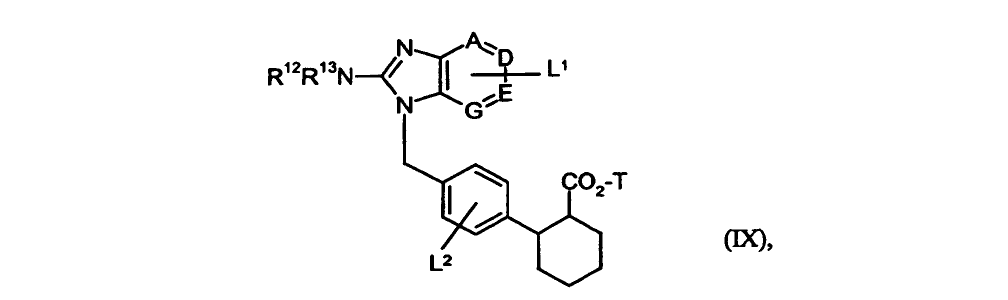 in welcher
A, D, E, G, L¹, L², R¹², R¹³ und T die zuvor angegebene Bedeutung haben,
umsetzt,
in den nächsten Schritten, wie unter [A] beschrieben durch Hydrolyse in die entsprechenden Carbonsäuren der allgemeinen Formel (X) 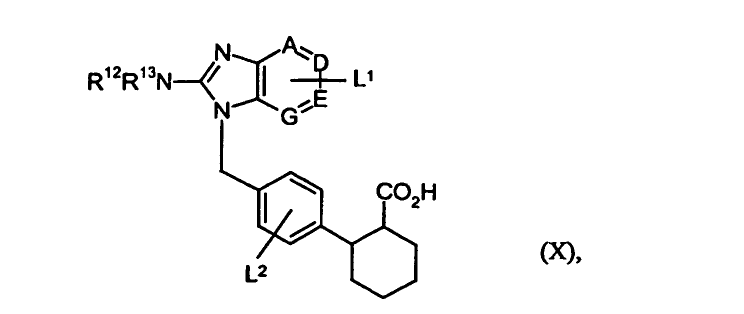 in welcher
A, D, E, G, L¹, L², R¹² und R¹³ die zuvor angegebene Bedeutung haben,
überführt,
und abschließend nach bekannten Methoden zur Herstellung von Amiden aus Carbonsäuren und Aminen mit den Verbindungen der allgemeinen Formel (VI) zu den Verbindungen der allgemeinen Formel (I) umsetzt
wobei die nach der Verfahrensvariante [A] oder [B] erhaltenen Verbindungen der allgemeinen Formel (I) gegebenenfalls anschließend durch Umsetzung z.B. mit einer Säure in die entsprechenden Salze überführt werden können.

9. (*S*)-(4-Fluorphenyl)glycinamid 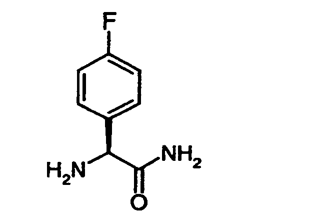 und dessen Salze.

10. (*S*)-(3-Pyridyl)glycinamid 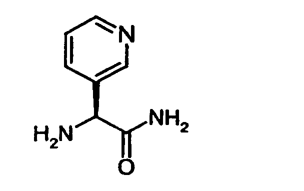 und dessen Salze.

11. Arzneimittel, enthaltend eine Verbindung der allgemeinen Formel (I) gemäß einem der Ansprüche 3 bis 7 zusammen mit mindestens einem pharmazeutisch verträglichen, im wesentlichen ungiftigen Träger oder Exzipienten.

12. Verbindungen gemäß einem der Ansprüche 3 bis 7 zur Verwendung als Arzneimittel in der Behandlung von Menschen und Tieren.

13. Verbindungen gemäß einem der Ansprüche 3 bis 7 zur Prophylaxe und/oder Behandlung von Erkrankungen bei Menschen und Tieren.

14. Verwendung von Verbindungen gemäß einem der Ansprüche 3 bis 7 zur Herstellung von Arzneimitteln zur Prophylaxe und/oder Behandlung von Erkrankungen bei Menschen und Tieren.

15. Verwendung von Verbindungen gemäß einem der Ansprüche 3 bis 7 zur Herstellung von Arzneimitteln zur Behandlung und/oder Prophylaxe von ischämischen Erkrankungen des Herz-Kreislauf-Systems.

## Claims

1. Use of compounds of the general formula (I) 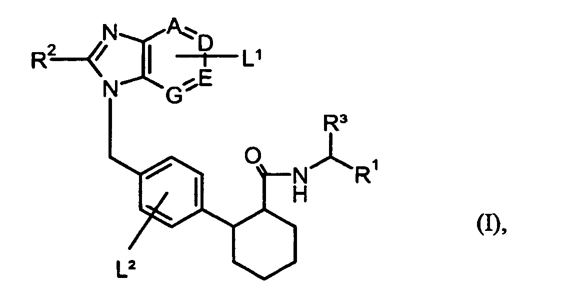 in which
A, D, E and G are identical or different and represent CH groups or nitrogen atoms,
L¹ and L² are identical or different and independently of one another each represents one or more radicals selected from the group consisting of hydrogen, halogen, hydroxyl, carboxyl, cyano, nitro, trifluoromethyl, trifluoromethoxy, (C₁-C₆)-alkyl, (C₁-C₆)-alkoxy or (C₁-C₆)-alkoxycarbonyl,
R¹ represents the CH₂-OH group, or
represents a radical of the formula CO-NR⁴R⁵,
in which
R⁴ and R⁵ are identical or different and each represents hydrogen or (C₁-C₆)-alkyl,
R² represents (C₃-C₈)-cycloalkyl,
represents (C₁-C₈)-alkyl which is optionally interrupted by an oxygen or sulphur atom or by a radical NR⁶,
represents a 4- to 8-membered saturated heterocycle which is attached to the imidazole ring via a nitrogen atom and which optionally contains a further oxygen or sulphur atom, or
represents a 4- to 8-membered saturated heterocycle which contains a radical of the formula NR⁷ and optionally additionally one nitrogen, oxygen or sulphur atom,
where (C₃-C₈)-cycloalkyl, (C₁-C₈)-alkyl which is optionally interrupted by an oxygen or sulphur atom, the 4- to 8-membered saturated heterocycle which is attached to the imidazole ring via a nitrogen atom and which optionally contains a further oxygen or sulphur atom and optionally (C₁-C₈)-alkyl which is interrupted by a radical of the formula NR⁶ and optionally the 4- to 8-membered saturated heterocycle which contains a radical NR⁷ and optionally additionally one nitrogen, oxygen or sulphur atom are substituted by one to three hydroxyl groups and/or by a radical of the formula -NR⁸R⁹
in which
R⁶ and R⁷ are identical or different and each represents hydrogen, (C₁-C₆)-alkyl, hydroxy-(C₁-C₆)-alkyl or (C₃-C₇)-cycloalkyl,
R⁸ and R⁹ are identical or different and each represents hydrogen, (C₁-C₆)-alkyl or (C₃-C₇)-cycloalkyl,
or
R⁸ and R⁹ together with the nitrogen atom form a 4- to 8-membered saturated heterocycle which may optionally additionally contain one oxygen or sulphur atom or a radical of the formula NR¹⁰,
in which
R¹⁰ represents hydrogen, (C₁-C₆)-alkyl or (C₃-C₇)-cycloalkyl,
and
R³ represents a phenyl, naphthyl, pyrimidinyl, pyridyl, furyl or thienyl ring, where the rings are optionally mono- or polysubstituted by radicals selected from the group consisting of halogen, hydroxyl, carboxyl, cyano, nitro, trifluoromethyl, trifluoromethoxy, (C₁-C₆)-alkyl, (C₁-C₆)-alkoxy and (C₁-C₆)-alkoxycarbonyl,
and their enantiomers and diastereomers and their respective salts and hydrates for preparing medicaments for the treatment and/or prophylaxis for ischaemic disorders of the cardiovascular system.

2. Use of compounds according to Claim 1
where
A, D, E and G each represent the CH group,
or one of the radicals A, D, E and G represents a nitrogen atom and the others each represent the CH group,
L¹ and L² are identical or different and independently of one another each represents one or more radicals selected from the group consisting of hydrogen, fluorine, chlorine, cyano, trifluoromethyl and trifluoromethoxy,
R¹ represents the -CH₂-OH group, or
represents a radical of the formula -CO-NR⁴R⁵,
in which
R⁴ and R⁵ are identical or different and each represents hydrogen or (C₁-C₃)-alkyl,
R² represents (C₃-C₇)-cycloalkyl,
represents (C₁-C₆)-alkyl which is optionally interrupted by an oxygen or sulphur atom or by a radical NR⁶,
represents a 5- to 7-membered saturated heterocycle which is attached to the imidazole ring via a nitrogen atom and which optionally contains a further oxygen or sulphur atom, or
represents a 5- to 7-membered saturated heterocycle which contains a radical of the formula NR⁷ and optionally additionally one nitrogen, oxygen or sulphur atom,
where (C₃-C₇)-cycloalkyl, (C₁-C₆)-alkyl which is optionally interrupted by an oxygen or sulphur atom, the 5- to 7-membered saturated heterocycle which is attached to the imidazole ring via a nitrogen atom and which optionally contains one further oxygen or sulphur atom and optionally (C₁-C₆)-alkyl which is interrupted by a radical NR⁶ and optionally the 5- to 7-membered saturated heterocycle which contains a radical of the formula NR⁷ and optionally additionally one nitrogen, oxygen or sulphur atom are substituted by one hydroxyl group and/or by a radical of the formula -NR⁸R⁹,
in which
R⁶ and R⁷ are identical or different and each represents hydrogen, (C₁-C₄)-alkyl, hydroxy-(C₁-C₄)-alkyl or (C₃-C₆)-cycloalkyl,
R⁸ and R⁹ are identical or different and each represents hydrogen, (C₁-C₄)-alkyl or (C₃-C₆)-cycloalkyl,
or
R⁸ and R⁹ together with the nitrogen atom form a 5- to 7-membered saturated heterocycle which may optionally additionally contain one oxygen or sulphur atom or a radical of the formula NR¹⁰,
in which
R¹⁰ represents hydrogen, (C₁-C₄)-alkyl or (C₃-C₆)-cycloalkyl,
and
R³ represents a phenyl, pyridyl or thienyl ring which is optionally mono- or polysubstituted by radicals selected from the group consisting of fluorine, chlorine, cyano, trifluoromethyl and trifluoromethoxy,
and their enantiomers and diastereomers and their respective salts and hydrates.

3. Compounds according to Claim 1 or 2
where
A, D and E each represent the CH group,
G represents a nitrogen atom or represents the CH group,
L¹ and L² each represent hydrogen,
R¹ represents a radical of the formula -CO-NR⁴R⁵,
in which
R⁴ and R⁵ each represent hydrogen,
R² represents (C₁-C₄)-alkyl which is optionally interrupted by an oxygen atom, or
represents a 4-R⁷-piperazin-1-yl radical
where (C₁-C₄)-alkyl, which is optionally interrupted by an oxygen atom, is substituted by a hydroxyl group or by a radical of the formula -NR⁸R⁹,
in which
R⁷ represents hydrogen, (C₁-C₄)-alkyl or (C₃-C₆)-cycloalkyl,
R⁸ and R⁹ are identical or different and each represents hydrogen, (C₁-C₄)-alkyl or (C₃-C₆)-cycloalkyl,
or
R⁸ and R⁹ together with the nitrogen atom form a morpholine radical,
and
R³ represents a phenyl or pyridyl radical, where the phenyl radical may be mono- or polysubstituted by fluorine,
and their enantiomers and diastereomers and their respective salts and hydrates.

4. Compounds according to Claim 3
where
the radical R¹ represents a radical of the formula CO-NR⁴R⁵ where R⁴ and R⁵ are as defined above
and
the other radicals are as defined in Claim 3.

5. Compounds according to either of Claims 3 or 4, **characterized by** the following stereochemistry according to formula (Ia): 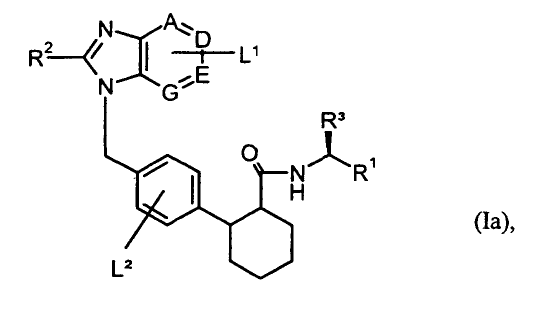 where the substituents R¹, R², R³, L¹ and L² and the radicals A, D, E and G are each as defined in Claims 3 or 4.

6. Compounds according to any of Claims 3 to 5, **characterized by** the following stereochemistry according to formula (Ib) 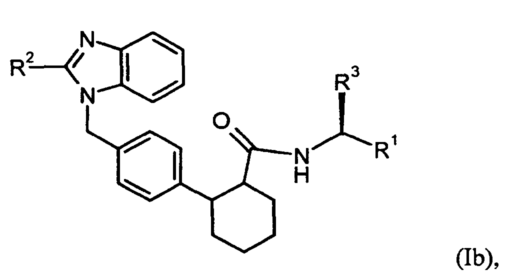 in which
R¹ represents a group -C(O)-NH₂,
R² represents (C₁-C₄)-alkyl which is substituted at the terminal C atom by a hydroxyl group,
R³ represents a phenyl ring which is substituted in the para position by fluorine,
or
represents a pyridyl radical,
and their diastereomers and their respective salts and hydrates.

7. Compounds according to Claim 3 of the general formula (I) of the following structures: 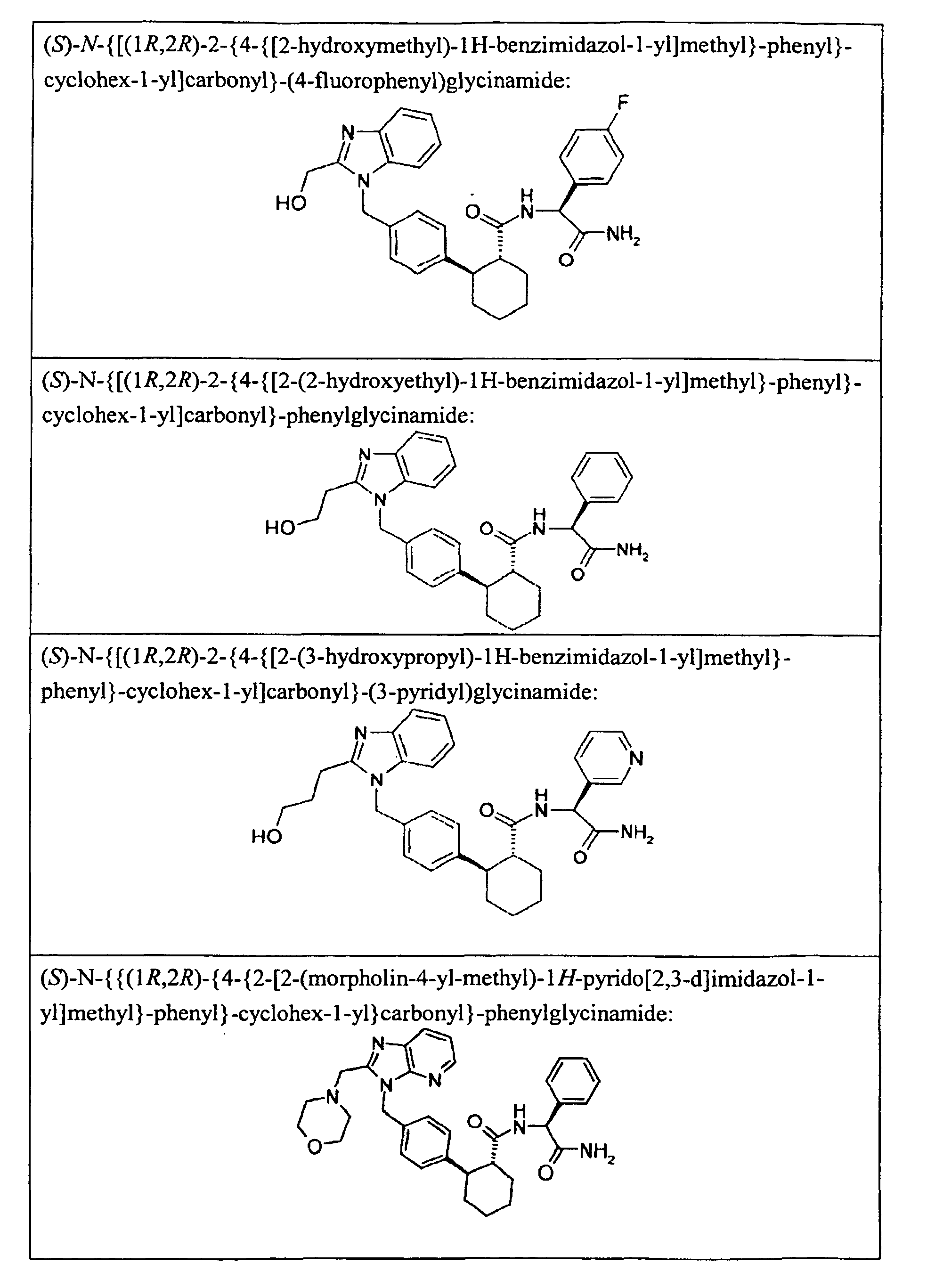 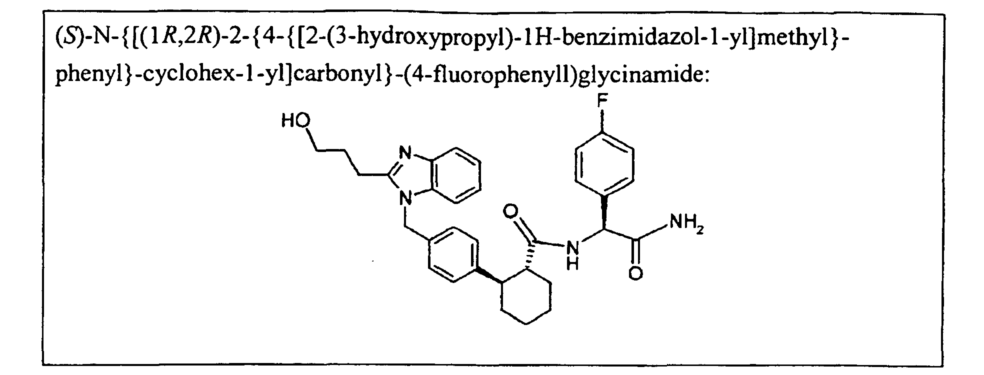 and their salts and hydrates.

8. Process for preparing compounds of the general formula (I) according to Claims 1 to 7, **characterized in that**
[A] compounds of the general formula (II) 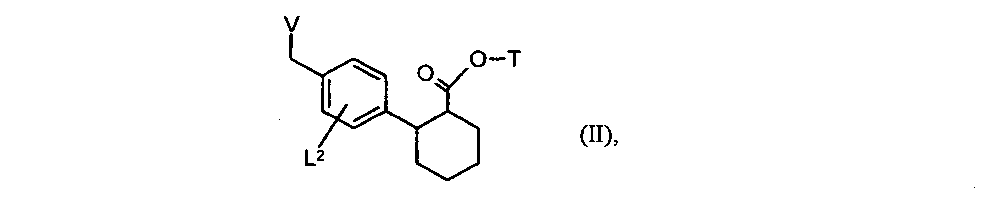 in which
L² is as defined above,
T represents (C₁-C₄)-alkyl, preferably methyl or tert-butyl,
and
V represents a suitable leaving group, such as, for example, halogen, mesylate or tosylate, preferably bromine,
are initially converted by reaction with compounds of the general formula (III) 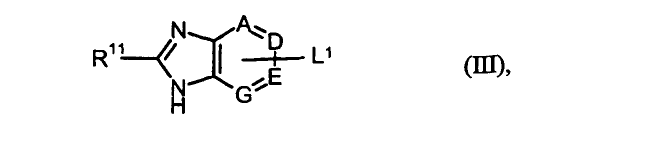 in which
A, D, E, G and L¹ are each as defmed above
and
R¹¹ has the meaning of R² given above, where amino and hydroxyl functions are optionally blocked by suitable amino- or hydroxyl-protective groups,
in inert solvents, depending on the definition of R¹¹ optionally in the presence of a base, into the compounds of the general formula (IV) 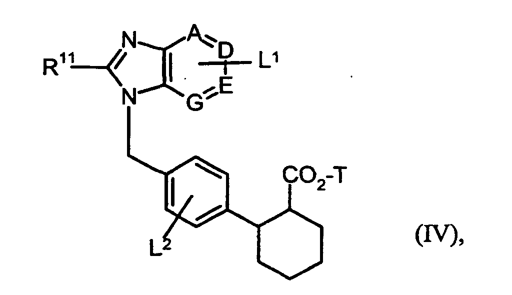 in which
R¹¹, A, D, E, G, L¹, L² and T are each as defined above,
which are converted in a subsequent step using acids or bases into the corresponding carboxylic acids of the general formula (V) 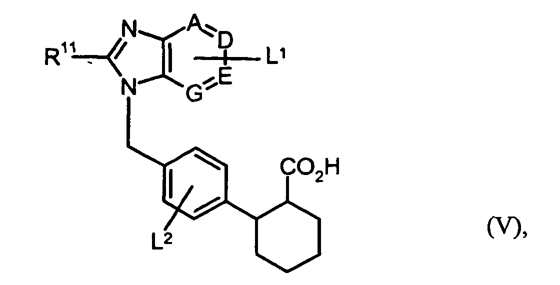 in which
R¹¹, A, D, E, G, L¹ and L² are each as defined above,
which are, if appropriate, activated, in particular by conversion into a corresponding carboxylic acid derivative, such as carbonyl halide, carboxylic anhydride or carboxylic ester,
and which are subsequently reacted by known methods with compounds of the general formula (VI) or salts thereof 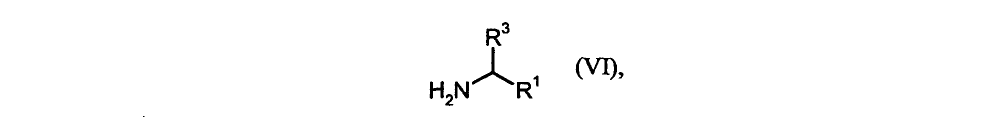 in which
R¹ and R³ are each as defined in Claims 3 to 6
in inert solvents,
and, if R¹¹ carries one of the abovementioned protective groups, this is optionally removed by customary methods either in the hydrolysis to the acids (IV) → (V) or after the reaction with the compounds of the general formula (VI),
or
[B] if R² represents a saturated heterocycle which is attached directly to the imidazole ringvia a nitrogen atom,
the abovementioned compounds of the general formula (II) are initially converted with compounds of the general formula (IIIa) 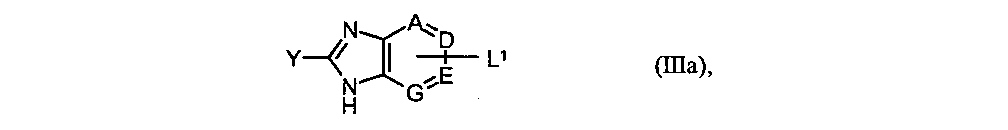
in which
A, D, E, G and L¹ are each as defined above
and
Y represents halogen or mesylate, preferably chlorine, bromine or mesylate,
in inert solvents into the corresponding compounds of the formula (VII) 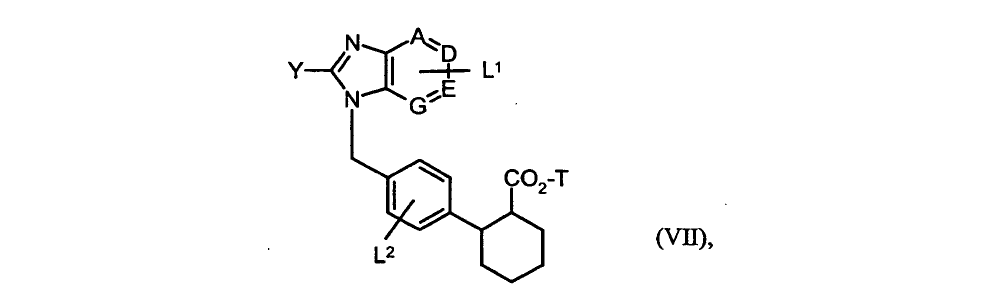 in which
Y, A, D, E, G, L¹, L² and T are each as defined above,
which are reacted in a subsequent step with compounds of the general formula (VIII)
HNR¹²R¹³ (VIII),
in which
R¹² and R¹³ together with the nitrogen atom form a heterocycle according to the definition of R²
to give compounds of the general formula (IX) 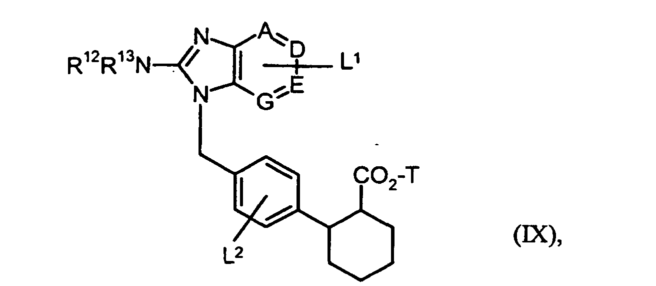 in which
A, D, E, G, L¹, L², R¹², R¹³ and T are each as defined above,
which are, in the subsequent steps, converted as described under [A] by hydrolysis into the corresponding carboxylic acids of the general formula (X) 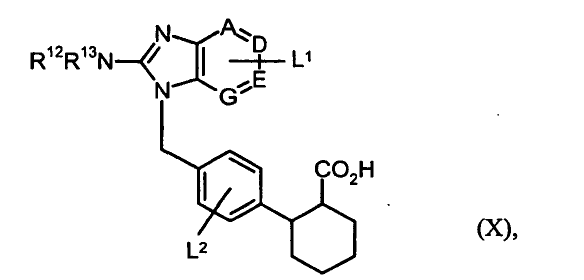 in which
A, D, E, G, L¹, L², R¹² and R¹³ are each as defined above
and these compounds are finally reacted with the compounds of the general formula (VI) according to known methods for preparing amides from carboxylic acids and amines and converted into the compounds of the general formula (I)
where the compounds of the general formula (I) obtained according to process variant [A] or [B] can, if appropriate, subsequently be converted into the corresponding salts by reaction with, for example, an acid.

9. (*S*)-(4-Fluorophenyl)glycinamide 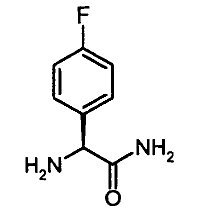 and its salts.

10. (*S*)-(3-Pyridyl)glycinamide 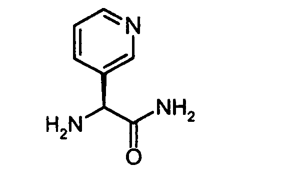 and its salts.

11. Medicaments, comprising a compound of the general formula (I) according to any of Claims 3 to 7 in admixture with at least one pharmaceutically acceptable, essentially non-toxic carrier or excipient.

12. Compounds according to any of Claims 3 to 7 for use as medicament in the treatment of humans and animals.

13. Compounds according to any of Claims 3 to 7 for the prophylaxis and/or treatment of disorders in humans and animals.

14. Use of compounds according to any of Claims 3 to 7 for preparing medicaments for the prophylaxis and/or treatment of disorders in humans and animals.

15. Use of compounds according to any of Claims 3 to 7 for preparing medicaments for the treatment and/or prophylaxis of ischaemic disorders of the cardiovascular system.

## Revendications

1. Utilisation de composés de formule générale (I) 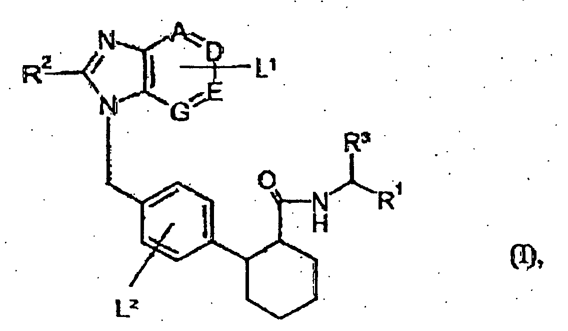 dans laquelle
A, D, E et G sont identiques ou différents et représentent des groupes CH ou des atomes d'azote,
L¹ et L² sont identiques ou différents et représentent, indépendamment l'un de l'autre, un ou plusieurs restes choisis dans le groupe hydrogène, halogène, hydroxy, carboxyle, cyano, nitro, trifluorométhyle, trifluorométhoxy, alkyle en C₁ à C₆, alkoxy en C₁ à C₆ ou (alkoxy en C₁ à C₆)-carbonyle,
R¹ représente le groupe CH₂-OH ou
un reste de formule CO-NR⁴R⁵,
où
R⁴ et R⁵ sont identiques ou différents et représentent l'hydrogène ou un reste alkyle en C₁ à C₆,
R² est un reste cycloalkyle en C₃ à C₈,
un reste alkyle en C₁ à C₈ qui est éventuellement interrompu par un atome, d'oxygène ou de soufre ou par un reste NR⁶,
un hétérocycle saturé tétragonal à octogonal, lié au noyau imidazole par l'intermédiaire d'un atome d'azote, qui contient éventuellement un autre atome d'oxygène ou de soufre, ou bien
un hétérocycle saturé tétragonal à octogonal qui contient un reste de formule NR⁷ et en outre, le cas échéant, un atome d'azote, d'oxygène ou de soufre,
le reste cycloalkyle en C₃ à C₈, le reste alkyle en C₁ à C₈ qui est éventuellement interrompu par un atome d'oxygène ou de soufre, l'hétérocycle tétragonal à octogonal lié au noyau imidazole par l'intermédiaire d'un atome d'azote, qui contient éventuellement un autre atome d'oxygène ou de soufre ainsi que, le cas échéant, le reste alkyle en C₁ à C₈ qui est interrompu par un reste NR⁶, et le cas échéant, l'hétérocycle saturé tétragonal à octogonal qui contient un reste de formule NR⁷ et en outre, le cas échéant, un atome d'azote, d'oxygène ou de soufre, étant substitué par un à trois groupes hydroxyle et/ou par un reste de formule -NR⁸R⁹,
où
R⁶ et R⁷ sont identiques ou différents et représentent l'hydrogène, un reste alkyle en C₁ à C₆, hydroxyalkyle en C₁ à C₆ ou cycloalkyle en C₃ à C₇,
R⁸ et R⁹ sont identiques ou différents et représentent l'hydrogène, un reste alkyle en C₁ à C₆ ou cycloalkyle en C₃ à C₇,
ou bien
R⁸ et R⁹ forment, conjointement avec l'atome d'azote, un hétérocycle saturé tétragonal à octogonal qui peut éventuellement contenir en outre un atome d'oxygène ou de soufre ou un reste de formule NR¹⁰,
dans laquelle
R₁₀ représente l'hydrogène, un reste alkyle en C₁ à C₆ ou cycloalkyle en C₃ à C₇,
et
R³ représente un noyau phényle, naphtyle, pyrimidinyle, pyridyle, furyle ou thiényle, ces noyaux étant éventuellement substitués une ou plusieurs fois avec des restes choisis dans le groupe consistant en halogène, hydroxy, carboxyle, cyano, nitro, trifluorométhyle, trifluorométhoxy, alkyle en C₁ à C₆, alkoxy en C₁ à C₆ ou (alkoxy en C₁ à C₆) carbonyle,
et leurs énantiomères et leurs diastéréo-isomères ainsi que les sels et hydrates correspondants, pour la préparation de médicaments destinés au traitement et/ou à la prophylaxie de maladies ischémiques du système cardiovasculaire.

2. Utilisation de composés suivant la revendication 1,
dans laquelle
A, D, E et G représentent chacun un groupe CH,
ou bien l'un des restes A, D, E et G désigne un atome d'azote et les autres représentent chacun un groupe CH,
L¹ et L² sont identiques ou différents et représentent, indépendamment l'un de l'autre, un ou plusieurs restes choisis dans le groupe hydrogène, fluor, chlore, cyano, trifluorométhyle ou trifluorométhoxy,
R¹ représente le groupe CH₂-OH, ou bien un reste de formule CO-NR⁴R⁵,
où
R⁴ et R⁵ sont identiques ou différents et représentent l'hydrogène ou un reste alkyle en C₁ à C₃,
R² est un reste cycloalkyle en C₃ à C₇,
un reste alkyle en C₁ à C₆ qui est éventuellement interrompu par un atome d'oxygène ou de soufre ou par un reste NR⁶,
un hétérocycle saturé pentagonal à heptagonal, lié au noyau imidazole par l'intermédiaire d'un atome d'azote, qui contient éventuellement un autre atome d'oxygène ou de soufre, ou bien
un hétérocycle saturé pentagonal à heptagonal qui contient un reste de formule NR⁷ et en outre, le cas échéant, un atome d'azote, d'oxygène ou de soufre,
le reste cycloalkyle en C₃ à C₇, le reste alkyle en C₁ à C₆ qui est éventuellement interrompu par un atome d'oxygène ou de soufre, l'hétérocycle saturé pentagonal à heptagonal, lié au noyau imidazole par l'intermédiaire d'un atome d'oxygène, qui contient éventuellement un autre atome d'oxygène ou de soufre, ainsi que, le cas échéant, le reste alkyle en C₁ à C₆ qui est interrompu par un reste NR⁶, et le cas échéant, l'hétérocycle saturé pentagonal à heptagonal qui contient un reste de formule NR⁷ et en outre, le cas échéant, un atome d'azote, d'oxygène ou de soufre, étant substitué par un groupe hydroxyle et/ou par un reste de formule -NR⁸R⁹,
où
R⁶ et R⁷ sont identiques ou différents et représentent l'hydrogène, un reste alkyle en C₁ à C₄, hydroxyalkyle en C₁ à C₄ ou cycloalkyle en C₃ à C₆,
R⁸ et R⁹ sont identiques ou différents et représentent l'hydrogène, un reste alkyle en C₁ à C₄ ou cycloalkyle en C₃ à C₆,
ou bien
R⁸ et R⁹ forment, conjointement avec l'atome d'azote, un hétérocycle saturé pentagonal à heptagonal qui peut éventuellement contenir en outre un atome d'oxygène ou de soufre ou un reste de formule NR¹⁰,
dans laquelle
R¹⁰ représente l'hydrogène, un reste alkyle en C₁ à C₆ ou cycloalkyle en C₃ à C₆,
et
R³ représente un noyau phényle, pyridyle ou thiényle, qui est éventuellement substitué une ou plusieurs fois avec des restes choisis dans le groupe fluor, chlore, cyano, trifluorométhyle ou trifluorométhoxy,
et de leurs énantiomères et leurs diastéréo-isomères ainsi que de leurs sels et hydrates correspondants.

3. Composés suivant la revendication 1 ou 2,
dans lesquels
A, D et E représentent chacun un groupe CH,
G est un atome d'azote ou représente le groupe CH,
L¹ et L² représentent l'hydrogène,
R¹ est un reste de formule CO-NR⁴R⁵,
dans laquelle
R⁴ et R⁵ représentent l'hydrogène,
R² représente un reste alkyle en C₁ à C₄ qui est éventuellement interrompu par un atome d'oxygène, ou bien
un reste 4-R⁷-pipérazine-1,
le reste alkyle en C₁ à C₄, qui est éventuellement interrompu par un atome d'oxygène, étant substitué par un groupe hydroxyle ou par un reste de formule -NR⁸R⁹,
où
R⁷ représente l'hydrogène, un reste alkyle en C₁ à C₄ ou cycloalkyle en C₃ à C₆,
R⁸ et R⁹ sont identiques ou différents et représentent l'hydrogène, un reste alkyle en C₁ à C₄ ou cycloalkyle en C₃ à C₆,
ou bien
R⁸ et R⁹ forment, conjointement avec l'atome d'azote, un reste morpholine,
et
R³ est un reste phényle ou pyridyle, le reste phényle pouvant être substitué une ou plusieurs fois par du fluor,
et de leurs énantiomères et leurs diastéréo-isomères ainsi que de leurs sels et hydrates correspondants.

4. Composés suivant la revendication 3,
dans lesquels
le reste R¹ représente un reste de formule CO-NR⁴R⁵ dans laquelle R⁴ et R⁵ sont tels que définis ci-dessus
et
les autres restes ont la définition indiquée dans la revendication 3.

5. Composés suivant l'une des revendications 3 ou 4, **caractérisés par** la stéréochimie selon la formule (Ia) : 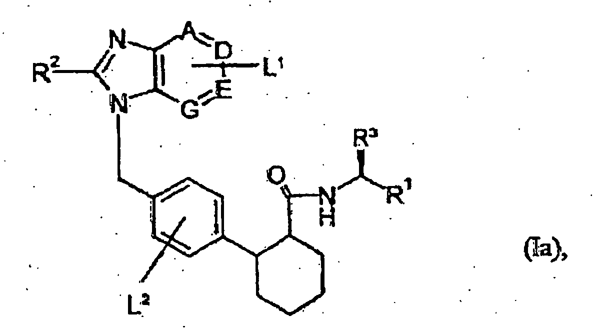 les substituants R¹, R², R³, L¹ et L² et les restes A, D, E et G ayant la définition indiquée dans les revendications 3 ou 4.

6. Composés suivant l'une des revendications 3 à 5, **caractérisés par** la stéréochimie suivante selon la formule (Ib) 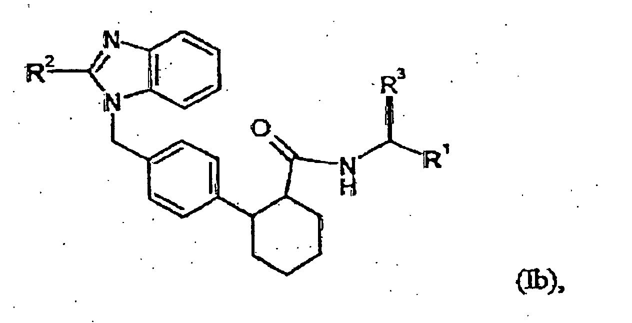 dans laquelle
R¹ représente un groupe -C(O)-NH₂,
R² est un reste alkyle en C₁ à C₄ qui est substitué sur l'atome terminal de carbone par un groupe hydroxyle,
R³ représente un noyau phényle qui est substitué en position para par du fluor,
ou
représente un reste pyridyle,
et leurs diastéréo-isomères ainsi que leurs sels et hydrates correspondants.

7. Composés suivant la revendication 3, de formule générale (I), présentant les structures suivantes : 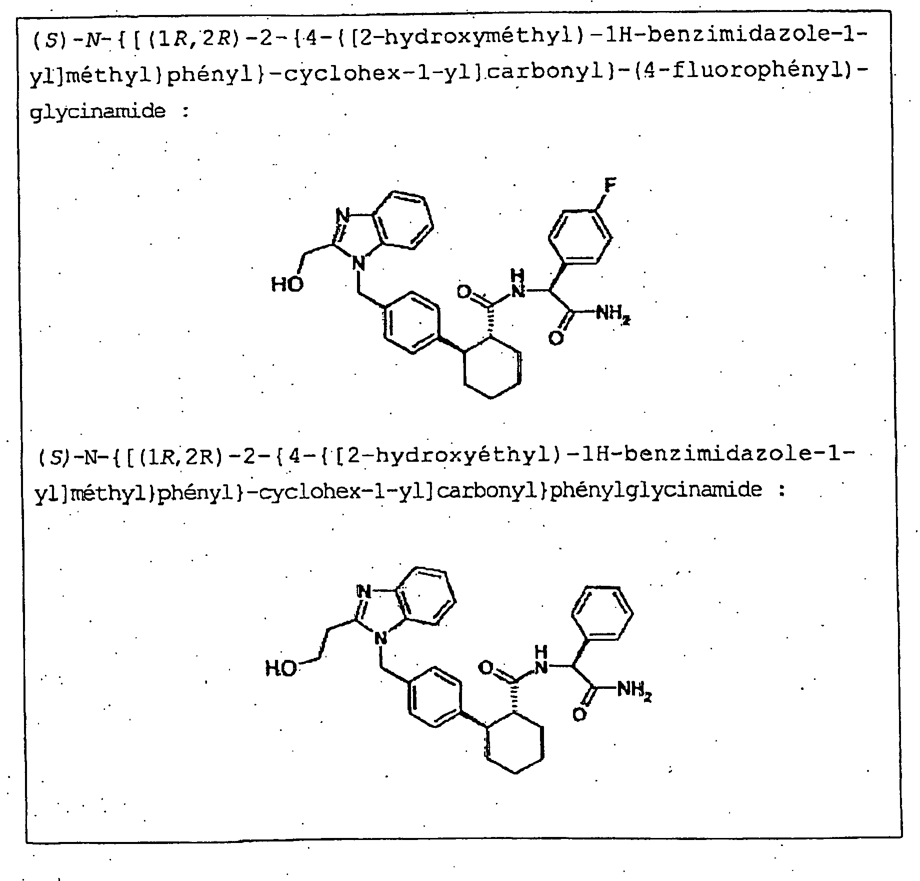 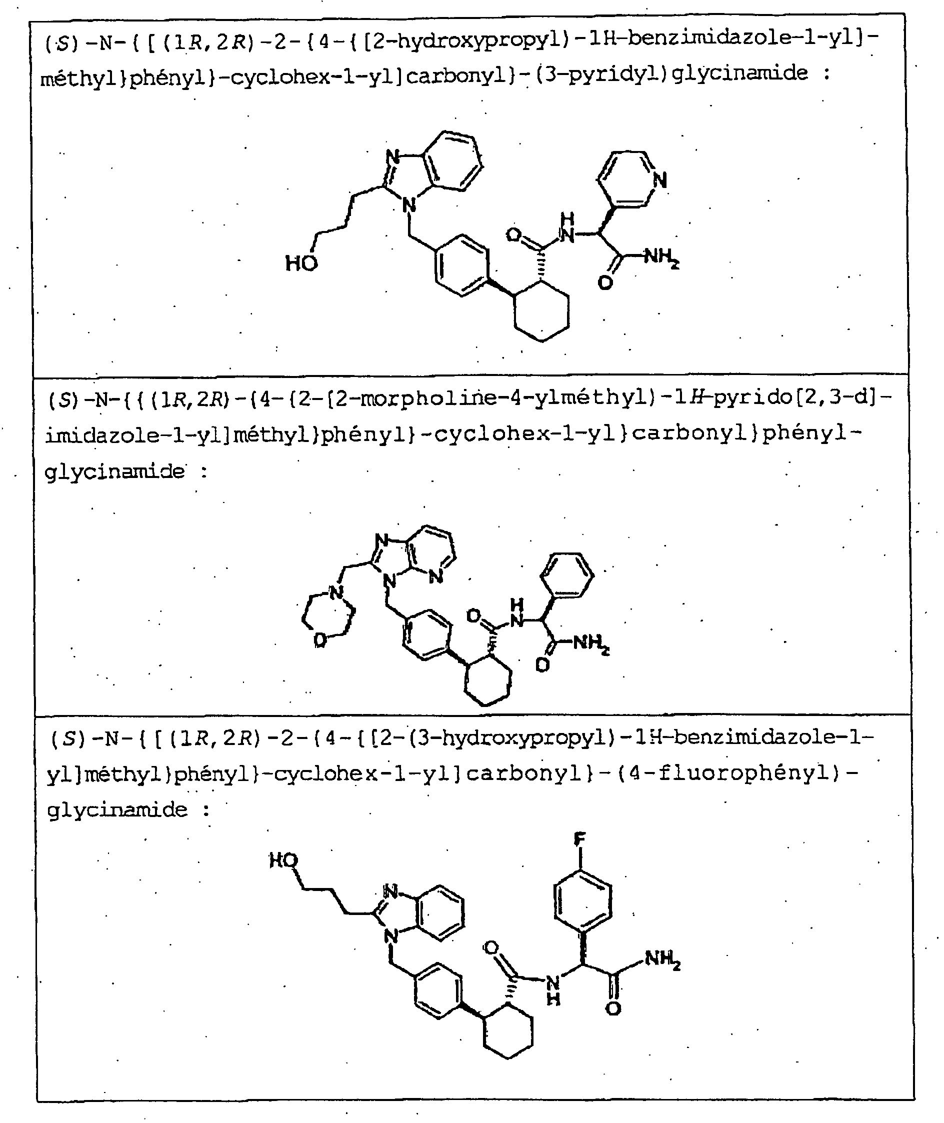 et leurs sels et hydrates.

8. Procédé de production de composés de formule générale (I) suivant les revendications 1 à 7, **caractérisé en ce que** :
[A] on transforme tout d'abord des composés de formule générale (II) 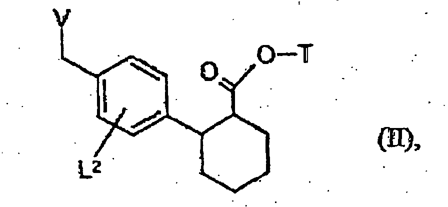 dans laquelle
L² a la définition indiquée ci-dessus,
T est un reste alkyle en C₁ à C₄, avantageusement un reste méthyle ou tertiobutyle,
et
V représente un groupe partant convenable tel que, par exemple, halogène, mésylate ou tosylate, avantageusement le brome,
par réaction avec des composés de formule générale (III) 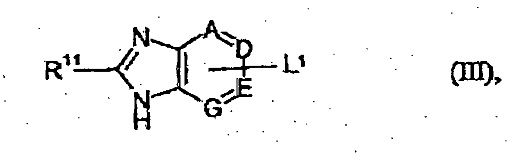 dans laquelle
A, D, E, G et L¹ ont la définition indiquée ci-dessus,
et
R¹¹ a la définition indiquée ci-dessus pour R², les fonctions amino et hydroxy étant éventuellement protégées par des groupes protecteurs de la fonction amino ou hydroxy,
dans des solvants inertes, en fonction de la définition de R¹¹, éventuellement en présence d'une base, en composés de formule générale (IV) 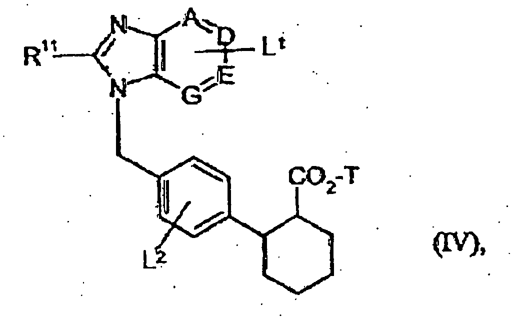 dans laquelle
R¹¹, A, D, E, G, L¹, L² et T ont la définition indiquée ci-dessus,
on transforme ces composés dans une étape subséquente avec des acides ou des bases en les acides carboxyliques correspondants de formule générale (V) 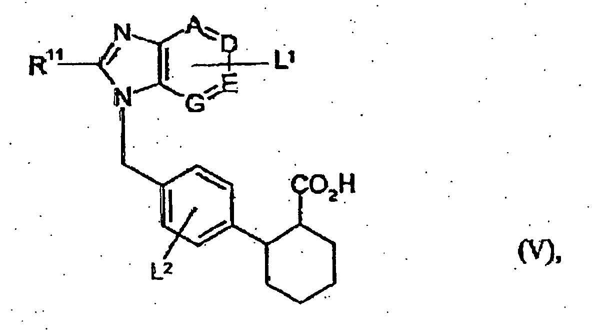 dans laquelle
R¹¹, A, D, E, G, L¹ et L² ont la définition indiquée ci-dessus,
on active éventuellement ces composés, en particulier par transformation en un dérivé correspondant d'acide carboxylique tel qu'halogénure d'acide carboxylique, anhydride d'acide carboxylique ou ester d'acide carboxylique,
et on les fait finalement réagir selon des modes opératoires connus avec des composés de formule générale (VI) ou leurs sels 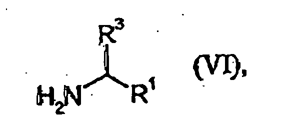 formule dans laquelle
R¹ et R³ ont la définition indiquée dans les revendications 3 à 6,
dans des solvants inertes,
et dans le cas éventuel où R¹¹ porte l'un des groupes protecteurs indiqués ci-dessus, on élimine ces groupes, soit lors de l'hydrolyse en acides (IV)-(V), ou après réaction avec les composés de formule générale (VI) par des moyens usuels,
ou bien
[B] au cas où R² représente un hétérocycle saturé lié directement au noyau imidazole par un atome d'azote, on transforme tout d'abord les composés indiqués ci-dessus de formule générale (II) par réaction avec des composés de formule générale (IIIa) 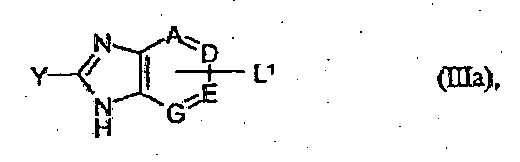
dans laquelle
A, D, E, G et L¹ ont la définition indiquée ci-dessus,
et
Y représente un halogène ou un groupe mésylate, avantageusement le chlore, le brome ou le groupe mésylate,
dans des solvants inertes, en composés correspondants de formule générale (VII) 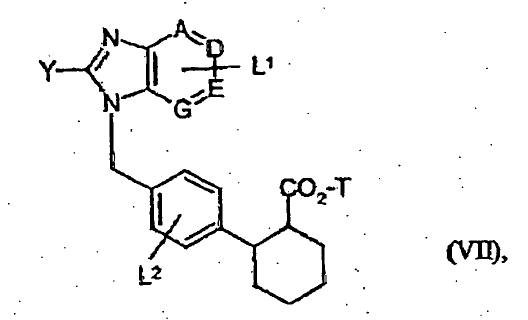 dans laquelle
Y, A, D, E, G, L¹, L² et T ont la définition indiquée ci-dessus,
on les fait réagir dans une étape subséquente avec des composés de formule générale (VIII)
HNR¹²R¹³ (VIII),
dans laquelle
R¹² et R¹³ forment conjointement avec l'atome d'azote un hétérocycle répondant à la définition de R²,
pour obtenir des composés de formule générale (IX) 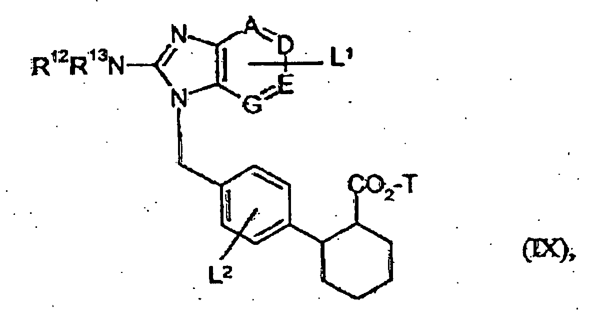 dans laquelle
A, D, E, G, L¹, L², R¹², R¹³ et T ont la définition indiquée ci-dessus,
on les transforme dans les étapes subséquentes, de la manière décrite en [A], par hydrolyse en les acides carboxyliques correspondants de formule générale (X) 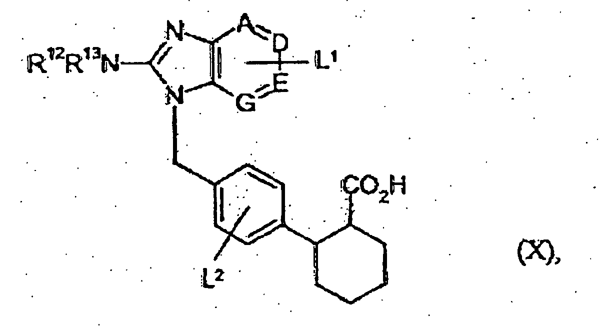 dans laquelle
A, D, E, G, L¹, L², R¹² et R¹³ ont la définition indiquée ci-dessus,
et finalement, on les fait réagir selon des modes opératoires connus pour la préparation d'amides à partir d'acides carboxyliques et d'amines avec les composés de formule générale (VI) pour obtenir les composés de formule générale (I),
les composés de formule générale (I) obtenus selon la variante [A] ou [B] du procédé pouvant éventuellement être transformés ensuite, par réaction par exemple avec un acide, en les sels correspondants.

9. Le (*S*)-(4-fluorophényl)glycinamide 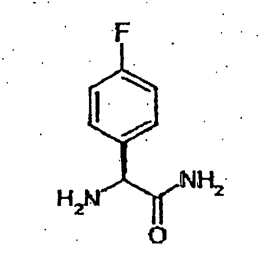 et ses sels.

10. Le (*S*)-(3-pyridyl)glycinamide 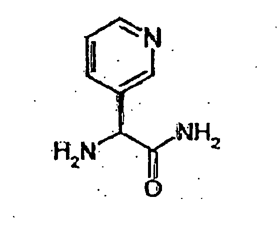 et ses sels.

11. Médicament, contenant un composé de formule générale (I) suivant l'une des revendications 3 à 7 conjointement avec au moins un support ou excipient principalement non toxique, acceptable du point de vue pharmaceutique.

12. Composés suivant l'une des revendications 3 à 7, destinés à être utilisés comme médicaments dans le traitement d'êtres humains et d'animaux.

13. Composés suivant l'une des revendications 3 à 7, destinés à la prophylaxie et/ou au traitement de maladies chez des êtres humains et des animaux.

14. Utilisation de composés suivant l'une des revendications 3 à 7 pour la préparation de médicaments destinés à la prophylaxie ét/ou au traitement de maladies chez des êtres humains et des animaux.

15. Utilisation de composés suivant l'une des revendications 3 à 7 pour la préparation de médicaments destinés au traitement et/ou à la prophylaxie de maladies systémiques du système cardiovasculaire.
